# EUROPEAN PATENT APPLICATION

(11) **EP 2 518 064 A1**
(43) Date of publication of application: **31.10.2012**
(21) Application number: 10839597.1
(22) Date of filing: 24.12.2010
(51) Int. Cl.: C07D 311/68, A61K 31/4433, A61K 31/454, A61K 31/496, A61K 31/501, A61P 25/04, A61P 43/00, C07D 401/12, C07D 405/04, C07D 405/12, C07D 405/14, C07D 413/04, C07D 413/12, C07D 417/12

(54) **NOVEL ARYL UREA DERIVATIVE**

(30) Priority: 25.12.2009 JP 2009295500
(71) Applicant: Mochida Pharmaceutical Co., Ltd., Tokyo 160-8515 (JP)
(72) Inventor: KAMINO Tomoyuki, Tokyo 160-8515 (JP); MAEDA Yoshitaka, Tokyo 160-8515 (JP)
(74) Representative: von Kreisler Selting Werner
(86) International application number: PCT/JP2010/073458
(87) International publication number: WO 2011/078369

(57) **Abstract**

There is a need for FAAH inhibitors capable of oral administration and having excellent efficacy, particularly agents for the prevention and treatment of pain. Disclosed are novel arylurea compounds represented by formula (I), salts or solvates thereof, and pharmaceutical compositions comprising the same as an active ingredient. The pharmaceutical composition is used primarily for FAAH inhibitors, or agents for prevention and treatment of pain.

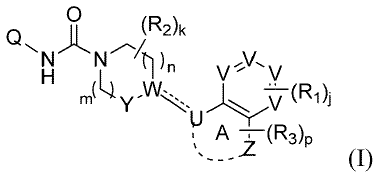

## Description

### TECHNICAL FIELD

The present invention relates to medical drugs, particularly to compounds useful for inhibiting the function of fatty acid amide hydrolase (hereinafter abbreviated as "FAAH"), and more particularly to an arylurea derivative represented by formula (I), a pharmaceutical composition comprising the derivative as an active ingredient, an agent for the prevention or treatment of diseases including pain in which FAAH is related, and so on.

### BACKGROUND ART

FAAH is an intracellular membrane-bound enzyme that hydrolyzes endocannabinoids and is known to extinguish the endocannabinoid's activity through its catabolism (Nature, 384, 83-87, 1996).

The term endogenous cannabinoid or endocannabinoid is used to refer collectively to endogenous substances such as anandamide, palmitoylethanolamine, oleamide, glycerol 2-arachidonate, etc., which act on cannabinoid receptors. These endocannabinoids are known to have a variety of physiological activities including analgesia (Nature, 394, 277-281, 1998), regulation of feeding (Nature, 414, 209 -212, 2001), promotion of sleep (Science, 268, 1506-1509, 1995), etc.

The currently known receptors for endocannabinoids are CB1 receptors (Nature, 346, 561-567, 1990), CB2 receptors (Eur. J. Biochem., 232, 54-61, 1995) and GPR55 (Br. J. Pharmacol., 152, 1092-1101, 2007). The active ingredient Δ9-tetrahydrocannabinol of *Cannabis sativa* is an agonist at CB1 receptors, and has useful pharmacological effects such as an analgesic activity. On the other hand, the compound has unwanted central side effects including hypothermia, catalepsy, etc., resulting in limited application for clinical use.

In mice genetically lacking the FAAH gene (Proc. Natl. Acad. Sci., 98, 9371-9376, 2001), brain levels of anandamide increase more than 10-fold, but CB1 receptor-mediated changes including abnormal motility or body temperature and catalepsy are not observed. On the other hand, analgesic effects e.g. increasing the reaction threshold in the tail immersion test or hot-plate test, and reducing the duration time of pain behaviors in the formalin test, have been confirmed.

Also there are many reports on FAAH inhibitors. Lichtman et al. reported on α-keto-heterocycle OL-135 (J. Pharmacol. Exp. Ther. 311, 441-448, 2004), showing increase in pain threshold in both the tail immersion test and the hot-plate test with mice as well as reduction in the duration time of pain behaviors in the formalin test. Carbamate type inhibitor URB-597 improves mechanical allodynia and thermal hyperalgesia in the rat CFA (complete Freund's adjuvant)-induced inflammatory pain model, without affecting any spontaneous motor activity (J. Pharmacol. Exp. Ther. 322, 236-242, 2007). Sit SY et al. further showed the effectiveness of another carbamate type inhibitor in the rat formalin test and on neuropathic pain models (Bioorg. Med. Chem. Lett., 17, 3287-3291, 2007).

These reports suggest that selective inhibition of FAAH would be an analgesic therapy with minimal side effects. It is thus expected that FAAH inhibitors would be useful analgesic drugs.

In 1981, International Association for the Study of Pain defined the term "pain" as "an unpleasant sensory and emotional experience associated with actual or potential tissue damage, or the same experience as represented by the term meaning such damage." Its presence may be serious to patients and a critical factor which markedly lowers QOL. Therefore, active analgesic therapies are needed.

In clinical practice, non-steroidal anti-inflammatory drugs, narcotic analgesics such as morphine, etc., anti-depressive agents such as amitriptyline, etc., antiepileptics such as gabapentin, pregabalin, carbamazepine, phenytoin, etc., antiarrhythmic drugs such as mexiletine, etc., which are nonselective sodium channel blockers, are diverted/prescribed for the purpose of pain relief of chronic pain. However, the analgesic effects of non-steroidal anti-inflammatory drugs are not completely satisfied and the drugs have the problematic side effects including gastrointestinal disturbances, renal disturbances, etc. Narcotic analgesics such as morphine, etc. are very effective mainly for nociceptive pain but due to big problems of side effects on the alimentary system, respiratory system and central nervous system, these drugs generally have limited usefulness in neuropathic pain. Side effects of thirst, drowsiness, sedation, constipation, difficulty in urination, etc. are known for amitriptyline; those of incoordination, rash, gastrointestinal symptom, cardiotoxicity, etc. for carbamazepine and phenytoin; and those of somnolence or dizziness for gabapentin, and dizziness, gastrointestinal symptom, etc. for mexiletine.

These drugs described above have poor dissociation between drug efficacy and side effects and the satisfaction levels of these treatments are low. Accordingly, an agent for the treatment of chronic pain, which shows a higher efficacy by oral administration and has minimal side effect, is desired.

Under the foregoing circumstances, expectations for FAAH inhibitors have increased in recent years as agents for the treatment of chronic pain with minimal side effects on the cardiovascular, alimentary and central nervous systems, etc.

Recently, studies of compounds having the FAAH inhibitory action have been extended. There are known W02006/054652 pamphlet (Patent Document 1), W02006/074025 pamphlet (Patent Document 2), W02007/005510 pamphlet (Patent Document 3), WO2009/127943 pamphlet (Patent Document 4), W02009/127944 pamphlet (Patent Document 5), WO2009/127946 pamphlet (Patent Document 6), WO2009/127948 pamphlet (Patent Document 7), W02009/127949 pamphlet (Patent Document 8), WO2010/049841 pamphlet (Patent Document 18), WO2010/053120 pamphlet (Patent Document 19), WO2010/058318 pamphlet (Patent Document 20), WO2010/068452 pamphlet (Patent Document 21), WO2010/068453 pamphlet (Patent Document 22), WO2010/117014 pamphlet (Patent Document 23), W02010/141809 pamphlet (Patent Document 24), W02010/141817 pamphlet (Patent Document 25), etc. However, compounds having both a cyclic amino structure bound to fused heterocycles and an arylurea moiety are not disclosed in these patent documents.

Related arts which disclose compounds bearing an arylurea moiety or a cyclic group-bound urea structure include WO2007/007069 pamphlet (Patent Document 9), US3290313 pamphlet (Patent Document 10), EP0745597 pamphlet (Patent Document 11), JPA2007/016011 pamphlet (Patent Document 12), JPA2003/192673 pamphlet (Patent Document 13) and W02003/084948 pamphlet (Patent Document 14).

Patent Document 9 discloses the compounds bearing an arylurea moiety as amine derivatives which are Nav1.8 inhibitors. However, the document does not disclose any compound having cyclic ether bound on the piperazine ring or any compound having the FAAH inhibitory action as in the invention.

Patent Document 10 discloses the compounds bearing an arylurea moiety as compounds exhibiting an anti-tumor action. However, the document does not disclose any compound having an ether ring bound on the piperazine ring or any compound having the FAAH inhibitory action as in the invention.

Patent Document 11 discloses the derivatives bearing a urea moiety as sleep improvement drugs. However, the document does not disclose any compound bearing an arylurea moiety, or any compound having the FAAH inhibitory action as in this invention.

Patent Document 12 discloses, as compounds having an antioxidant activity, the compounds having a specific substituted fused aryl group, etc. bound to the piperazine structure. However, the document does not disclose any compound having the FAAH inhibitory action which bears both the cyclic amino moiety bound to a fused hetero ring and the arylurea moiety, as in this invention.

Patent Document 13 discloses, as TRPV1 inhibitors, the derivatives having a quinoline bound to the piperazine structure. However, the document does not disclose any compound having the FAAH inhibitory action which bears both the cyclic amino moiety bound to a fused hetero ring and the arylurea moiety, as in this invention.

Patent Document 14 discloses the derivatives having the piperazine structure as Na channel blockers. However, the document does not disclose any compound having the FAAH inhibitory action which bears both the cyclic amino moiety bound to a fused hetero ring and the arylurea moiety, as in this invention.

Patent Document 15 discloses derivatives having a silyl structure as TRPV1 inhibitors. However, the document does not disclose any compound having the FAAH inhibitory action which bears both the cyclic amino moiety bound to a fused hetero ring and the arylurea moiety, as in this invention.

Patent Document 16 discloses the derivatives having a benzolactam structure as oxytocin receptor antagonists. However, the document does not disclose any compound having the FAAH inhibitory action which bears both the cyclic amino moiety bound to a fused hetero ring and the arylurea moiety, as in this invention.

Patent Document 17 discloses the derivatives having a benzolactam structure as vasopressin receptor antagonists. However, the document does not disclose any compound having the FAAH inhibitory action which bears both the cyclic amino moiety bound to a fused hetero ring and the arylurea moiety, as in this invention.

As such, the above documents disclose the compounds, which structures are the same in part as those of the compounds in accordance with the present invention. However, the documents do not teach or suggest at all that these compounds have the FAAH inhibitory action.

In the development of pharmaceuticals, it is required to have satisfactory properties not only for the targeted pharmacological activity but also for various aspects of absorption, distribution, metabolism, excretion, and the like. Various factors need to be considered in, for example, drug interactions, desensitization or tolerance, digestive absorption upon oral administration, a rate of transfer to the small intestine, an absorption rate and first-pass effect, an organ barrier, protein binding, induction of a drug-metabolizing enzyme, an excretion pathway and *in vivo* clearance, a method of administration (application sites, methods and purposes), and the like. However, a drug that satisfies these requirements is hardly found. These general problems in drug development always exist, also for FAAH inhibitors, which have not yet been released onto the market. More specifically, even compounds having the FAAH inhibitory action encounter problems in terms of efficacy and safety, which are poor solubility, low metabolic stability resulting in difficulties in systemic exposure, unsatisfactory drugs pharmacokinetics of absorption, duration, etc., or which might exert an inhibitory activity of the human ether-a-go-go related gene (hERG) channel that may be at risk of causing arrhythmia, and the like. Furthermore, some problems come up at the stage of clinical trials. It has thus been desired to solve these problems as many as possible and discover compounds having high efficacy.

In addition, a compound with fewer side effects mentioned above than known drugs that are currently used for the treatment of pain including the inflammatory pain and neuropathic pain as described above has been desired.

### [Related Prior Art Documents]

[Patent Document 1] WO2006/054652 pamphlet
[Patent Document 2] W02006/074025 pamphlet
[Patent Document 3] W02007/005510 pamphlet
[Patent Document 4] WO2009/127943 pamphlet
[Patent Document 5] W02009/127944 pamphlet
[Patent Document 6] WO2009/127946 pamphlet
[Patent Document 7] W02009/127948 pamphlet
[Patent Document 8] W02009/127949 pamphlet
[Patent Document 9] W02007/007069 pamphlet
[Patent Document 10] US3290313 pamphlet
[Patent Document 11] EP0745597 pamphlet
[Patent Document 12] JPA2007/016011 pamphlet
[Patent Document 13] JPA 2003/192673 pamphlet
[Patent Document 14] W02003/084948 pamphlet
[Patent Document 15] WO2010/0092342 pamphlet
[Patent Document 16] WO1995/0002405 pamphlet
[Patent Document 17] EP0382185 pamphlet
[Patent Document 18] WO2010/049841 pamphlet
[Patent Document 19] WO2010/053120 pamphlet
[Patent Document 20] W02010/058318 pamphlet
[Patent Document 21] W02010/068452 pamphlet
[Patent Document 22] W02010/068453 pamphlet
[Patent Document 23] W02010/117014 pamphlet
[Patent Document 24] W02010/141809 pamphlet
[Patent Document 25] W02010/141817 pamphlet

### SUMMARY OF INVENTION

### TECHNICAL PROBLEM

Under such circumstances, FAAH inhibitors that can be orally administered, have high safety and/or exhibit excellent efficacy, in particular, agents for the prevention or treatment of FAAH-related diseases (especially agents for the prevention or treatment of pain) have been desired. The above-described problems exist particularly in the related art. More specifically, the problems include gastrointestinal disturbances, renal disturbances, etc., which are side effects caused by non-steroidal anti-inflammatory drugs; disturbances on the alimentary system, respiratory system and central nervous system, which are side effects caused by narcotic analgesics such as morphine, etc.; thirst, drowsiness, sedation, constipation, dysuria, etc., which are side effects caused by amitriptyline; incoordination, rash, gastrointestinal symptoms, cardiotoxicity, etc., which are side effects caused by carbamazepine and phenytoin; somnolence, dizziness, etc., which are side effects caused by gabapentin; dizziness, gastrointestinal symptoms, etc., which are side effects caused by mexiletine; or heart failure, etc., which are side effects caused by COX2 inhibitors; or, the problems to be solved are reduction in inhibitory effects on hERG currents, improvement of metabolic stability or absorbance, possibility of oral administration, improvement of pharmacokinetics or solubility, and the like. Accordingly, compounds useful in drugs which overcome at least one of these problems and can be orally administered to mammal including human, and in agents for the prevention or treatment of FAAH-related disease (especially agents for the prevention or treatment of pain) have been desired.

### SOLUTION TO PROBLEM

The present inventor has made extensive investigations in an attempt to solve the foregoing problems and obtain compounds capable of inhibiting FAAH with high selectivity, which have high safety and/or excellent efficacy. As a result, it has been found that novel arylurea compounds represented by formula (I) or pharmaceutically acceptable salts thereof or solvates thereof possess an excellent FAAH inhibitory activity, and the present invention has thus been accomplished.

The present invention relates to the compounds represented by formula (I) or pharmaceutically acceptable salts thereof or solvates thereof, and pharmaceutical compositions comprising these derivatives as an active ingredient. The pharmaceutical compositions are used especially as FAAH inhibitors or agents for the prevention or treatment of pain, in particular, agents for the prevention or treatment of inflammatory pain and/or agents for the prevention or treatment of neuropathic pain.

### ADVANTAGEOUS EFFECTS OF INVENTION

The present invention provides the compounds represented by the formula (I) bearing a novel arylurea moiety or pharmaceutically acceptable salts thereof, or solvates thereof, and the pharmaceutical compositions comprising these derivatives as an active ingredient. The compounds in a preferred embodiment of the invention are the FAAH inhibitors.

The pharmaceutical compositions comprising the compounds of the present invention as an active ingredient are expected as orally available agents for preventing or treating pain, in particular, as agents for preventing or treating neuropathic pain or fibromyalgia syndrome, agents for preventing or treating inflammatory pain or agents for preventing or treating cancer pain, and as agents for preventing or treating pain associated with multiple sclerosis. A group of the compounds which are particularly preferred in the present invention also possess a highly selective FAAH inhibitory activity and hence provides high usefulness.

### DESCRIPTION OF EMBODIMENTS

The present invention provides the novel arylurea derivatives represented by formula (I) in the following embodiments, salts thereof or solvates thereof, and pharmaceutical compositions comprising the same as an active ingredient as well as medical use of the derivatives or salts thereof.

### [1] Embodiment 1 of the Invention

A first embodiment of the invention is the compound represented by formula (I) below, a pharmaceutically acceptable salt thereof, or a solvate thereof: 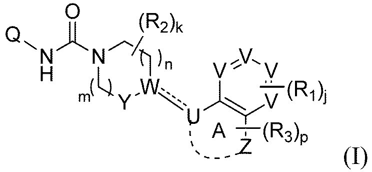 (wherein:
U represents C, CH or N, V may be the same or different and each independently represents CH or N, W represents C, CH or N, Y represents CH₂ or C=O, and Z represents O (oxygen atom) or NR₄, wherein when U is CH, the hydrogen atom bonded to the carbon atom may be substituted with R₃, when W is CH or Y is CH₂, 1 or 2 of the hydrogen atoms bonded to the carbon atoms may be substituted with R₂; j represents an integer of 0 to 3, k represents an integer of 0 to 2, m represents an integer of 0 to 2, n represents an integer of 0 to 2, and p represents an integer of 0 to 4;
Q represents a (6- to 10-membered ring) aryl or a (5- to 12-membered ring) heteroaryl, which may be substituted with 1 to 4 substituents optionally selected from substituent group T;
the substituent group T consists of the groups below:
1) a halogen;
2) a -(C₁-C₆)-alkyl, a -(C₂-C₆)-alkenyl or a -(C₂-C₆)-alkynyl, wherein the alkyl, alkenyl or alkynyl is unsubstituted or each independently may be substituted with 1 to 5 substituents optionally selected from =O (oxo), a halogen, a -(C₁-C₆)-haloalkyl, an -OR₅ or an -N(R₅)(R₆);
3) a -(C₁-C₆)-haloalkyl;
4) -OH;
5) an -O-(C₁-C₆)-alkyl, wherein the alkyl is unsubstituted or each independently may be substituted with 1 to 3 substituents from a halogen or a -(C₁-C₆)-haloalkyl;
6) -CN;
7) a -C(O)-N(R₅)(R₆);
8) a -(C₁-C₆)-alkyl-OR₅;
9) a -(3- to 12-membered ring) heteroalicyclic;
10) an -N(R₅)(R₄); and,
11) an -N(R₅)-C(O)-R₆;
ring A in formula (I): 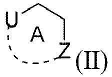 is a 5- to 7-membered ring, the dotted line in the ring A is a linkage having 1 to 3 atoms selected from carbon, nitrogen, oxygen and sulfur atoms, wherein the linkage may be saturated bonds or may partially contain unsaturated bonds;
the bond between W and U: 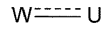
represents N-CH, N-C, CH-N, CH-CH, CH-C or C=C;
R₁ may be the same or different and each independently represents a group optionally selected from the groups below:
1) a halogen;
2) a -(C₁-C₆)-alkyl, a -(C₂-C₆)-alkenyl or a -(C₂-C₆)-alkynyl, wherein the alkyl, alkenyl or alkynyl is unsubstituted or each independently may be substituted with 1 to 5 substituents optionally selected from =O (oxo), a halogen, a -(C₁-C₆)-haloalkyl, an -OR₅ or an -N(R₅)(R₆);
3) a -(C₁-C₆)-haloalkyl;
4) -OH;
5) an -O-(C₁-C₆)-alkyl, wherein the alkyl is unsubstituted or each independently may be substituted with 1 to 3 substituents selected from a halogen and a -(C₁-C₆)-haloalkyl;
6) -CN;
7) an -N(R₅)(R₆);
8) a -C(O)-N(R₅)(R₆);
9) an -N(R₅)-C(O)-R₆;
10) an -N(R₅)-SO₂-R₆;
11) an -SO₂-(C₁-C₆)-alkyl;
12) an -SO₂-N(R₅)(R₆);
13) an -SO₂-(C₁-C₆)-haloalkyl;
14) an -S-(C₁-C₆)-alkyl;
15) an -S-(C₁-C₆)-haloalkyl;
16) a -(C₁-C₆)-alkyl-N(R₅)(R₆);
17) an -N(R₅)-C(O)-N(R₅)(R₆);
18) an -N(R₅)-C(O)-OR₅;
19) a -(C₁-C₆)-alkyl-OR₅;
20) a -(3- to 12-membered ring) heteroalicyclic;
21) a -(6- to 10-membered ring) aryl;
22) a -(5- to 12-membered ring) heteroaryl;
23) an -O-(6- to 10-membered ring) aryl;
24) an -O-(5- to 12-membered ring) heteroaryl;
25) a -C(O)-(6- to 10-membered ring) aryl;
26) a -C(O)-(5- to 12-membered ring) heteroaryl;
27) an -SO₂-(6- to 10-membered ring) aryl;
28) an -SO₂-(5- to 12-membered ring) heteroaryl;
29) an -N(R₅)-(CO)-(6- to 10-membered ring) aryl; and,
30) an -N(R₅)-(CO)-(5- to 12-membered ring) heteroaryl;
wherein each of the (6- to 10-membered ring) aryl and the (5- to 12-membered ring) heteroaryl in 21) the -(6- to 10-membered ring) aryl, 22) the -(5-to 12-membered ring) heteroaryl, 23) the -O-(6- to 10-membered ring) aryl, 24) the -O-(5- to 12-membered ring) heteroaryl, 25) the -C(O)-(6- to 10-membered ring) aryl, 26) the -C(O)-(5- to 12-membered ring) heteroaryl, 27) the -SO₂-(6- to 10-membered ring) aryl, 28) the -SO₂-(5- to 12-membered ring) heteroaryl, 29) the -N(R₅)-(CO)-(6-to 10-membered ring) aryl and 30) the -N(R₅)-(CO)-(5- to 12-membered ring) heteroaryl may be substituted with 1 to 4 substituents optionally selected from the substituent groups 1) to 20) for R₁;
R₂ may be the same or different and is selected from a -(C₁-C₆)-alkyl, a -(C₂-C₆)-alkenyl, a -(C₂-C₆)-alkynyl, -OH and an -O-(C₁-C₆)-alkyl, wherein the -(C₁-C₆)-alkyl, -(C₂-C₆)-alkenyl or -(C₂-C₆)-alkynyl is unsubstituted or each independently may be substituted with 1 to 5 substituents selected from =O (oxo), a -(C₁-C₆)-haloalkyl, an -OR₅ and an N(R₅)(R₆), and said R₂ may combine with each other to form a 3- to 6-membered carbon ring;
R₃ represents a group optionally selected from the groups below:
1) a -(C₁-C₆)-alkyl, a -(C₂-C₆)-alkenyl or a -(C₂-C₆)-alkynyl, wherein said alkyl, alkenyl or alkynyl is unsubstituted or each independently may be substituted with 1 to 5 substituents optionally selected from =O (oxo), a halogen, a -(C₁-C₆)-haloalkyl, an -OR₅ or an -N(R₅)(R₆);
2) -OH;
3) a -C(O)-N(R₅)(R₆);
4) an -N(R₅)-C(O)-R₆;
5) an -N(R₅)-SO₂-R₆;
6) an -N(R₅)-C(O)-N(R₅)(R₆);
7) an -N(R₅)-(CO)-(6- to 10-membered ring) aryl; and,
8) an -N(R₅)-(CO)- (5- to 12-membered ring) heteroaryl;
wherein the (6- to 10-membered ring) aryl and (5- to 12-membered ring) heteroaryl in 7) the -NR₅-(CO)-(6- to 10-membered ring) aryl and 8) the -NR₅-(CO)-(5- to 12-membered ring) heteroaryl each may be substituted with 1 to 4 substituents optionally selected from the substituent groups 1) to 21) for R₁;
R₄ represents a group optionally selected from the groups below:
1) hydrogen atom;
2) a -(C₁-C₆)-alkyl, a -(C₁-C₆)-alkenyl or a -(C₁-C₆)-alkynyl, wherein the alkyl, alkenyl or alkynyl is unsubstituted or each independently may be substituted with 1 to 5 substituents optionally selected from =O (oxo), a halogen, a -(C₁-C₆)-haloalkyl, an -OR₅ and an -N(R₅)(R₆);
3) a -C(O)-N(R₅)(R₆);
4) an -SO₂-(C₁-C₆)-alkyl;
5) a -(3- to 12-membered ring) heteroalicyclic;
6) a -(6- to 10-membered ring) aryl;
7) a -(5- to 12-membered ring) heteroaryl;
8) a -C(O)-(6- to 10-membered ring) aryl;
9) a -C(O)-(5- to 12-membered ring) heteroaryl;
10) an -SO₂-(6- to 10-membered ring) aryl;
11) an -SO₂-(5- to 12-membered ring) heteroaryl; and,
12) a -C(O) -(C₁-C₆)-alkyl; and,
R₅ and R₆ each independently represents an atom or a group selected from hydrogen atom, -(C₃-C₈)-cycloalkyl and -(C₁-C₆)-alkyl, wherein the -(C₁-C₆)-alkyl independently may be substituted with 1 to 5 substituents selected from -OH, -O-(C₁-C₆)-alkyl, -(C₁-C₆)-haloalkyl and -O-(C₁-C₆)-haloalkyl).

The respective groups in formula (I) above in the embodiment [1] described above are specifically described below.

In the description related to the compounds of the present invention, for example, the term "C₁-C₆" is used to mean, unless otherwise stated, a linear, branched or cyclic group having 1 to 6 carbon atoms as carbon atoms constituting a ring, and includes a linear or branched group substituted with a cyclic group, or a cyclic group substituted with a linear or branched group.

The term "(6- to 10-membered) aryl" is used to mean a monocyclic or fused cyclic (6- to 10-membered) aryl group and includes, for example, phenyl, 1-naphthyl, 2-naphthyl, etc., or a partially hydrogenated fused aryl group such as indanyl, indenyl, tetrahydronaphthyl, etc. As used herein, the partially hydrogenated aryl group represents a monovalent group obtained by removing an optional hydrogen atom from the partially hydrogenated fused ring. Either the hydrogen atom(s) on the aromatic moiety or the hydrogen atom(s) on the hydrogenated moiety in the fused ring may be removed. The group includes, for example, 1,2,3,4-tetrahydronaphthalen- (-1-yl, -2-yl, -3-yl, -4-yl, -5-yl, -6-yl, -7-yl, -8-yl), etc. for tetrahydronaphthyls.

The term "(5- to 12-membered) heteroaryl" includes a monocyclic or fused cyclic group. The monocyclic heteroaryl group contains preferably a 5- to 7-membered ring, and more preferably a 5- or 6-membered ring. Examples thereof include pyrrolyl, furyl, thienyl, imidazolyl, pyrazolyl, oxazolyl, isoxazolyl, thiazolyl, isothiazolyl, 1,2,3-triazolyl, 1,2,4-triazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, tetrazolyl, pyridyl, pyridazinyl, pyrimidinyl, pyrazinyl, 1,2,3-triazinyl, 1,2,4-triazinyl, 1,3,5-triazinyl, 2H-1,2,3-thiadiazinyl, 4H-1,2,4-thiadiazinyl, 6H-1,3,4-thiadiazinyl, 1,4-diazepinyl, 1,4-oxazepinyl, etc.

Preferably, the fused cyclic heteroaryl group contains a 8- to 12-membered ring. This group includes, for example, a monovalent group obtained by removing an optional hydrogen atom from a fused ring formed by fusing the above 5- to 7-membered hetero ring to a monocyclic aryl group (e.g., benzene ring, etc.) or a monocyclic heteroaryl group, etc. The optional hydrogen atom(s) may be removed from any ring of the fused ring(s).

Specific examples of the fused cyclic heteroaryl groups include indolyl (-7-yl), isoindolyl, benzofuranyl, isobenzofuranyl, benzothienyl, isobenzothienyl, benzoxazolyl, 1,2-benzisoxazolyl, benzothiazolyl, 1,2-benzisothiazolyl, (1H-)benzimidazolyl, 1H-indazolyl, 1H-benzotriazolyl, 2,1,3-benzothiadiazinyl, chromenyl (2H-chromenyl), isochromenyl(1H-isochromenyl), 4H-1,4-benzoxazinyl, 4H-1,4-benzothiadinyl, quinolyl (-8-yl), isoquinolyl, cinnolinyl, quinazolinyl, quinoxalinyl, phthalazinyl, benzoxazepinyl, benzazepinyl, benzodiazepinyl, naphthylizinyl, purinyl, pteridinyl, carbazolyl, carbolinyl, acridinyl, phenoxazinyl, phenothiazinyl, phenazinyl, phenoxathiinyl, thianthrenyl, phenanthridinyl, phenanthrolinyl, indolizinyl, thieno[3,2-c]pyridyl, thiazolo[5,4-c]pyridyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,5-a]pyrimidinyl, 1,2,4-triazolo[4,3-a]pyridyl, 1,2,4-triazolo[4,3-b]pyridazinyl, 1H-pyrazolo[3,4-b]pyridyl, 1,2,4-triazolo[1,5-a]pyrimidinyl, and the like (preferred embodiments are indicated in the parenthesis).

Furthermore, examples of the fused cyclic heteroaryl groups also include a partially hydrogenated fused heteroaryl group such as indolinyl, dihydrobenzoxazonyl, dihydrobenzothiazolyl, chromanyl, isochromanyl, 3,4-dihydro-2H-1,4-benzoxazinyl, 3,4-dihydro-2H-1,4-benzothiazinyl, tetrahydroquinolyl, tetrahydroisoquinolyl, tetrahydroquinoxalinyl, 1,4-benzodioxanyl, 1,3-benzodioxolyl (-4-yl), tetrahydrobenzoxazepinyl, tetrahydrobenzazepinyl, 6,7,8,9-tetrahydro-5H-cyclohepta[b]pyridyl, etc. Preferably, the partially hydrogenated fused cyclic heteroaryl group contains a 8- to 10-membered ring and refers to a monovalent group obtained by removing an optional hydrogen atom from the ring where a fused ring is formed by fusing a 5- to 7-membered hetero ring to a monocyclic aryl group (e.g., a benzene ring, etc.) or to a monocyclic heteroaryl group and partially hydrogenated, in which either the hydrogen atom(s) of the aryl group or heterocyclic moiety or the hydrogen atom(s) of the hydrogenated moiety may be removed. In the case of, e.g., tetrahydroquinolyls, the group includes 5,6,7,8-tetrahydroquinolyl, 1,2,3,4-tetrahydroquinolyl, etc. , Depending on positions at which optional hydrogen atom(s) may be removed, these groups include, for example, -2-yl, -3-yl, -4-yl, -5-yl, -6-yl, -7-yl, -8-yl, etc. for 5,6,7,8-tetrahydroquinolyls, and 1-yl, -2-yl, -3-yl, -4-yl, -5-yl, -6-yl, -7-yl, -8-yl, etc. for 1,2,3,4-tetrahydroquinolyls.

Examples of "halogen" include fluorine atom, chlorine atom, bromine atom and iodine atom.

The "(C₁-C₆)-alkyl" is a C₁-C₆ linear, branched or cyclic alkyl group, including, e.g., methyl, ethyl, propyl, isopropyl, n-butyl, isobutyl, sec-butyl, tert-butyl, pentyl, isopentyl, neopentyl, tert-pentyl, 1-methylbutyl, 2-methylbutyl, 1,2-dimethylpropyl, 1-ethylpropyl, hexyl, isohexyl, 1-methylpentyl, 2-methylpentyl, 3-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, cyclopropyl, cyclobutyl, cyclopentyl, cyclohexyl, cyclopropylmethyl, cyclobutylmethyl, cyclopentylmethyl, 1-cyclopropylethyl, 2-cyclopropylethyl, 2-cyclobutylethyl, 2-methylcyclopropyl, and the like.

The "(C₃-C₈)-cycloalkyl" includes a C₃-C₈ cyclic alkyl group, e.g., cyclopropyl, cyclobutyl, cyclopentyl, cyclohexyl, cycloheptyl, cyclooctyl, cyclopropylmethyl, cyclobutylmethyl, cyclopentylmethyl, 1-cyclopropylethyl, 2-cyclopropylethyl, 2-cyclobutylethyl, 2-methylcyclopropyl, and the like.

The "(C₂-C₆)-alkenyl" includes a C₂-C₆ linear, branched or cyclic alkenyl group, e.g., vinyl, allyl, isopropenyl, 2-methylallyl, butenyl, pentenyl, hexenyl, 1-cyclopropen-1-yl, 2-cyclopropen-1-yl, 1-cyclobuten-1-yl, 1-cyclopenten-1-yl, 2-cyclopenten-l-yl, 3-cyclopenten-1-yl, 1-cyclohexen-1-yl, 2-cyclohexen-1-yl, 3-cyclohexen-1-yl, 2,4-cyclopentadien-1-yl, 2,5-cyclohexadien-1-yl, and the like.

The "(C₂-C₆)-alkynyl" is a C₂-C₆ linear, branched or cyclic alkenyl group, including, e.g., ethynyl, 1-propynyl, 2-propynyl, butynyl, pentynyl, hexynyl, and the like.

The "(C₁-C₆)-haloalkyl" represents a group wherein the "(C₁-C₆)-alkyl" described above is optionally substituted with 1 to 6 halogen atoms, and includes, e.g., trifluoromethyl, trifluoromethylmethyl, trifluoroethyl, tetrafluoroethyl, pentafluoroethyl, and the like.

In the "-OR₅," R₅ is selected from hydrogen atom, a -(C₃-C₈)-cycloalkyl and a -(C₁-C₆)-alkyl, wherein the -(C₁-C₆)-alkyl each independently may be substituted with 1 to 5 groups selected from -OH, an -O-(C₁-C₆)-alkyl, a -(C₁-C₆)-haloalkyl and an -O-(C₁-C₆)-haloalkyl; examples of -OR₅ are hydroxy, methoxy, and the like.

In the "-N(R₅)(R₆)", R₅ and R₆ each independently is selected from hydrogen atom, a -(C₃-C₈)-cycloalkyl and a -(C₁-C₆)-alkyl, wherein the -(C₁-C₆)-alkyl each independently may be substituted with 1 to 5 groups selected from -OH, an -O-(C₁-C₆)-alkyl, a -(C₁-C₆)-haloalkyl and an -O-(C₁-C₆)-haloalkyl. Examples of the "-N(R₅)(R₆)" are amino, a "mono/di(C₁-C₆)-alkylamino", a "halogenated mono/di(C₁-C₆)-alkylamino," a "-(C₁-C₆)-amino," and the like.

The "mono/di(C₁-C₆)-alkylamino" means an amino group wherein one or two hydrogen atoms in the amino group is/are substituted with a linear, branched or cyclic "-(C₁-C₆)-alkyl group." Specific examples include methylamino, ethylamino, propylamino, isopropylamino, butylamino, isobutylamino, pentylamino, isopentylamino, hexylamino, isohexylamino, cyclopropylamino, cyclobutylamino, cyclopentylamino, cyclohexylamino, 1-cyclopropylmethylamino, 1-cyclobutylmethylamino, 1-cyclopentylmethylamino, 1-cyclohexylmethylamino, dimethylamino, diethylamino, dipropylamino, diisopropylamino, dibutylamino, dipentylamino, ethylmethylamino, methylpropylamino, ethylpropylamino, butylmethylamino, butylethylamino, butylpropylamino, N-cyclopropyl-N-methylamino, N-cyclobutyl-N-methylamino, N-cyclopentyl-N-methylamino, N-cyclohexyl-N-methylamino, and the like.

The "(C₁-C₆)-amino" refers to a group wherein the "(C₁-C₆)-alkyl" described above is bonded to an amino group, and includes, e.g., methylamino, cyclopropylamino, cyclohexylamino group, and the like.

The "halogenated mono/di(C₁-C₆)-alkylamino" represents a group wherein the "mono/di(C₁-C₆)-alkylamino" described above is/are optionally substituted with 1 to 5 halogen atoms, and includes, e.g., trifluoromethylamino, and the like.

The "-O-(C₁-C₆)-alkyl" refers to a C₁-C₆ linear, branched or cyclic alkoxyl group, and includes, e.g., methoxy, ethoxy, propoxy, isopropoxy, butoxy, isobutoxy, sec-butoxy, tert-butoxy, pentyloxy, isopentyloxy, neopentyloxy, tert-pentyloxy, 1-methylbutoxy, 2-methylbutoxy, 1,2-dimethylpropoxy, 1-ethylpropoxy, hexyloxy, isohexyloxy, 3,3-dimethylbutoxy, cyclopropyloxy, cyclobutyloxy, cyclopentyloxy, cyclohexyloxy, cyclopropylmethoxy, 1-cyclopropylethoxy, 2-cyclopropylethoxy, cyclobutylmethoxy, 2-cyclobutylethoxy, cyclopentylmethoxy, 2-methylcyclopropyloxy, and the like.

The "-O-(C₁-C₆)-haloalkyl" represents a group in which the (C₁-C₆)-haloalkyl group described above is bonded to oxygen atom, and includes, e.g., trifluoromethoxy, trifluoroethoxy, tetrafluoroethoxy, pentafluoroethoxy, and the like.

The "-C(O)-N(R₅)(R₆)" represents a group in which carbonyl is bonded to the -N(R₅)(R₆) described above, and includes, e.g., dimethylaminocarbonyl, and the like.

The "-(C₁-C₆)-alkyl-OR₅" represents a group wherein the -OR₅ described above is bonded to the "-(C₁-C₆)-alkyl described above, and includes, e.g., methoxyethyl, etc.

The "(3- to 12-membered ring) heteroalicyclic" represents a saturated or unsaturated non-aromatic (3- to 12-membered ring) heterocyclic group, and more specifically, means a monovalent group formed by removing an optional hydrogen atom from the rings having a mono ring or a fused ring of a 3- to 8-membered ring, preferably a 3- to 7-membered ring, and more preferably a 5- to 6-membered ring, which contain at least one hetero atom (preferably 1 to 4) optionally selected from N, O and S other than carbon atoms, and includes, e.g., aziridinyl, azetidinyl, oxiranyl, oxetanyl, thietanyl, pyrrolidinyl, tetrahydrofuryl, thioranyl, pyrazolinyl, pyrazolidinyl, piperidyl, tetrahydropyranyl, piperazinyl, morpholinyl, oxazolinyl, thiazolinyl, thiomorpholinyl, quinuclidinyl, oxepanyl, and the like.

The "-N(R₅)-C(O)-R₆" represents an amino group wherein hydrogen atoms on the amino group are substituted with the "R₅" and "-C(O)-R₆" described above, e.g., "hydrogen atom" as "R₅" and "-C(O)-(C₁-C₆)-alkyl" as "-C(O)-R₆," and specifically includes acetamido, propionamido, butylamido, isobutylamido, valeramido, isovaleramido, pivalamido, hexanamido, heptanamido, cyclopropanecarboxamido, cyclobutanecarboxamido, cyclopentanecarboxamido, cyclohexanecarboxamido, 4-methylcyclohexanecarboxamido, and the like.

The "-N(R₅)-SO₂-R₆" represents a group formed by bonding the -(C₁-C₆)-alkyl and NR₅ via sulfonyl when R₆ is -(C₁-C₆)-alkyl, and by bonding NR₅ and sulfonic acid when R₆ is OH, and includes, e.g., methanesulfonylamino group, and the like.

The "-SO₂-(C₁-C₆)-alkyl" represents a group wherein the -(C₁-C₆)-alkyl described above is bonded to sulfonyl, and includes, e.g., methanesulfonyl.

The "-SO₂-N(R₅)(R₆)" represents a group wherein the N(R₅)(R₆) described above is bonded to sulfonyl, and includes, e.g., dimethylaminosulfonyl.

The "-SO₂-(C₁-C₆)-haloalkyl" represents a group wherein the (C₁-C₆)-haloalkyl described above is bonded to sulfonyl, and includes, e.g., trifluoromethanesulfonyl.

The "-S-(C₁-C₆)-alkyl" represents a linear, branched or cyclic -(C₁-C₆)-alkylthio having 1 to 6 carbon atoms, and includes, e.g., methylthio, ethylthio, propylthio, isopropylthio, butylthio, isobutylthio, sec-butylthio, tert-butylthio, pentylthio, isopentylthio, tert-pentylthio, neopentylthio, 1-methylbutylthio, 2-methylbutylthio, 1,2-dimethylpropylthio, 1-ethylpropylthio, hexylthio, isohexylthio, 3,3-dimethylbutylthio, cyclopropylthio, cyclobutylthio, cyclopentylthio, cyclohexylthio, cyclopropylmethylthio, 1-cyclopropylethylthio, 2-cyclopropylethylthio, cyclobutylmethylthio, 2-cyclobutylethylthio, cyclopentylmethylthio, 2-methylcyclopropylthio, and the like.

The "-S-(C₁-C₆)- haloalkyl" represents a group wherein the "-(C₁-C₆)-alkylthio" described above is optionally substituted with 1 to 5 halogen atoms, and includes, e.g., trifluoromethylthio, and the like.

The "-(C₁-C₆)-alkyl-N(R₅)(R₆)" represents a group wherein the N(R₅)(R₆) described above is bonded to the -(C₁-C₆)-alkyl described above, and includes, e.g., dimethylaminopropyl.

The "-N(R₅)-C(O)-N(R₅)(R₆)" represents a group wherein R₅ bonded to N each is independent and the -N(R₅)(R₆) is bonded to the -N(R₅)(R₆) described above via carbonyl, and includes, e.g., dimethylaminocarbonylamino.

The "-N(R₅)-C(O)-OR₅" represents a group wherein the OR₅ described above is bonded to -N(R₅) via carbonyl, and includes, e.g., methoxycarbonylamino group, and the like.

The "-(C₁-C₆)-alkyl-OR₅" represents a group wherein the OR₅ described above is bonded to the -(C₁-C₆)-alkyl described above, and includes, e.g., methoxyethyl.

The "-SO₂-(6- to 10-membered ring) aryl" represents a group wherein the (6- to 10-membered ring) aryl described above is bonded to sulfonyl, and includes, e.g., benzenesulfonyl, and the like.

The "-SO₂-(5- to 12-membered ring) heteroaryl" represents a group wherein the (5- to 12-membered ring) heteroaryl described above is bonded to sulfonyl, and includes, e.g., pyridinesulfonyl, and the like.

The "-N(R₅)-(CO)-(6- to 10-membered ring) aryl" represents a group wherein the (6- to 10-membered ring) aryl described above is bonded to -N(R₅) via carbonyl, and includes, e.g., benzoylamino, and the like.

The "-N(R₅)-(CO)-(5- to 12-membered ring) heteroaryl" represents a group wherein the (5- to 12-membered ring) heteroaryl described above is bonded to -N(R₅) via carbonyl, and includes, e.g., pyridinoylamino, and the like.

The "-O-(6- to 10-membered ring) aryl" represents a group wherein the "aryl" described above is substituted with oxygen atom(s), and includes, e.g., phenoxy, 1-naphthyloxy, 2-naphthyloxy, 2-anthryloxy, phenanthryloxy, 1,2,3,4-tetrahydronaphthalen(8-yloxy), and the like.

The "-O-(5- to 12-membered ring) heteroaryl" represents a group wherein the "heteroaryl" described above is substituted with oxygen atom(s), and includes, e.g., pyrrolyloxy, furyloxy, thienyloxy, imidazolyloxy, pyrazolyloxy, oxazolyloxy, isoxazolyloxy, thiazolyloxy, isothiazolyloxy, pyridyloxy, pyridazinyloxy, pyrimidinyloxy, pyrazinyloxy, indolyloxy, quinolyloxy, isoquinolyloxy, indolinyloxy, 1,2,3,4-tetrahydroquinolyloxy, 1,3-benzodioxolyloxy, and the like.

The "-C(O)-(6- to 10-membered ring) aryl" represents a group wherein carbonyl is bonded to the aryl described above, and includes a (6- to 10-membered ring) arylcarbonyl, e.g., benzoyl, naphthylcarbonyl, etc.

The "-C(O)-(5- to 12-membered ring) heteroaryl" represents a group wherein carbonyl is bonded to the heteroaryl described above, and includes:
a carbonyl having a "monocyclic heteroaryl" bonded thereto, such as pyrrolylcarbonyl, furylcarbonyl, thienylcarbonyl, imidazolylcarbonyl, pyrazolylcarbonyl, oxazolylcarbonyl, isoxazolylcarbonyl, thiazolylcarbonyl, isothiazolylcarbonyl, 1,2,3-triazolylcarbonyl, 1,2,4-triazolylcarbonyl, 1,2,3-oxadiazolylcarbonyl, 1,2,4-oxadiazolylcarbonyl, 1,3,4-oxadiazolylcarbonyl, furazanylcarbonyl, 1,2,3-thiadiazolylcarbonyl, 1,2,4-thiadiazolylcarbonyl, 1,3,4-thiadiazolylcarbonyl, tetrazolylcarbonyl, pyridylcarbonyl, pyridazinylcarbonyl, pyrimidinylcarbonyl, pyrazinylcarbonyl, 1,2,3-triazinylcarbonyl, 1,2,4-triazinylcarbonyl, 1,3,5-triazinylcarbonyl, 2H-1,2,3-thiadiazinylcarbonyl, 4H-1,2,4-thiadiazinylcarbonyl, 6H-1,3,4-thiadiazinylcarbonyl, 1,4-diazepinylcarbonyl, 1,4-oxazepinylcarbonyl, etc.; and
a carbonyl having a "fused ring heteroaryl" bonded thereto which may be partially hydrogenated, such as indolylcarbonyl, isoindolylcarbonyl, benzofuranylcarbonyl, isobenzofuranylcarbonyl, benzothienylcarbonyl, isobenzothienylcarbonyl, benzoxazolylcarbonyl, 1,2-benzisoxazolylcarbonyl, benzothiazolylcarbonyl, 1,2-benzisothiazolylcarbonyl, (1H-)benzimidazolylcarbonyl, 1H-indazolylcarbonyl, 1H-benzotriazolylcarbonyl, 2,1,3-benzothiadiazinylcarbonyl, chromenylcarbonyl, isochromenylcarbonyl, 4H-1,4-benzoxazinylcarbonyl, 4H-1,4-benzothiazinylcarbonyl, quinolylcarbonyl, isoquinolylcarbonyl, cinnolinylcarbonyl, quinazolinylcarbonyl, quinoxalinylcarbonyl, phthalazinylcarbonyl, benzoxazepinylcarbonyl, benzazepinylcarbonyl, benzodiazepinylcarbonyl, naphthyridinylcarbonyl, purinylcarbonyl, puteridinylcarbonyl, carbazolylcarbonyl, carbolinylcarbonyl, acridinylcarbonyl, phenoxazinylcarbonyl, phenothiazinylcarbonyl, phenazinylcarbonyl, phenoxathiinylcarbonyl, thianthrenylcarbonyl, phenanthridinylcarbonyl, phenanthrolinylcarbonyl, indolizinylcarbonyl, thieno[3,2-c]pyridylcarbonyl, thiazolo[5,4-c]pyridylcarbonyl, pyrrolo[1,2-b]pyridazinylcarbonyl, pyrazolo[1,5-a]pyridylcarbonyl, imidazo[1,2-a]pyridylcarbonyl, imidazo[1,5-a]pyridylcarbonyl, imidazo[1,2-b]pyridazinylcarbonyl, imidazo[1,5-a]pyrimidinylcarbonyl, 1,2,4-triazolo[4,3-a]pyridylcarbonyl, 1,2,4-triazolo[4,3-b]pyridazinylcarbonyl, 1H-pyrazolo[3,4-b]pyridylcarbonyl, 1,2,4-triazolo[1,5-a]pyrimidinylcarbonyl, indolinylcarbonyl, dihydrobenzoxazolylcarbonyl, chromanylcarbonyl, tetrahydroquinolylcarbonyl, 1,4-benzodioxanylcarbonyl, 1,3-benzodioxolylcarbonyl, etc.

The "-N(R₅)-(CO)-(6- to 10-membered ring) aryl" represents an amino group wherein a hydrogen atom on the amino group is substituted with the "-(CO)-(6- to 10-membered ring) aryl" described above, and includes specifically a (6- to 10-membered ring) arylcarbonylamino such as benzamide, naphthamide, etc.

The "-N(R₅)-(CO)-(5- to 12-membered ring) heteroaryl" represents an amino group wherein hydrogen atoms on the amino group is substituted with the "R₅" and "-(CO)-(5- to 12-membered ring) heteroaryl" described above, and includes specifically a heterocyclic carbonylamino such as pyrrolecarboxamido, furancarboxamido, thiophenecarboxamido, imidazolecarboxamido, pyrazolecarboxamido, pyridinecarboxamido, indolecarboxamido, quinolinecarboxamido, piperidinecarboxamido, etc.

The "-(C₁-C₆)-alkyl, -(C₂-C₆)-alkenyl or -(C₂-C₆)-alkynyl, wherein the alkyl, alkenyl or alkynyl is unsubstituted or each independently may be substituted with 1 to 5 substituents optionally selected from =O (oxo), a halogen, a -(C₁-C₆)-haloalkyl, an -OR₅ or an -N(R₅)(R₆)" represents, in addition to the "-(C₁-C₆)-alkyl", "-(C₂-C₆)-alkenyl" or "-(C₂-C₆)-alkynyl" described above, a group wherein the alkyl, alkenyl or alkynyl each independently may be substituted with 1 to 5 substituents optionally selected from =O (oxo), a halogen, a -(C₁-C₆)-haloalkyl, an -OR₅ or an -N(R₅)(R₆), and includes, e.g., a "-(C₁-C₆)-alkyl which may be substituted with 1 to 5 halogen substituents," etc. In addition to methyl, ethyl, propyl, isopropyl, butyl, isobutyl, sec-butyl and tert-butyl, specific examples are trifluoromethyl, trifluoroethyl, tetrafluoroethyl, pentafluoroethyl, etc.

The "-(C₁-C₆)-alkyl, each of which independently may be substituted with 1 to 5 substituents selected from -OH, -O-(C₁-C₆)-alkyl, -(C₁-C₆)-haloalkyl and -O-(C₁-C₆)-haloalkyl" represents, in addition to the "(C₁-C₆)-alkyl" described above, a group wherein the alkyl may optionally be substituted with 1 to 5 substituents selected from -OH, an -O-(C₁-C₆)-alkyl, a -(C₁-C₆)-haloalkyl and an -O-(C₁-C₆)-haloalkyl, and includes, more specifically, hydroxypropyl, methoxyethyl, 2,2,2-trifluoroethyl, trifluoromethoxyethyl, and the like.

The "-O-(C₁-C₆)-alkyl, each of which independently may be substituted with 1 to 3 substituents selected from a halogen and a -(C₁-C₆)-haloalkyl" represents, in addition to the "-O-(C₁-C₆)-alkyl" described above, a group wherein the alkyl may optionally be substituted with 1 to 3 substituents selected from a halogen or a -(C₁-C₆)-haloalkyl, and includes, more specifically, trifluoromethoxy, 2,2,2-trifluoroethoxy, and the like.

The "(6- to 10-membered ring) aryl or (5- to 12-membered ring) heteroaryl in 21) the -(6- to 10-membered ring) aryl, 22) the -(5- to 12-membered ring) heteroaryl, 23) the -O-(6- to 10-membered ring) aryl, 24) the -O-(5- to 12-membered ring) heteroaryl, 25) the -C(O)-(6- to 10-membered ring) aryl, 26) the -C(O)-(5- to 12-membered ring) heteroaryl, 27) the -SO₂-(6 to 10-membered ring) aryl, 28) the -SO₂-(5- to 12-membered ring) heteroaryl, 29) the -N(R₅)-(CO)-(6- to 10-membered ring) aryl and 30) the -N(R₅)-(CO)-(5- to 12-membered ring) heteroaryl, which may be substituted with 1 to 4 substituents optionally selected from each of the substituent groups 1) to 20)" represents, in addition to the "(6- to 10-membered ring) aryl" or "(5- to 12-membered ring) heteroaryl," a group wherein the "(6- to 10-membered ring) aryl" or "(5- to 12-membered ring) heteroaryl" may optionally be substituted with 1 to 4 substituents optionally selected from the substituent groups 1) to 20); and includes, more specifically, 5-trifluoromethyl-2-pyridinyl, 5-trifluoromethyl-2-pyridinyloxy, 5-trifluoromethyl-2-pyridinylcarboxy, and the like.

The "(6- to 10-membered ring) aryl or (5- to 12-membered ring) heteroaryl in the 7) -NR₅-(CO)-(6- to 10-membered ring) aryl and 8) -NR₅-(CO)-(5- to 12-membered ring) heteroaryl, which may be substituted with 1 to 4 substituents optionally selected from each of the substituent groups 1) to 21) for R₁" represents, in addition to these "(6- to 10-membered ring) aryl" or "(5- to 12-membered ring) heteroaryl," a group which may optionally be substituted with 1 to 4 substituents optionally selected from the substituent groups 1) to 21) shown in R₁; and includes, more specifically, 4-chlorobenzoylamino,
5-trifluoromethyl-2-pyridinylcarbonylamino, and the like.

### [1-1-a]

In the compound of formula (I) used in the embodiment [1] above, W represents C, CH or N, and preferably N.

### [1-1-b]

In the compound of formula (I) used in the embodiment [1] above, V represents CH or N, and preferably CH.

### [1-1-c]

In the compound of formula (I) used in the embodiment [1] above, U represents C, CH or N, preferably C or CH, and more preferably CH.

### [1-2]

In the compound of formula (I) used in the embodiment [1] above, Y represents CH₂ or C=O, and preferably CH₂.

### [1-3]

In the compound of formula (I) used in the embodiment [1] above, j represents an integer of 0 to 3, preferably an integer of 0 to 2, and more preferably an integer of 1 to 2.

### [1-3-a]

In the compound of formula (I) used in the embodiment [1] above, k represents an integer of 0 to 2, and preferably 0.

### [1-3-b]

In the compound of formula (I) used in the embodiment [1] above, m represents an integer of 0 to 2, and preferably 1.

### [1-3-c]

In the compound of formula (I) used in the embodiment [1] above, n represents an integer of 0 to 2, preferably 1.

### [1-3-d]

In the compound of formula (I) used in the embodiment [1] above, p represents an integer of 0 to 4, and preferably 0.

### [1-3-e]

In the compound of formula (I) used in the embodiment [1] above, the bond between W and U is shown by:
W 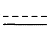 U
which represents N-CH, N-C, CH-N, CH-CH, CH-C or C=C, preferably N-CH, CH-N, CH-CH, CH-C or C=C, and more preferably N-CH, CH-CH or C=C.

### [1-4]

In the compound of formula (I) used in the embodiment [1] above, Q represents a (6- to 10-membered ring) aryl or a (5- to 12-membered ring) heteroaryl, which may be substituted with 1 to 4 substituents optionally selected from the substituent group T; preferably a (5- to 12-membered ring) heteroaryl which may be substituted with 1 to 4 substituents optionally selected from the substituent group T, more preferably a 6-membered heteroaryl which may be substituted with 1 to 4 substituents optionally selected from the substituent group T, and most preferably a 6-membered heteroaryl which may be substituted with one substituent optionally selected from the substituent group T.

### [1-5]

In the compound of formula (I) used in the embodiment [1] above, the substituent group T consists of the groups below, i.e.,
1) a halogen;
2) a -(C₁-C₆)-alkyl, a -(C₂-C₆)-alkenyl or a -(C₂-C₆)-alkynyl, wherein the alkyl, alkenyl or alkynyl is unsubstituted or each independently may be substituted with 1 to 5 substituents optionally selected from =O (oxo), a halogen, a -(C₁-C₆)-haloalkyl, an -OR₅ or an -N(R₅)(R₆);
3) a -(C₁-C₆)-haloalkyl;
4) -OH;
5) an -O-(C₁-C₆)-alkyl, wherein the alkyl is unsubstituted or each independently may be substituted with 1 to 3 substituents from a halogen or a -(C₁-C₆)-haloalkyl;
6) -CN;
7) a -C(O)-N(R₅)(R₆);
8) a -(C₁-C₆)-alkyl-OR₅;
9) a -(3- to 12-membered ring) heteroalicyclic;
10) an -N(R₅)(R₄); and,
11) an -N(R₅)-C(O)-R₆.

### [1-5-a]

In the compound of formula (I) used in the embodiment [1] above, the substituent group T preferably consists of the groups below, i.e.,
1) a halogen;
2) a -(C₁-C₆)-alkyl;
3) a -(C₁-C₆)-haloalkyl;
5) an -O-(C₁-C₆)-alkyl;
6) a -CN;
7) a -C(O)-N(R₅)(R₆);
8) a -(C₁-C₆)-alkyl-OR₅;
10) an -N(R₅)(R₄) and,
11) an -N(R₅)-C(O)-R₆;
and more preferably consists of:
1) a halogen;
2) a -(C₁-C₆)-alkyl;
5) an -O-(C₁-C₆)-alkyl;
10) an -N(R₅)(R₆) and,
11) an -N(R₅)-C(O)-R₆.

### [1-6]

In the compound of formula (I) above which is used in the embodiment [1] described above, ring A in formula (I): 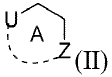 is a 5- to 7-membered ring, the dotted line in the ring A is a linkage having 1 to 3 atoms selected from carbon, nitrogen, oxygen and sulfur atoms, wherein the linkage may be saturated bonds or may partially contain unsaturated bonds; preferably ring A is a saturated 6- to 7-membered ring, and more preferably a saturated 6-membered ring.

### [1-7]

In the above compound of formula (I) used in the above embodiment [1], R₁ represents a group optionally selected from the groups below, i.e.,
1) a halogen;
2) a -(C₁-C₆)-alkyl, a -(C₂-C₆)-alkenyl or a -(C₂-C₆)-alkynyl, wherein the alkyl, alkenyl or alkynyl is unsubstituted or each independently may be substituted with 1 to 5 substituents optionally selected from =O (oxo), a halogen, a -(C₁-C₆)-haloalkyl, an -OR₅ or an -N(R₅)(R₆);
3) a -(C₁-C₆)-haloalkyl;
4) -OH;
5) an -O-(C₁-C₆)-alkyl, wherein the alkyl is unsubstituted or each independently may be substituted with 1 to 3 substituents selected from a halogen and a -(C₁-C₆)-haloalkyl;
6) -CN;
7) an -N(R₅)(R₆);
8) a -C(O)-N(R₅)(R₆);
9) an -N(R₅)-C(O)-R₆;
10) an -N(R₅)-SO₂-R₆;
11) an -SO₂-(C₁-C₆)-alkyl;
12) an -SO₂-N(R₅)(R₆);
13) an -SO₂-(C₁-C₆)-haloalkyl;
14) an -S-(C₁-C₆)-alkyl;
15) an -S-(C₁-C₆)-haloalkyl;
16) a -(C₁-C₆)-alkyl-N(R₅)(R₆);
17) an -N(R₅)-C(O)-N(R₅)(R₆);
18) an -N(R₅)-C(O)-OR₅;
19) a -(C₁-C₆)-alkyl-OR₅;
20) a -(3- to 12-membered ring) heteroalicyclic;
21) a -(6- to 10-membered ring) aryl;
22) a -(5- to 12-membered ring) heteroaryl;
23) an -O-(6- to 10-membered ring) aryl;
24) an -O-(5- to 12-membered ring) heteroaryl;
25) a -C(O)-(6- to 10-membered ring) aryl;
26) a -C(O)-(5- to 12-membered ring) heteroaryl;
27) an -SO₂-(6- to 10-membered ring) aryl;
28) an -SO₂-(5- to 12-membered ring) heteroaryl;
29) an -N(R₅)-(CO)-(6- to 10-membered ring) aryl; and,
30) an -N(R₅)-(CO)-(5- to 12-membered ring) heteroaryl;
wherein each of the (6- to 10-membered ring) aryl and the (5- to 12-membered ring) heteroaryl in 21) the -(6- to 10-membered ring) aryl, 22) the -(5-to 12-membered ring) heteroaryl, 23) the -O-(6- to 10-membered ring) aryl, 24) the -O-(5- to 12-membered ring) heteroaryl, 25) the -C(O)-(6- to 10-membered ring) aryl, 26) the -C(O)-(5- to 12-membered ring) heteroaryl, 27) the -SO₂-(6- to 10-membered ring) aryl, 28) the -SO₂-(5- to 12-membered ring) heteroaryl, 29) the -N(R₅)-(CO)-(6-to 10-membered ring) aryl and 30) the -N(R₅)-(CO)-(5- to 12-membered ring) heteroaryl may be substituted with 1 to 4 substituents optionally selected from the substituent groups 1) to 20) for R₁.

### [1-7-a]

In the above compound of formula (I) used in the above embodiment [1], R₁ preferably represents a group optionally selected from the groups below, i.e.,
1) a halogen;
2) a -(C₁-C₆)-alkyl, a -(C₂-C₆)-alkenyl or a -(C₂-C₆)-alkynyl, wherein the alkyl, alkenyl or alkynyl is unsubstituted or each independently may be substituted with 1 to 5 substituents optionally selected from =O (oxo), a halogen, a -(C₁-C₆)-haloalkyl, an -OR₅ or an -N(R₅)(R₆), and more preferably a -(C₁-C₆)-alkyl;
3) a -(C₁-C₆)-haloalkyl;
5) an -O-(C₁-C₆)-alkyl;
6) -CN;
23) an -O-(6- to 10-membered ring) aryl; and,
24) an -O-(5- to 12-membered ring) heteroaryl;
wherein each of the (6- to 10-membered)aryl or (5-12-membered)heteroaryl in the 23) -O-(6- to 10-membered ring) aryl and 24) -O-(5- to 12-membered ring) heteroaryl may be substituted with 1 to 4 substituents optionally selected from the substituent groups 1) to 3) and 5) for R₁, preferably a group substituted with one substituent; and more preferably represents a group optionally selected from the groups below, i.e.,
1) a halogen; and,
3) a -(C₁-C₆)-haloalkyl.

### [1-8]

In the above compound of formula (I) used in the above embodiment [1], R₂ may be the same or different and is selected from the groups below, i.e., a -(C₁-C₆)-alkyl, a -(C₂-C₆)-alkenyl, a -(C₂-C₆)-alkynyl, -OH or an -O-(C₁-C₆)-alkyl, and the -(C₁-C₆)-alkyl, -(C₂-C₆)-alkenyl or -(C₂-C₆)-alkynyl is unsubstituted or each independently may be substituted with 1 to 5 substituents with =O (oxo), a -(C₁-C₆)-haloalkyl, an OR₅ or an N(R₅)(R₆), or said R₂ may be combined with each other to form a 3- to 6-membered carbon ring; and preferably represents a -(C₁-C₆)-alkyl.

### [1-9]

In the compound of the above formula (I) used in the above embodiment [1], R₃ represents a group optionally selected from the groups below, i.e.,
1) a -(C₁-C₆)-alkyl, a -(C₂-C₆)-alkenyl or a -(C₂-C₆)-alkynyl, wherein said alkyl, alkenyl or alkynyl is unsubstituted or each independently may be substituted with 1 to 5 substituents optionally selected from =O (oxo), a halogen, a -(C₁-C₆)-haloalkyl, an -OR₅ or an -N(R₅)(R₆);
2) -OH;
3) a -C(O)-N(R₅)(R₆);
4) an -N(R₅)-C(O)-R₆;
5) an -N(R₅)-SO₂-R₆;
6) an -N(R₅)-C(O)-N(R₅)(R₆);
7) an -N(R₅)-(CO)-(6- to 10-membered ring) aryl; and,
8) an -N(R₅)-(CO)- (5- to 12-membered ring) heteroaryl;
wherein the (6- to 10-membered ring) aryl and (5- to 12-membered ring) heteroaryl in the 7) -NR₅-(CO)-(6- to 10-membered ring) aryl and 8) -NR₅-(CO)- (5-to 12-membered ring) heteroaryl each may be substituted with 1 to 4 substituents optionally selected from the substituent groups 1) to 21) for R₁.

### [1-10]

In the above compound of formula (I) used in the above embodiment [1], R₄ represents a group optionally selected from the groups below, i.e.,
1) hydrogen atom;
2) a -(C₁-C₆)-alkyl, a -(C₁-C₆)-alkenyl or a -(C₁-C₆)-alkynyl, wherein the alkyl, alkenyl or alkynyl is unsubstituted or each independently may be substituted with 1 to 5 substituents optionally selected from =O (oxo), a halogen, a -(C₁-C₆)-haloalkyl, an -OR₅ and an -N(R₅)(R₆);
3) a -C(O)-N(R₅)(R₆);
4) an -SO₂-(C₁-C₆)-alkyl;
5) a -(3- to 12-membered ring) heteroalicyclic;
6) a -(6- to 10-membered ring) aryl;
7) a -(5- to 12-membered ring) heteroaryl;
8) a -C(O)-(6- to 10-membered ring) aryl;
9) a -C(O)-(5- to 12-membered ring) heteroaryl
10) an -SO₂-(6- to 10-membered ring) aryl
11) an -SO₂-(5- to 12-membered ring) heteroaryl; and,
12) a -C(O) -(C₁-C₆)-alkyl;
and preferably represents 1) hydrogen atom.

### [1-11]

In the above compound of formula (I) used in the above embodiment [1], R₅ and R₆ each independently represents an atom or group selected from hydrogen atom, a -(C₃-C₈)-cycloalkyl and a -(C₁-C₆)-alkyl, wherein the -(C₁-C₆)-alkyl independently may be substituted with 1 to 5 substituents selected from -OH, an -O-(C₁-C₆)-alkyl, a -(C₁-C₆)-haloalkyl and an -O-(C₁-C₆)-haloalkyl; and preferably R₅ and R₆ each independently represents an atom or group selected from hydrogen atom and a -(C₁-C₆)-haloalkyl.

### [1-12]

In formula (I) above, the cyclic moiety of Q represents a (6- to 10-membered ring) aryl and a (5- to 12-membered ring) heteroaryl, which may be substituted with 1 to 4 substituents optionally selected from the substituent group T, and more specifically includes Group a below, i.e., (a1) through (a16).

### Group a:

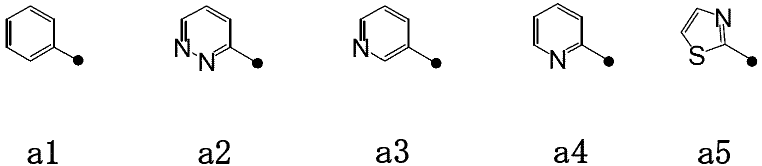

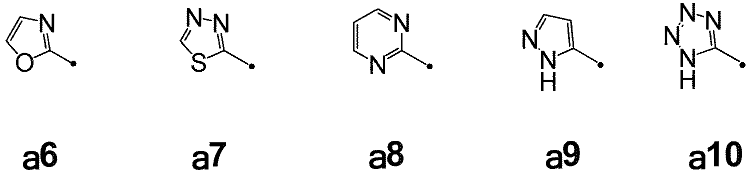

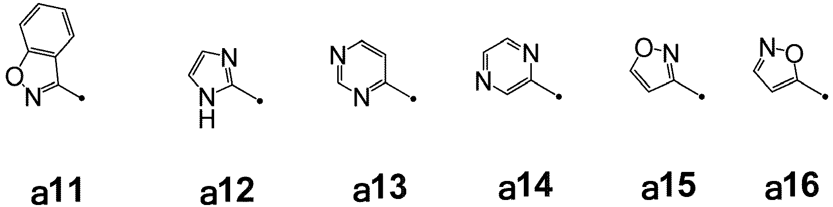

These cyclic groups shown in Group a, when substituted, may be substituted preferably with 1 to 2 substituents from the substituent group T below, i.e.,
1) a halogen;
2) a -(C₁-C₆)-alkyl, a -(C₂-C₆)-alkenyl or a -(C₂-C₆)-alkynyl, wherein the alkyl, alkenyl or alkynyl is unsubstituted or each independently may be substituted with 1 to 5 substituents optionally selected from =O (oxo), a halogen, a -(C₁-C₆)-haloalkyl, an -OR₅ or an -N(R₅)(R₆);
3) a -(C₁-C₆)-haloalkyl;
5) an -O-(C₁-C₆)-alkyl, wherein the alkyl is unsubstituted or each independently may be substituted with 1 to 3 substituents from a halogen or a -(C₁-C₆)-haloalkyl;
6) -CN;
7) a -C(O)-N(R₅)(R₆);
8) a -(C₁-C₆)-alkyl-OR₅;
10) an -N(R₅)(R₆); and,
11) an -N(R₅)-C(O)-R₆;
and more preferably, may be substituted with one substituent from:
1) a halogen;
2) a -(C₁-C₆)-alkyl;
5) an -O-(C₁-C₆)-alkyl;
10) an -N(R₅)(R₆); and,
11) an -N(R₅)-C(O)-R₆.

### [1-13]

In formula (I) above, the moiety shown by formula (III) below: 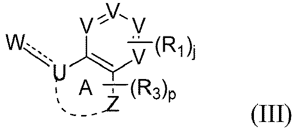 more specifically includes Group b below, i.e., formulae (b1) to (b8) or formulae (b1-1) to (b8-1).

### Group b:

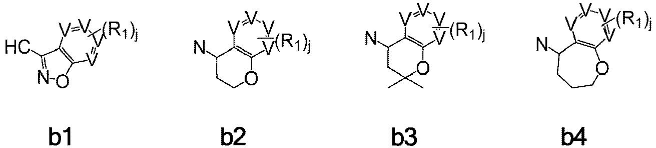

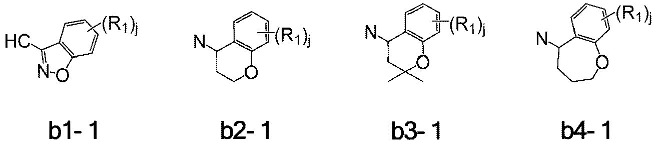

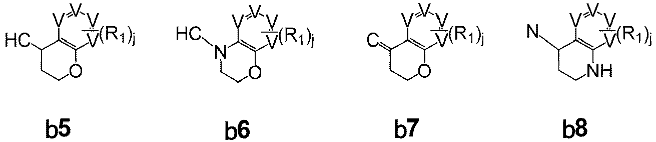

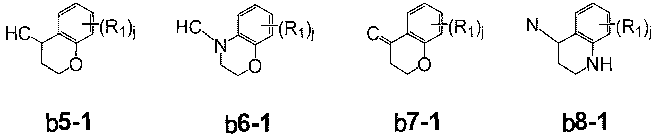

Preferably, these cyclic groups shown in Group b may be substituted with 0 to 3 substituents, and preferably 0 to 2 substituents as R₁, which are optionally selected from the groups below, i.e.;
1) a halogen;
2) a -(C₁-C₆)-alkyl, a -(C₂-C₆)-alkenyl or a -(C₂-C₆)-alkynyl, wherein the alkyl, alkenyl or alkynyl is unsubstituted or each independently may be substituted with 1 to 5 substituents optionally selected from =O (oxo), a halogen, a -(C₁-C₆)-haloalkyl, an -OR₅ or an -N(R₅)(R₆);
3) a -(C₁-C₆)-haloalkyl;
5) an -O-(C₁-C₆)-alkyl, wherein the alkyl is unsubstituted or each independently may be substituted with 1 to 3 substituents from a halogen or a -(C₁-C₆)-haloalkyl;
6) -CN;
23) an -O-(6- to 10-membered ring) aryl; and,
24) an -O-(5- to 12-membered ring) heteroaryl.

### [1-13-a]

In formula (I) above, the moiety shown by formula (III) below: 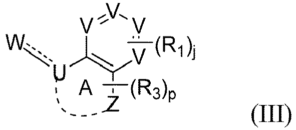 represents preferably Group b below, i.e., formulae (b1-1), (b2-1), (b6-1) and (b7-1). Group b:

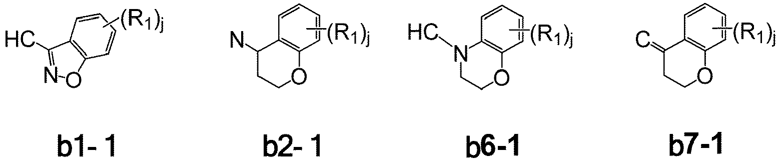

These cyclic groups shown in Group b may be substituted preferably with 0 to 2 substituents, and preferably 1 to 2 substituents as R₁, which are optionally selected from the groups below, i.e.:
1) a halogen;
2) a -(C₁-C₆)-alkyl, a -(C₂-C₆)-alkenyl or a -(C₂-C₆)-alkynyl, wherein the alkyl, alkenyl or alkynyl is unsubstituted or each independently may be substituted with 1 to 5 substituents optionally selected from =O (oxo), a halogen, a -(C₁-C₆)-haloalkyl, an -OR₅ or an -N(R₅)(R₆), and more preferably a -(C₁-C₆)-alkyl;
3) a -(C₁-C₆)-haloalkyl;
5) an -O-(C₁-C₆)-alkyl;
6) -CN;
23) an -O-(6- to 10-membered ring) aryl; and,
24) an -O-(5- to 12-membered ring) heteroaryl.

### [1-14]

In the above compounds of formula (I) used in the compounds of the above embodiment [1], the compounds obtained by appropriately combining the respective groups listed in Groups a and b described above can be produced appropriately, and are shown by formula (IV) below: 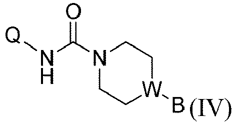 (wherein W is the same as in formula (I), Q represents a group optionally selected from formula (a1) to formula (a16) described in the above embodiment [1-12] given as a specific example, and B represents a group optionally selected from those of formula (b1) to formula (b8) or formula (b1-1) to formula (b8-1) described in the above embodiment [1-13] given as a specific example), and constitute a part of the compounds represented by formula (I) of the invention.

### [1-15]

Preferred embodiments of the compounds represented by formula (I) used in the compounds of the above embodiment [1] may optionally be formed by appropriately combining the respective embodiments [1-1] to [1-14] of the present invention and their preferred embodiments, further combining the definitions of the substituents.

### [1-16-1]

The compounds represented by formula (I) below, pharmaceutically acceptable salts thereof, or solvents thereof: 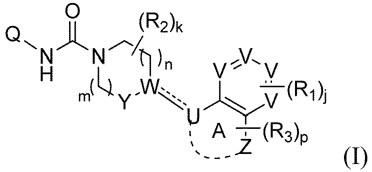 (wherein:
U represents C, CH or N, V represents CH, W represents C, CH or N, Y represents CH₂ or C=O, and Z represents O (oxygen atom) or NR₄, wherein when U is CH, the hydrogen atoms bonded to the carbon atom may be substituted with R₃, when W is CH or Y is CH₂, 1 or 2 of the hydrogen atoms bonded to the carbon atoms may be substituted with R₂; j represents an integer of 1 to 2, k represents an integer of 0 to 1, m represents 1, n represents 1, and p represents an integer of 0 to 1;
Q represents a (6- to 10-membered ring) aryl or a (5- to 12-membered ring) heteroaryl, which may be substituted with one substituent optionally selected from substituent group T;
the substituent group T consists of the groups below, i.e.:
1) a halogen;
2) a -(C₁-C₆)-alkyl;
3) a -(C₁-C₆)-haloalkyl;
5) an -O-(C₁-C₆)-alkyl, wherein the alkyl may be substituted with 1 to 3 substituents from a halogen;
6) -CN;
7) a -C(O)-N(R₅)(R₆);
10) an -N(R₅)(R₆); and,
11) an -N(R₅)-C(O)-R₆;
ring A in formula (I): 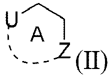 is a 6- or 7-membered ring, the dotted line in the ring A is a linkage having 2 carbon atoms, wherein the linkage is a saturated bond;
the bond between W and U:
W 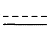 U
represents N-CH, CH-N, CH-CH or C=C;

R₁ may be the same or different and represents a group optionally selected from the groups below:
1) a halogen;
2) a -(C₁-C₆)-alkyl;
3) a -(C₁-C₆)-haloalkyl;
5) an -O-(C₁-C₆)-alkyl;
6) -CN; and
24) an -O-(5- to 12-membered ring) heteroaryl;
wherein the (5- to 12-membered ring) heteroaryl in 24) the -O-(5- to 12-membered ring) heteroaryl may be substituted with one substituent of a -(C₁-C₆)-haloalkyl;
R₂ is selected from a -(C₁-C₆)-alkyl-OH and an -O-(C₁-C₆)-alkyl;
R₃ represents a group optionally selected from the groups below, i.e.:
1) a -(C₁-C₆)-alkyl, a -(C₂-C₆)-alkenyl or a -(C₂-C₆)-alkynyl, wherein said alkyl, alkenyl or alkynyl is unsubstituted or each independently may be substituted with 1 to 5 substituents optionally selected from =O (oxo), a halogen, a -(C₁-C₆)-haloalkyl, an -OR₅ or an -N(R₅)(R₆);
2) -OH;
3) a -C(O)-N(R₅)(R₆);
4) an -N(R₅)-C(O)-R₆;
5) an -N(R₅)-SO₂-R₆;
6) an -N(R₅)-C(O)-N(R₅)(R₆);
7) an -N(R₅)-(CO)-(6- to 10-membered ring) aryl; and
8) an -N(R₅)-(CO)- (5- to 12-membered ring) heteroaryl;
wherein the (6- to 10-membered ring) aryl and (5- to 12-membered ring) heteroaryl in the 7) -NR₅-(CO)-(6- to 10-membered ring) aryl and 8) -NR₅-(CO)- (5-to 12-membered ring) heteroaryl each may be substituted with 1 to 4 substituents optionally selected from the substituent groups below, i.e.:
1) a halogen;
2) a -(C₁-C₆)-alkyl, a -(C₂-C₆)-alkenyl or a -(C₂-C₆)-alkynyl, wherein the alkyl, alkenyl or alkynyl is unsubstituted or each independently may be substituted with 1 to 5 substituents optionally selected from =O (oxo), a halogen, a -(C₁-C₆)-haloalkyl, an -OR₅ or an -N(R₅)(R₆);
3) a -(C₁-C₆)-haloalkyl;
4) -OH;
5) an -O-(C₁-C₆)-alkyl, wherein the alkyl is unsubstituted or each independently may be substituted with 1 to 3 substituents selected from a halogen or a -(C₁-C₆)-haloalkyl;
6) -CN;
7) an -N(R₅)(R₆);
8) a -C(O)-N(R₅)(R₆);
9) an -N(R₅)-C(O)-R₆;
10) an -N(R₅)-SO₂-R₆;
11) an -SO₂-(C₁-C₆)-alkyl;
12) an -SO₂-N(R₅)(R₆);
13) an -SO₂-(C₁-C₆)-haloalkyl;
14) an -S-(C₁-C₆)-alkyl;
15) an -S-(C₁-C₆)-haloalkyl;
16) a -(C₁-C₆)-alkyl-N(R₅)(R₆);
17) an -N(R₅)-C(O)-N(R₅)(R₆);
18) an -N(R₅)-C(O)-OR₅;
19) a -(C₁-C₆)-alkyl-OR₅;
20) a -(3- to 12-membered ring) heteroalicyclic; and
21) a -(6- to 10-membered ring) aryl;
R₄ represents a group optionally selected from the groups below, i.e.:
1) hydrogen atom;
4) an -SO₂-(C₁-C₆)-alkyl; and
12) a -C(O) -(C₁-C₆)-alkyl;
and,
R₅ and R₆ each independently is selected from hydrogen atom and an -O-(C₁-C₆)-alkyl).

### [1-16-2]

The compounds represented by formula (I) below, pharmaceutically acceptable salts thereof, or solvents thereof: 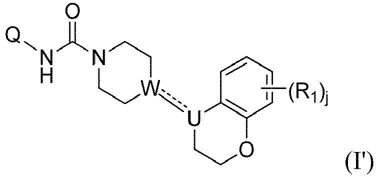 (wherein:
U represents C, CH or N; W represents C, CH or N; j represents an integer of 1 to 2;
Q represents a (5- to 12-membered ring) heteroaryl, which may be substituted with one group optionally selected from the substituent group T;
the substituent group T consists of the groups below, i.e.:
1) a halogen;
2) a -(C₁-C₆)-alkyl;
3) a -(C₁-C₆)-haloalkyl;
7) a -C(O)-N(R₅)(R₆);
10) an -N(R₅)(R₆); and
11) an -N(R₅)-C(O)-R₆;
the bond between W and U:
W 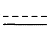 U
represents N-CH, CH-CH or C=C;

R₁, which may be the same or different, represents a group optionally selected from the groups below, i.e.:
1) a halogen;
3) a -(C₁-C₆)-haloalkyl;
5) an -O-(C₁-C₆)-alkyl;
6) -CN; and
24) an -O-(5- to 12-membered) heteroaryl;
wherein the (5- to 12-membered) heteroaryl in 24) the -O-(5- to 12-membered) heteroaryl may be substituted with one substituent from a -(C₁-C₆)-haloalkyl;
and,
each of R₅ and R₆ is independently selected from hydrogen atom and a -(C₁-C₆)-alkyl).

### [1-17]

In the above compound of formula (I) used in the compounds of the above embodiment [1] or as preferred compounds, the following compounds are given by way of example. Names of the compounds in EXAMPLES 1 to 22 and names of the compounds in EXAMPLES 23 to 69 are generated using LEXICHEM, v2.0.0 (OpenEye Scientific Software, Inc.) or ChemBioDraw Ultra (12.0, Cambridgesoft):
N-phenyl-4-(6-fluoro-1,2-benzisoxazo-3-yl)piperidine-1-carboxamide (EXAMPLE 1);
N-pyridazin-3-yl-4-(5-bromo-3,4-dihydro-2H-1-benzopyran-4-yl)piperazine-1-carbo xamide (EXAMPLE 2);
N-pyridazin-3-y1-4-(7-bromo-3,4-dihydro-2H-1-benzopyran-4-yl)piperazine-1-carbo xamide (EXAMPLE 3);
N-pyridazin-3-yl-4-[6-(trifluoromethyl)-3,4-dihydro-2H-1-benzopyran-4-yl]piperazi ne-1-carboxamide (EXAMPLE 4);
N-pyridazin-3-yl-4-[6-[[5-(trifluoromethyl)-2-pyridinyl]oxy]-3,4-dihydro-2H-1-benz opyran-4-yl]piperazine-1-carboxamide (EXAMPLE 5);
N-pyridazin-3-yl-4-[7-(2,2,2-trifluoroethoxy)-3,4-dihydro-2H-1-benzopyran-4-yl]pip erazine-1-carboxamide (EXAMPLE 6);
N-pyridazin-3-yl-4-(7-((5-(trifluoromethyl)-2-pyridinyl]oxy]-3,4-dihydro-2H-1-benz opyran-4-yl]piperazine-1-carboxamide (EXAMPLE 7);
N-pyridazin-3-yl-4-(7-(trifluoromethoxy)-3,4-dihydro-2H-1-benzopyran-4-yl]piperaz ine-1-carboxamide (EXAMPLE 8);
N-pyridazin-3-yl-4-(2,2-dimethyl-7-(trifluoromethyl)-3,4-dihydro-2H-1-benzopyran-4-yl]piperazine-1-carboxamide (EXAMPLE 9);
N-pyridazin-3-yl-4-[8-(trifluoromethyl)-2,3,4,5-tetrahydrobenz-l-oxepin-5-yl]pipera zine-1-carboxamide (EXAMPLE 10);
N-pyridazin-3-yl-4-[7-(trifluoromethyl)-3,4-dihydro-2H-l-benzopyran-4-yl]piperazi ne-1-carboxamide (EXAMPLE 11);
N-pyridazin-3-yl-4-[6-fluoro-7-(trifluoromethyl)-3,4-dihydro-2H-1-benzopyran-4-yl] piperazine-1-carboxamide (EXAMPLE 12);
N-pyridazin-3-yl-4-[8-fluoro-7-(trifluoromethyl)-3,4-dihydro-2H-1-benzopyran-4-yl] piperazine-1-carboxamide (EXAMPLE 13);
N-pyridazin-3-yl-4-(3,4-dihydro-2H-1-benzopyran-4-yl)piperazine-1-carboxamide (EXAMPLE 14);
N-phenyl-3-yl-4-[7-(trifluoromethyl)-3,4-dihydro-2H-1-benzopyran-4-yl)]-3-oxo-pip erazine-1-carboxamide (EXAMPLE 15);
N-phenyl-4-[7-(trifluoromethyl)-3,4-dihydro-2H-1-benzopyran-4-yljpiperazine-1-car boxamide (EXAMPLE 16);
N-pyridin-3-yl-4-(7-(trifluoromethyl)-3,4-dihydro-2H-1-benzopyran-4-yl]piperazine-1-carboxamide (EXAMPLE 17);
N-(6-methyl-pyridin-3-yl)-4-(7-(trifluoromethyl)-3,4-dihydro-2H-1-benzopyran-4-yl ]piperazine-1-carboxamide (EXAMPLE 18);
N-(6-chloropyridin-3-yl)-4-(7-(trifluoromethyl)-3,4-dihydro-2H-1-benzopyran-4-yl]p iperazine-1-carboxamide (EXAMPLE 19);
N-[5-(trifluoromethyl)-pyridin-2-yl]-4-[7-(trifluoromethyl)-3,4-dihydro-2H-1-benzop yran-4-yl]piperazine-1-carboxamide (EXAMPLE 20);
N-(6-methoxy-pyridazin-2-yl)-4-[7-(trifluoromethyl)-3,4-dihydro-2H-1-benzopyran-4-yl]piperazine-1-carboxamide (EXAMPLE 21);
N-(1,3-thiazol-2-yl)-4-(7-(trifluoromethyl)-3,4-dihydro-2H-1-benzopyran-4-yl]piper azine-1-carboxamide (EXAMPLE 22);
N-pyridazin-3-yl-4-[7-(trifluoromethyl)-2,3-dihydrochromen-4-ylidene]piperidine-1-carboxamide (EXAMPLE 23);
N-pyridazin-3-yl-4-[7-(trifluoromethyl)-2,3-dihydro-1,4-benzoxazin-4-yl]piperidine-1-carboxamide (EXAMPLE 24);
N-pyridazin-3-yl-4-[7-(trifluoromethyl)-3,4-dihydro-2H-chromen-4-yl]piperidine-1-c arboxamide (EXAMPLE 25);
4-hydroxy-N-pyridazin-3-yl-4-(7-(trifluoromethyl)-3,4-dihydro-2H-chromen-4-yl]pi peridine-1-carboxamide (EXAMPLE 26);
4-[1-acetyl-7-(trifluoromethyl)-3,4-dihydro-2H-quinolin-4-yl]-N-pyridazin-3-ylpiper azine-1-carboxamide (EXAMPLE 27);
N-(pyridazin-3-yl)-4-(7-(trifluoromethyl)-1,2,3,4-tetrahydroquinolin-4-yl]piperazine-1-carboxamide (EXAMPLE 28);
4-[1-methylsulfonyl-7-(trifluoromethyl)-3,4-dihydro-2H-quinolin-4-yl]-N-pyridazin-3-ylpiperazine-1-carboxamide (EXAMPLE 29);
(3S)-3-methyl-N-pyridazin-3-yl-4-[7-(triftuoromethyl)-3,4-dihydro-2H-chromen-4-yl ]piperazine-1-carboxamide (EXAMPLE 30);
(3R)-3-methyl-N-pyridazin-3-yl-4-(7-(trifluoromethyl)-3,4-dihydro-2H-chromen-4-y l]piperazine-1-carboxamide (EXAMPLE 31);
4-(7-cyano-3,4-dihydro-2H-chromen-4-yl)-N-pyridazin-3-ylpiperazine-1-carboxamid e (EXAMPLE 32);
4-(7,8-difluoro-3,4-dihydro-2H-chromen-4-yl)-N-pyridazin-3-ylpiperazine-1-carbox amide (EXAMPLE 33);
N-(pyridazin-3-yl)-4-[8-(trifluoromethyl)-3,4-dihydro-2H-chromen-4-yl]piperazine-1 -carboxamide (EXAMPLE 34);
4-(7,8-difluoro-3,4-dihydro-2H-chromen-4-yl)-N-(pyridin-3-yl)piperazine-1-carboxa mide (EXAMPLE 35);
N-pyridazin-3-yl-4-[8-(triftuoromethyl)-3,4-dihydro-2H-chromen-4-yl]piperazine-1-carboxamide (EXAMPLE 36);
4-(8-chloro-7-(trifluoromethyl)-3,4-dihydro-2H-chromen-4-yl]-N-(pyridazin-3-yl)pip erazine-1-carboxamide (EXAMPLE 37);
N-(pyridazin-3-yl)-4-[8-[5-(trifluoromethyl)pyridin-2-yl]oxy-3,4-dihydro-2H-chrom en-4-yl]piperazine-1-carboxamide (EXAMPLE 38);
4-(7,8-dichloro-3,4-dihydro-2H-chromen-4-yl)-N-(pyridazin-3-yl)piperazine-1-carbo xamide (EXAMPLE 39);
N-(5-chloropyridin-2-yl)-4-[7-(trifluoromethyl)-3,4-dihydro-2H-chromen-4-yl]pipera zine-1-carboxamide (EXAMPLE 40);
N-(pyridin-2-yl)-4[7-(trifluoromethyl)-3,4-dihydro-2H-chromen-4-yl]piperazine-1-ca rboxamide (EXAMPLE 41);
N-(5-bromopyridin-2-yl)-4-(7-(trifluoromethyl)-3,4-dihydro-2H-chromen-4-yl]piper azine-1-carboxamide (EXAMPLE 42);
N-(6-methoxypyridin-3-yl)-4-[7-(trifluoromethyl)-3,4-dihydro-2H-chromen-4-yl]pip erazine-1-carboxamide (EXAMPLE 43);
N-[6-(dimethylamino)pyridin-3-yl]-4-[7-(trifluoromethyl)-3,4-dihydro-2H-chromen-4-yl]piperazine-1-carboxamide (EXAMPLE 44);
N-(1H-tetrazol-5-yl)-4-[7-(trifluoromethyl)-3,4-dihydro-2H-chromen-4-yl]piperazine -1-carboxamide (EXAMPLE 45);
N-(pyrimidin-2-yl)-4-(7-(trifluoromethyl)-3,4-dihydro-2H-chromen-4-yl]piperazine-1-carboxamide (EXAMPLE 46);
N-(6-chloropyridazin-3-yl)-4-(7-(trifluoromethyl)-3,4-dihydro-2H-chromen-4-yl]pip erazine-1-carboxamide (EXAMPLE 47);
N-(5-methylpyridin-3-yl)-4-[7-(trifluoromethyl)-3,4-dihydro-2H-chromen-4-yl]piper azine-1-carboxamide (EXAMPLE 48);
N-(4-methylpyridin-3-yl)-4-(7-(trifluoromethyl)-3,4-dihydro-2H-chromen-4-yl]piper azine-1-carboxamide (EXAMPLE 49);
N-(6-methoxypyrimidin-4-yl)-4-(7-(trifluoromethyl)-3,4-dihydro-2H-chromen-4-yl]p iperazine-1-carboxamide (EXAMPLE 50);
N-(1,2-benzoxazol-3-yl)-4-(7-(trifluoromethyl)-3,4-dihydro-2H-chromen-4-yl]pipera zine-1-carboxamide (EXAMPLE 51);
N-pyrazin-2-yl-4-[7-(trifluoromethyl)-3,4-dihydro-2H-chromen-4-yl]piperazine-1-ca rboxamide (EXAMPLE 52);
N-(5-methylpyridin-2-yl)-4-[7-(trifluoromethyl)-3,4-dihydro-2H-chromen-4-yl]piper azine-1-carboxamide (EXAMPLE 53);
N-(5-bromopyrimidin-2-yl)-4-(7-(trifluoromethyl)-3,4-dihydro-2H-chromen-4-yl]pip erazine-1-carboxamide (EXAMPLE 54);
N-(1,2-oxazol-3-yl)-4-(7-(trifluoromethyl)-3,4-dihydro-2H-chromen-4-yl]piperazine-1-carboxamide (EXAMPLE 55);
N-(5-methyl-1,2-oxazol-3-yl)-4-(7-(trifluoromethyl)-3,4-dihydro-2H-chromen-4-yl]p iperazine-1-carboxamide (EXAMPLE 56);
N-(3,4-dimethyl-1,2-oxazol-5-yl)-4-[7-(trifluoromethyl)-3,4-dihydro-2H-chromen-4-y1]piperazine-1-carboxamide (EXAMPLE 57);
N-(1,3,4-thiadiazol-2-yl)-4-(7-(trifluoromethyl)-3,4-dihydro-2H-chromen-4-yl]pipera zine-1-carboxamide (EXAMPLE 58);
N-(pyrimidin-4-yl)-4-(7-(trifluoromethyl)-3,4-dihydro-2H-chromen-4-yl]piperazine-1-carboxamide (EXAMPLE 59);
N-(1H-pyrazol-5-yl)-4-(7-(trifluoromethyl)-3,4-dihydro-2H-chromen-4-yl]piperazine -1-carboxamide (EXAMPLE 60);
N-(5-methyl-1H-pyrazol-3-yl)-4-(7-(trifluoromethyl)-3,4-dihydro-2H-chromen-4-yl] piperazine-1-carboxamide (EXAMPLE 61);
N-[6-(methylamino)pyridin-3-yl]-4-[7-(trifluoromethyl)-3,4-dihydro-2H-chromen-4-yl]piperazine-1-carboxamide (EXAMPLE 62);
N-(2-ethylpyrazol-3-yl)-4-(7-(trifluoromethyl)-3,4-dihydro-2H-chromen-4-yl]piperaz ine-1-carboxamide (EXAMPLE 63);
N-(2-methylpyrazol-3-yl)-4-(7-(trifluoromethyl)-3,4-dihydro-2H-chromen-4-yl]piper azine-1-carboxamide (EXAMPLE 64);
N-(6-aminopyridin-3-yl)-4-(7-(trifluoromethyl)-3,4-dihydro-2H-chromen-4-yl]pipera zine-1-carboxamide (EXAMPLE 65);
N-(6-acetamidopyridin-3-yl)-4-(7-(trifluoromethyl)-3,4-dihydro-2H-chromen-4-yl]pi perazine-1-carboxamide (EXAMPLE 66);
N-(6-carbamoylpyridin-3-yl)-4-(7-(trifluoromethyl)-3,4-dihydro-2H-chromen-4-yl]pi perazine-1-carboxamide (EXAMPLE 67);
N-(3-chloropyrazin-2-yl)-4-(7-(trifluoromethyl)-3,4-dihydro-2H-chromen-4-yl]piper azine-1-carboxamide (EXAMPLE 68); and,
N-(6-cyanopyridin-3-yl)-4-[7-(trifluoromethyl)-3,4-dihydro-2H-chromen-4-yl]pipera zine-1-carboxamide (EXAMPLE 69); or optical isomers thereof, or pharmaceutically acceptable salts thereof, or solvates thereof.

More specifically, the definitions of j, k, m, n, p, U, V, W, Y, Z, Q, R₁, R₂, R₃, R₄, R₅, R₆, ring A, and the bond between W and U and preferred embodiments thereof are the same as the definitions and embodiments described in the embodiment [1] and any one of the embodiments [1-1-a] to [1-17].

### [2]

The second embodiment of the present invention is the pharmaceutical composition comprising as an active ingredient at least one of the above compounds represented by formula (I) and pharmaceutically acceptable salts thereof or solvates thereof.

An embodiment of the pharmaceutical composition may be a pharmaceutical composition comprising as an active ingredient at least one of the above compounds represented by formula (I) and pharmaceutically acceptable salts thereof or solvates thereof, which is concomitantly used with at least one of other drugs or agents.

Furthermore, one of the embodiments of the pharmaceutical composition for concomitant use may be a pharmaceutical composition comprising as an active ingredient at least one of the above compounds represented by formula (I) and pharmaceutically acceptable salts thereof or solvates thereof, and at least one of other drugs in the formulation.

### [3]

The third embodiment of the present invention is the FAAH inhibitor comprising as an active ingredient at least one of the above compounds represented by formula (I) and pharmaceutically acceptable salts thereof or solvates thereof.

As used herein, especially in the third embodiment of the invention, the term "FAAH inhibitor" is intended to mean an agent (pharmaceutical composition) comprising a compound capable of binding to FAAH and inhibiting the catabolism of endocannabinoids to continuously make the endocannabinoids physiologically active.

The FAAH inhibitors of the present invention are expected to show promising effects of preventing or treating various diseases below. Specifically, the FAAH inhibitors can be used for the treatment of at least one disease selected from the group consisting of acute or chronic pains caused by inflammatory disorders or nociceptive stimulus, i.e., pains caused by edema, burn injury, sprain, bone fracture, etc.; pains caused by postoperative pain, shoulder periarthritis, arthritis deformans, arthritis, rheumatoid arthritis, migraine, headache, tooth pain, neuralgia, myalgia, gout, hyperalgesia, sensory sensitivity, angina pectoris or menstruation; acute or chronic pains caused by neurogenic disorders, i.e., inflammatory pain, neuropathic pain, fibromyalgia syndrome, postherpetic neuralgia, trigeminal neuralgia, backache, post-spinal cord injury pain, lower limb pain, causalgic pain or diabetic neuralgia, pains caused by multiple sclerosis; dizziness, emesis or nausea induced by cancer or cancerous pain, or mainly by chemotherapy; pollakiuria, urinary incontinence, urge urinary incontinence and overactive bladder; sleeping disorders including sleep apnea, i.e., intrinsic or extrinsic sleeping disorders, circadian rhythm disorders and dyssomnia; acute or chronic neurodegenerative diseases, i.e., Alzheimer's disease, Parkinson's disease, senile dementia, cerebrovascular dementia, schizophrenia, Huntington's chorea and multiple sclerosis; neuropsychiatric disorders, i.e., anxiety, depression and epilepsy; posttraumatic stress disorders; chronic obstructive pulmonary diseases (COPD); asthma, bronchial hyperreactivity, wheeze or stridor, cough, rhinitis, mucosal inflammations of such as eyes; psychic skin diseases, i.e., inflammatory skin diseases such as psoriasis and eczema, and edema; allergic diseases; gastroduodenal ulcer; ulcerative colitis, irritable bowel and Crohn's disease; cystitis, nephritis, pancreatitis, uveitis and colitis; internal disorders; ischemia; apoplexy; hypertension; obesity; parasitic, viral or bacterial infections and sepsis; pruritus; eating disorders; hypoglycemia; diabetes mellitus; syndromes induced by drug intoxication, drug withdrawal symptoms and gas poisoning; osteoporosis; sexual dysfunction; abortion; obesity; rectal cancer, colorectal cancer; and hypertension. The compounds in some embodiments of the invention are expected to exhibit promising effects of treating at least one disease selected from the group consisting of neuropathic pains, fibromyalgia syndrome, inflammatory pains, cancerous pain, diabetic neuralgia and urinary incontinence.

### [4]

The fourth embodiment of the invention is an agent for the prevention and/or treatment of pains, comprising as an active ingredient at least one of the compounds represented by formula (I) described above, or pharmaceutically acceptable salts thereof or solvates thereof.

### [5]

The fifth embodiment of the invention is an agent for the prevention and/or treatment of neuropathic pains, comprising as an active ingredient at least one of the compounds represented by formula (I) described above, or pharmaceutically acceptable salts thereof or solvates thereof.

### [6]

The sixth embodiment of the invention is an agent for the prevention and/or treatment of inflammatory pains, comprising as an active ingredient at least one of the compounds represented by formula (I) described above, or pharmaceutically acceptable salts thereof or solvates thereof.

### [7]

The seventh embodiment of the invention is an agent for the prevention and/or treatment of cancerous pains, comprising as an active ingredient at least one of the compounds represented by formula (I) described above, or pharmaceutically acceptable salts thereof or solvates thereof.

### [8]

The eighth embodiment of the invention is an agent for the prevention and/or treatment of multiple sclerosis, comprising as an active ingredient at least one of the compounds represented by formula (I) described above, or pharmaceutically acceptable salts thereof, or solvates thereof.

In the second to eighth embodiments and their preferred embodiments, more preferred substituents and combinations thereof are described in the first embodiment.

In the embodiments of the invention described above, the term "agent for the treatment" is intended to mean not only the treatment of diseases or symptoms but also the improvement of diseases or symptoms.

In all of the embodiments described above, when the term "compound" is used, the term also embraces pharmaceutically acceptable salts thereof. The compounds of the present invention may sometimes contain an asymmetric carbon atom. In such a case, the compounds of the present invention include mixtures of various stereoisomers, such as geometrical isomers, tautomers and optical isomers, as well as isolated isomers. The isolation and purification of such stereoisomers can be performed by those skilled in the art in a conventional manner, including optical resolution using preferential crystallization or column chromatography, or asymmetric synthesis.

Where the compounds of the present invention contain an asymmetric carbon, either one of the optical isomers may have a stronger FAAH inhibitory activity than the other isomer when the optical isomers are optically resolved by, e.g., column chromatography.

The compounds represented by formula (I) of the present invention may form acid addition salts or salts with bases depending upon the type of substituents. These salts are not particularly limited as long as the salts are pharmaceutically acceptable salts. Specific examples of the salts include acid addition salts with a mineral acid such as hydrochloric acid, hydrobromic acid, hydroiodic acid, sulfuric acid, nitric acid, phosphoric acid, etc.; an aliphatic monocarboxylic acid, such as formic acid, acetic acid, propionic acid, butyric acid, valeric acid, enanthic acid, capric acid, myristic acid, palmitic acid, stearic acid, lactic acid, sorbic acid, mandelic acid, etc., an aromatic monocarboxylic acid, e.g., benzoic acid, salicylic acid, etc., an aliphatic dicarboxylic acid, e.g., oxalic acid, malonic acid, succinic acid, fumaric acid, maleic acid, malic acid, tartaric acid, etc. and an aliphatic tricarboxylic acid, e.g., citric acid, etc.; organic carboxylic acids such as cinnamic acid, glycolic acid, pyruvic acid, oxylic acid, salicylic acid and N- acetylcysteic acidganic carboxylic acid; an organic sulfonic acid such as an aliphatic sulfonic acid, e.g., methanesulfonic acid, ethanesulfonic acid, 2-hydroxyethanesulfonic acid, etc., or an aromatic sulfonic acid, e.g., benzenesulfonic acid, p-toluenesulfonic acid, etc.; or an acidic amino acid, e.g., aspartic acid, glutamic acid, etc.; salts with a metal such as an alkali metal, e.g., lithium, sodium, potassium, cesium, etc., or an alkaline earth metal, e.g., magnesium, calcium, barium, etc. (including, e.g., disodium salts and dipotassium salts in addition to mono salts); salts with a metal such as aluminum, iron, copper, nickel, cobalt, zinc, etc., salts with an organic base such as methylamine, ethylamine, t-butylamine, t-octylamine, diethylamine, triethylamine, cyclohexylamine, dibenzylamine, ethanolamine, diethanolamine, triethanolamine, piperidine, morpholine, pyridine, lysine, arginine, ornithine, ethylenediamine, N-methylglucamine, glucosamine, a phenylglycine alkyl ester, guanidine, etc; a glycine salt, a histidine salt, a choline salt, an ammonium salt, etc.

These salts can be obtained in a conventional manner, for example, by mixing an appropriate amount of the compound of the present invention with a solution containing a desired acid or base, etc. and then collecting the desired salt through filtration or removal of the solvent by distillation. The compound of the present invention or salts thereof can form solvates with a solvent such as water, ethanol, glycerol, etc.

The salts of the compound of the present invention include mono-salts, di-salts and tri-salts. Alternatively, the compound of the present invention can form acid addition salts and salts with bases at the same time depending on the type of substituents on the side chain. The present invention further includes the hydrates, various pharmaceutically acceptable solvates, crystal polymorphs, etc. of the compound of the present invention represented by formula (I). As a matter of course, the present invention is not limited to the compounds described in examples below and includes all of the compounds represented by formula (I) of the present invention, or pharmaceutically acceptable salts thereof.

The compound of the present invention may be labeled with an isotope (e.g., ³H, ¹⁴C, ³⁵S etc.).

The processes for producing the compounds of the present invention represented by formula (I) are shown below.

### [Processes for Producing the Compounds of the Invention]

The compounds of the present invention represented by formula (I) and related compounds can be obtained according to the processes shown below. Each of the processes is described below.

The reaction conditions used in the production processes are as described below, unless otherwise indicated. The reaction temperature is in the range of -78°C to the solvent-reflux temperature, and the reaction time is the time sufficient to proceed the reaction. Examples of solvents which are inert to the reaction include an aromatic hydrocarbon solvent such as toluene, xylene, benzene, etc.; a polar solvent such as alcohols, e.g., methanol, ethanol, etc., N,N-dimethylformamide, dimethyl sulfoxide, acetonitrile, water, etc.; a basic solvent such as triethylamine, pyridine, etc.; organic acid solvent such as acetic acid, etc.; a halogenated solvent such as chloroform, dichloromethane, 1,2-dichloroethane, etc.; an ether solvent such as diethyl ether, tetrahydrofuran, dioxane, dimethoxyethane, etc.; and a mixed solvent thereof, and the solvent to be used may be suitably chosen depending upon the reaction conditions. Examples of bases include inorganic bases such as potassium carbonate, sodium carbonate, cesium carbonate, sodium hydroxide, potassium hydroxide, sodium hydride, sodium hydrogencarbonate, etc.; and organic bases such as triethylamine, diethylamine, pyridine, an N,N-dialkylanilines, lithium diisopropylamide, lithium bis(trimethylsilyl)amide, etc. Examples of acids include inorganic acids such as hydrochloric acid, sulfuric acid, etc.; and organic acids such as acetic acid, trifluoroacetic acid, methanesulfonic acid, p-toluenesulfonic acid, etc. However, the solvent, base and acid are not necessarily limited to those described above.

The compounds represented by formula (I) of the present invention and salts thereof can be readily produced from commercially available compounds by known processes described in published documents, and can also be produced by production processes described below.

The present invention is not limited to the production processes described below.

The production processes are described in detail below. In the compounds represented by formulae (I), (I-a), (I-b), (I-c), (V), (V-a), (V-b), (V-c), (V-d), (V-e), (V-f), (V- g), (VI), (VII), (VIII), (IX), (X), (XI), (XII), (XIII), (XIV), (XV), (XVI), (XVII), (XVIII), (XVIII-a), (XVIII-b), (XIX), (XX), (XXI), (XXI-a), (XXII), (XXIII), (XXIV), (XXV), (XXVI), (XXVII), (XXVIII), (XXIX), (XXX), (XXXI), (XXXII), (XXXIII), (XXXIV), (XXXV), (XXXVI), (XXXVII), (XXXVIII) and (XXXIX) described below, R₁, R₂, R₃, U, Q, V, Y, W,Z, j, k, m, n, p, ring A and the bond between U and W are the same as already defined in formula (I), unless otherwise indicated. L and L' represent a leaving substituent such as a halogen (F, Cl, Br, I), a sulfonate ester, phenoxy, 2,2,2-trichloroethoxy, an alkoxy, etc.; P and P' represent a protecting group such as t-butoxycarbonyl, benzyloxycarbonyl, benzyl, phthalimide, etc.; M represents lithium (Li), a magnesium halide (MgX wherein X is a halogen atom), a borate ester, etc. R_{A} represents hydrogen atom or a (C₁-C₆)-alkyl.

### (Process 1)

The compound represented by formula (I) can be produced by the following process.

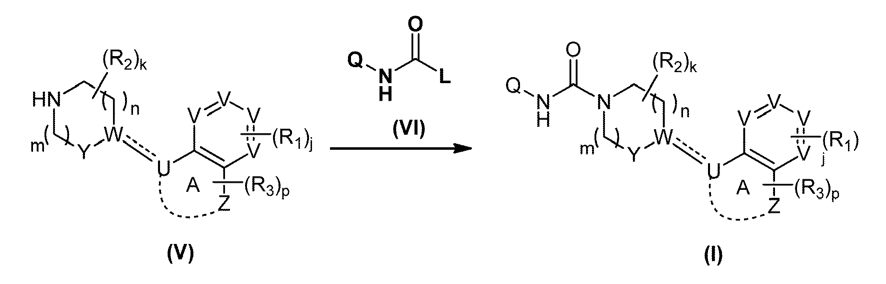

The compound represented by formula (I) can be produced according to known processes described in literatures, e.g., Organic Process Research & Development, 10(4), 822-828, 2006, using the compound represented by formula (V) and the compound represented by formula (VI), where the reaction is carried out, in the presence or absence of a base such as triehylamine, pyridine, etc., in a solvent inert to the reaction, e.g., a halogenated solvent such as dichloromethane, chloroform, etc., an ether solvent such as diethyl ether, tetrahydrofuran, etc., an aromatic hydrocarbon solvent such as toluene, benzene, etc., a polar solvent such as N,N-dimethylformamide, acetonitrile, dimethylsulfoxide, etc. or an alcohol solvent such as methanol, ethanol, 2-propanol, etc., at temperatures between 0°C and the solvent-reflux temperature.

### (Process 1-2)

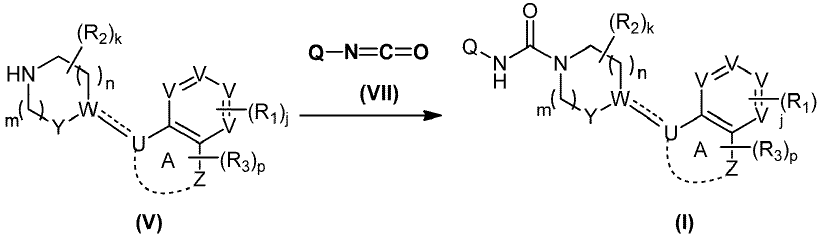

The compound represented by formula (I) can be produced according to known processes described in literatures, e.g., Journal of Medicinal Chemistry, 48(6), 1857-1872, 2005, using the compound represented by formula (V) and an isocyante represented by formula (VII), where the reaction is carried out, in the presence or absence of a base such as triehylamine, pyridine, etc., in a solvent inert to the reaction, e.g., a halogenated solvent such as dichloromethane, chloroform, etc., an ether solvent such as diethyl ether, tetrahydrofuran, etc., an aromatic hydrocarbon solvent such as toluene, benzene, etc., or a polar solvent such as N,N-dimethylformamide, acetonitrile, dimethylsulfoxide, etc., at temperatures between -78°C and the solvent-reflux temperature.

Where the compounds of formula (I) described above are racemic, isolation and purification of such stereoisomers can be performed by those skilled in the art in a conventional manner, including optical resolution using preferential crystallization or column chromatography, or asymmetric synthesis.

### (Process 1-3)

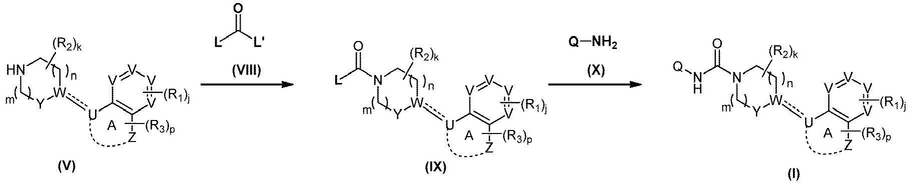

The compound represented by formula (IX) is first produced according to known processes described in literatures, e.g., Journal of Medicinal Chemistry, 45(2), 4513-4523, 2002, using the compound represented by formula (V) and a carbonylation reagent represented by formula (VIII) such as triphosgen, 1,1'-carbonyldiimidazole (CDI), etc., where the reaction is carried out, in the presence or absence of a base such as triehylamine, pyridine, etc., in a solvent inert to the reaction, e.g., a halogenated solvent such as dichloromethane, chloroform, etc., an ether solvent such as diethyl ether, tetrahydrofuran, etc., an aromatic hydrocarbon solvent such as toluene, benzene, etc., or a polar solvent such as N,N-dimethylformamide, acetonitrile, dimethylsulfoxide, etc., at temperatures between -78°C and the solvent-reflux temperature. Then, the compound represented by formula (I) can be produced according to known processes described in literatures, e.g., Journal of Medicinal Chemistry, 45(2), 4513-4523, 2002, using the compound represented by formula (X), where the reaction is carried out, in the presence or absence of a base such as triehylamine, pyridine, etc., in a solvent inert to the reaction, e.g., a halogenated solvent such as dichloromethane, chloroform, etc., an ether solvent such as diethyl ether, tetrahydrofuran, etc., an aromatic hydrocarbon solvent such as toluene, benzene, etc., a polar solvent such as N,N-dimethylformamide, acetonitrile, dimethylsulfoxide, etc., or an alcohol solvent such as methanol, ethanol, 2-propanol, etc., at temperatures between 0°C and the solvent-reflux temperature.

Where the compounds of formula (I) described above are racemic, isolation and purification of such stereoisomers can be performed by those skilled in the art in a conventional manner, including optical resolution using preferential crystallization or column chromatography, or asymmetric synthesis.

### (Process 1-4)

The compound represented by formula (I-a) wherein the bond between W and U is a single bond and W is N in formula (I) above can be produced by the following process.

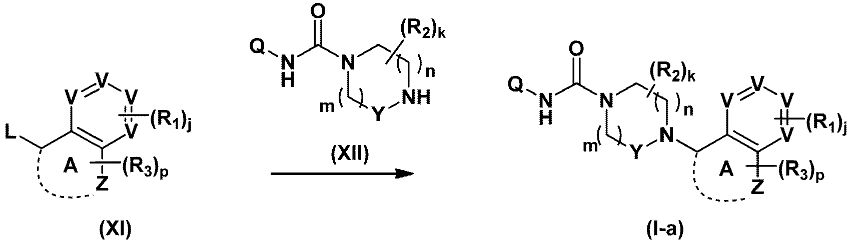

The compound represented by formula (I-a) can be produced according to known processes described in literatures, e.g., JIKKEN KAGAKU KOZA (Book Series of Experiments in Chemistry) (4th edition, 20 Synthesis of Organic Compound II, Alcohol/Amine, 284-282, 1992, Maruzen Publishing Co.), using the compound represented by formula (XI) and an amine represented by formula (XII), where the reaction is carried out, in the presence or absence of a base such as triehylamine, pyridine, potassium carbonate, etc. and in the presence or absence of an iodation reagent such as sodium iodide, potassium iodide, tetrabutylammonium iodide, etc., in a solvent inert to the reaction, e.g., a halogenated solvent such as dichloromethane, chloroform, etc., an ether solvent such as diethyl ether, tetrahydrofuran, etc., an aromatic hydrocarbon solvent such as toluene, benzene, etc., a polar solvent such as N,N-dimethylformamide, acetonitrile, dimethylsulfoxide, etc., or an alcohol solvent such as methanol, ethanol, 2-propanol, etc., at temperatures between 0°C and the solvent-reflux temperature.

Where the compounds of formula (I) described above are racemic, isolation and purification of such stereoisomers can be performed by those skilled in the art in a conventional manner, including optical resolution using preferential crystallization or column chromatography, or asymmetric synthesis.

### (Process 1-5)

The compound specifically represented by formula (I-a) wherein the bond between W and U is a single bond and W is N in formula (I) above can be produced by the following process.

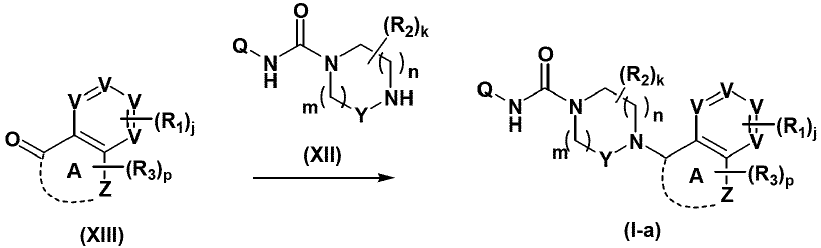

The compound represented by formula (I-a) can be produced according to known processes described in literatures, e.g., JIKKEN KAGAKU KOZA (Book Series of Experiments in Chemistry) (4th edition, 20 Synthesis of Organic Compound II, Alcohol/Amine, 300-302, 1992, Maruzen Publishing Co.), using the compound represented by formula (XIII) and an amine represented by formula (XII), where the reaction is carried out, in the presence or absence of an acid such as acetic acid, etc. and in the presence of a reducing agent such as sodium cyanoborohydride, sodium triacetoxyborohydride, etc., in a solvent inert to the reaction, e.g., a halogenated solvent such as dichloromethane, chloroform, etc., an ether solvent such as diethyl ether, tetrahydrofuran, etc., an aromatic hydrocarbon solvent such as toluene, benzene, etc., a polar solvent such as N,N-dimethylformamide, acetonitrile, dimethylsulfoxide, etc. or an alcohol solvent such as methanol, ethanol, 2-propanol, etc., at temperatures between 0°C and the solvent-reflux temperature.

Where the compounds of formula (I) described above are racemic, isolation and purification of such stereoisomers can be performed by those skilled in the art in a conventional manner, including optical resolution using preferential crystallization or column chromatography, or asymmetric synthesis.

### (Process 1-6)

The compound represented by formula (I) above can be produced by the following process.

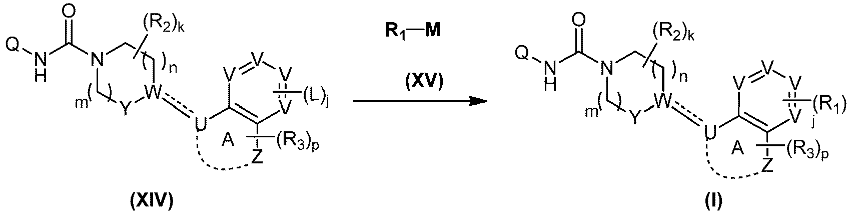

The compound represented by formula (I) can be produced according to known processes described in literatures, e.g., JIKKEN KAGAKU KOZA (Book Series of Experiments in Chemistry) (5th edition, 18 Synthesis of Organic Compound VI, Organic Synthesis with Metal, 327-352, 2004, Maruzen Publishing Co.), using the compound represented by formula (XIV) and the compound represented by formula (XV), where the reaction is carried out, in the presence or absence of a base such as triehylamine, pyridine, lithium hydroxyde, sodium hydroxyde, potassium carbonate, etc. and in the presence of a catalyst such as copper iodide, tetrakistriphenylphosphine palladium, etc., in a solvent inert to the reaction, e.g., a halogenated solvent such as dichloromethane, chloroform, etc., an ether solvent such as diethyl ether, tetrahydrofuran, etc., an aromatic hydrocarbon solvent such as toluene, benzene, etc., a polar solvent such as N,N-dimethylformamide, acetonitrile, dimethylsulfoxide, etc., or an alcohol solvent such as methanol, ethanol, 2-propanol, etc., at temperatures between 0°C and the solvent-reflux temperature.

Where the compounds of formula (I) described above are racemic, isolation and purification of such stereoisomers can be performed by those skilled in the art in a conventional manner, including optical resolution using preferential crystallization or column chromatography, or asymmetric synthesis.

### (Process A)

The compound specifically represented by formula (V-a) wherein the bond between W and U is a single bond and W is N in formula (V) above can be produced by the following process.

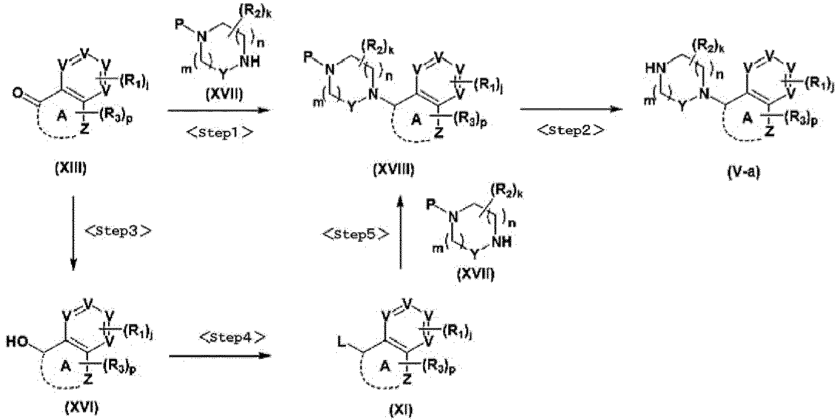

### <Step 1>

The compound represented by formula (XVIII) can be produced by the same process as used in the process (1-5), using the compound represented by formula (XIII) and the compound represented by formula (XVII).

### <Step 2>

The compound represented by formula (V-a) can be produced by deprotection of the compound represented by formula (XVIII) according to modifications of known processes described in literatures, e.g., Protective Groups In Organic Synthesis, (USA), 4th edition, 2006, etc.

### <Step 3>

The compound represented by formula (XVI) can be produced according to known processes described in literatures, e.g., JIKKEN KAGAKU KOZA (Book Series of Experiments in Chemistry) (4th edition, 20 Synthesis of Organic Compound II, Alcohol/Amine, 1-30, 2004, Maruzen Publishing Co.), using the compound represented by formula (XIII), where the reaction is carried out, in the presence of a reducing agent such as sodium borohydride, sodium diisobutylaluminum hydride, etc., in a solvent inert to the reaction, e.g., a halogenated solvent such as dichloromethane, chloroform, etc., an ether solvent such as diethyl ether, tetrahydrofuran, etc., an aromatic hydrocarbon solvent such as toluene, benzene, etc., a polar solvent such as N,N-dimethylformamide, acetonitrile, dimethylsulfoxide, etc., or an alcohol solvent such as methanol, ethanol, 2-propanol, etc., at temperatures between 0°C and the solvent-reflux temperature.

### <Step 4>

The compound represented by formula (XI) can be produced according to known processes described in literatures, e.g., JIKKEN KAGAKU KOZA (Book Series of Experiments in Chemistry) (4th edition, 20 Synthesis of Organic Compound I, Hydrocarbon/Halide, 438-472, 2004, Maruzen Publishing Co.), using the compound represented by formula (XVI), where the reaction is carried out, in the presence of a reagent such as methanesulfonyl chloride, triphenylphosphine/carbon tetrabromide, thionyl chloride, etc., in the presence or absence of a base such as triehylamine, pyridine, etc., in a solvent inert to the reaction, e.g., a halogenated solvent such as dichloromethane, chloroform, etc., an ether solvent such as diethyl ether, tetrahydrofuran, etc., an aromatic hydrocarbon solvent such as toluene, benzene, etc., or a polar solvent such as N,N-dimethylformamide, acetonitrile, dimethylsulfoxide, etc., at temperatures between -78°C and the solvent-reflux temperature.

### <Step 5>

The compound of formula (XVIII) can be produced by the same process as used in (Process 1-4), using the compound represented by formula (XI) and the compound represented by formula (XVII).

### (Process B)

The compound specifically represented by formula (XVIII) can be produced by the following process.

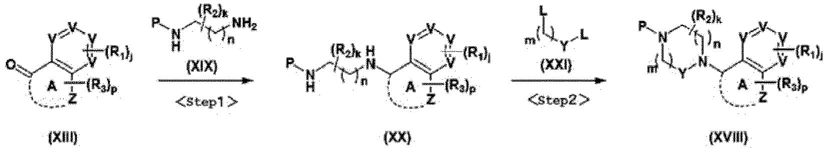

### <Step 1>

The compound of formula (XX) can be produced by the same process as used in the process (1-5), using the compound represented by formula (XIII) and the compound represented by formula (XIX).

### <Step 2>

The compound of formula (XVIII) can be produced by the same process as used in (Process 1-4), using the compound represented by formula (XX) and the compound represented by formula (XXI).

### (Process C)

The compound specifically represented by formula (V-b) wherein the bond between W and U is a single bond, W is N and Y is CO in formula (V) above as well as the compound specifically represented by formula (V-c) wherein the bond between W and U is a single bond, W is N and Y is CH₂ in formula (V) above can be produced by the following process.

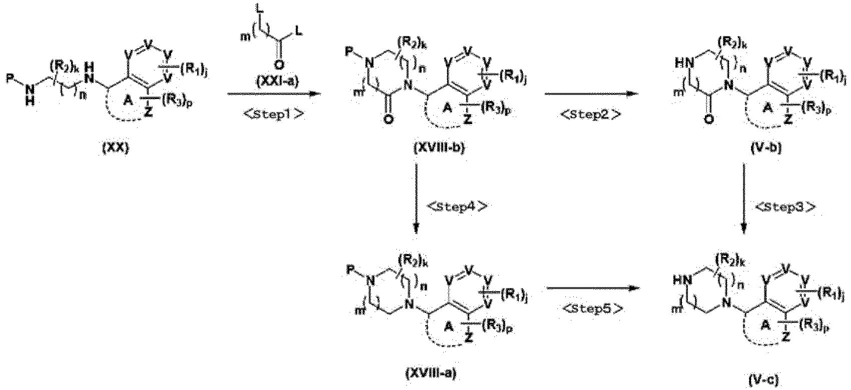

### <Step 1>

The compound represented by formula (XVIII-b) wherein Y is CO in formula (XVIII) described above can be produced according to known processes described in literatures, e.g., Journal of Medicinal Chemistry, 46(14), 2877-2894, 2003, using the compound represented by formula (XX) and the compound represented by formula (XXI-a) in which Y is CO in formula (XXI) described above, where the reaction is carried out, in the presence or absence of a base such as triehylamine, pyridine, sodium hydride, potassium carbonate, etc., in a solvent inert to the reaction, e.g., a halogenated solvent such as dichloromethane, chloroform, etc., an ether solvent such as diethyl ether, tetrahydrofuran, etc., an aromatic hydrocarbon solvent such as toluene, benzene, etc., a polar solvent such as N,N-dimethylformamide, acetonitrile, dimethylsulfoxide, etc., or an alcohol solvent such as methanol, ethanol, 2-propanol, etc., at temperatures between 0°C and the solvent-reflux temperature.

### <Step 2>

The compound of formula (V-b) can be produced by the same process as used in (Process A) <Step 2>, using the compound represented by formula (XVIII-b).

### <Step 3>

The compound represented by formula (V-c) can be produced according to known processes described in literatures, e.g., Journal of Medicinal Chemistry), 46(14), 2877-2894, 2003, using the compound represented by formula (V-b), where the reaction is carried out, in the presence of a reducing agent such as boran, boran-THF complex, lithium aluminum hydride, etc., in a solvent inert to the reaction, e.g., a halogenated solvent such as dichloromethane, chloroform, etc., an ether solvent such as diethyl ether, tetrahydrofuran, etc., an aromatic hydrocarbon solvent such as toluene, benzene, etc., or a polar solvent such as acetonitrile, dimethylsulfoxide, etc., at temperatures between 0°C and the solvent-reflux temperature.

### <Step 4>

The compound of formula (XVIII-a) can be produced by the same process as used in (Process C) <Step 3>, using the compound represented by formula (XVIII-b).

### <Step 5>

The compound of formula (V-c) can be produced by the same process as used in (Process A) <Step 2>, using the compound represented by formula (XVIII-a).

### (Process D)

The compound specifically represented by formula (V-a) described above can be produced by the following process.

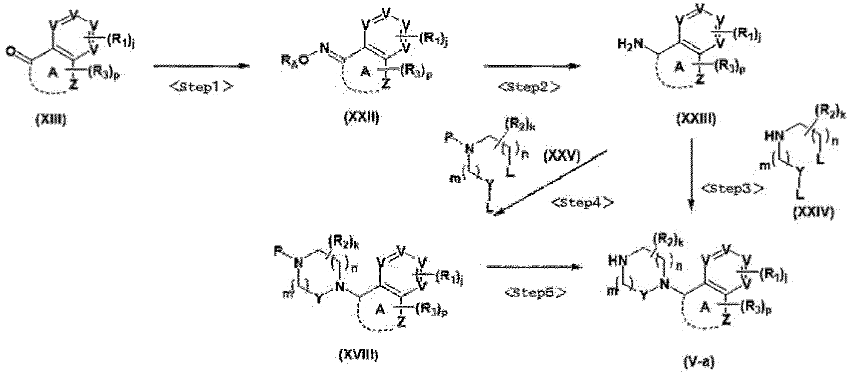

### <Step 1>

The compound represented by formula (XXII) can be produced according to known processes described in literatures, e.g., Bioorganic & Medicinal Chemistry, 16(11), 6124-6130, 2008, using the compound represented by formula (XIII), where the reaction is carried out, in the presence or absence of a base such as triehylamine, pyridine, sodium hydride, potassium carbonate, etc. and in the presence of a reagent such as hydroxylamine, etc., in a solvent inert to the reaction, e.g., a halogenated solvent such as dichloromethane, chloroform, etc., an ether solvent such as diethyl ether, tetrahydrofuran, etc., an aromatic hydrocarbon solvent such as toluene, benzene, etc., a polar solvent such as N,N-dimethylformamide, acetonitrile, dimethylsulfoxide, water, etc., or an alcohol solvent such as methanol, ethanol, 2-propanol, etc., at temperatures between 0°C and the solvent-reflux temperature.

### <Step 2>

The compound represented by formula (XXIII) can be produced according to known processes described in literatures, e.g., Bioorganic & Medicinal Chemistry, 16(11), 6124-6130, 2008, using the compound represented by formula (XXII), where the reaction is carried out, in the presence of a reducing agent such as lithium aluminum hydride, hydrogen/palladium-carbon, etc., in a solvent inert to the reaction, e.g., a halogenated solvent such as dichloromethane, chloroform, etc., an ether solvent such as diethyl ether, tetrahydrofuran, etc., an aromatic hydrocarbon solvent such as toluene, benzene, etc., a polar solvent such as N,N-dimethylformamide, acetonitrile, dimethylsulfoxide, water, etc., or an alcohol solvent such as methanol, ethanol, 2-propanol, etc., at temperatures between 0°C and the solvent-reflux temperature.

### <Step 3>

The compound of formula (V-a) can be produced by the same process as used in (Process 1-4), using the compound represented by formula (XXIII) and the compound represented by formula (XXIV).

### <Step 4>

The compound of formula (XVIII) can be produced by the same process as used in (Process 1-4), using the compound represented by formula (XXIII) and the compound represented by formula (XXV).

### <Step 5>

The compound of formula (V-a) can be produced by the same process as used in (Process A) <Step 2>, using the compound represented by formula (XVIII).

### (Process E)

The compound specifically represented by formula (XXIII) can be produced by the following process.

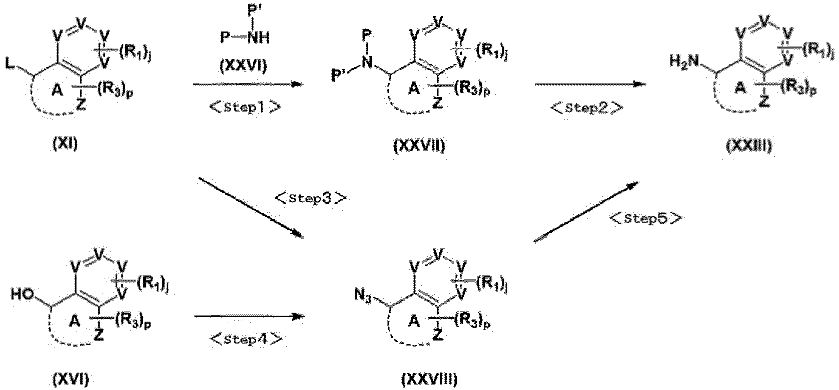

### <Step 1>

The compound of formula (XXVII) can be produced by the same process as used in (Process 1-4), using the compound represented by formula (XI) and the compound represented by formula (XXVI).

### <Step 2>

The compound of formula (XXIII) can be produced by the same process as used in (Process A) <Step 2>, using the compound represented by formula (XXVII).

### <Step 3>

The compound represented by formula (XXVIII) can be produced according to known processes described in literatures, e.g., JIKKEN KAGAKU KOZA (Book Series of Experiments in Chemistry) (4th edition, 20 Synthesis of Organic Compound II, Alcohol/Amine, 415-420, 1992, Maruzen Publishing Co.), using the compound represented by formula (XI), where the reaction is carried out, in the presence of an azidizing reagent such as sodium azide, etc., in a solvent inert to the reaction, e.g., a halogenated solvent such as dichloromethane, chloroform, etc., an ether solvent such as diethyl ether, tetrahydrofuran, etc., an aromatic hydrocarbon solvent such as toluene, benzene, etc., a polar solvent such as N,N-dimethylformamide, acetonitrile, dimethylsulfoxide, water, etc., or an alcohol solvent such as methanol, ethanol, 2-propanol, etc., at temperatures between 0°C and the solvent-reflux temperature.

### <Step 4>

The compound represented by formula (XXVIII) can be produced according to known processes described in literatures, e.g., Tetrahedron, 63(29), 6755-6763, 2007, using the compound represented by formula (XVI), where the reaction is carried out, in the presence of an azidizing reagent such as diphenylphosphoryl azide, etc., in a solvent inert to the reaction, e.g., a halogenated solvent such as dichloromethane, chloroform, etc., an ether solvent such as diethyl ether, tetrahydrofuran, etc., an aromatic hydrocarbon solvent such as toluene, benzene, etc., or a polar solvent such as N,N-dimethylformamide, acetonitrile, dimethylsulfoxide, etc., at temperatures between 0°C and the solvent-reflux temperature.

### <Step 5>

The compound represented by formula (XXIII) can be produced by modifications of known processes described in literatures, e.g., Tetrahedron, 63(29), 6755-6763, 2007, using the compound represented by formula (XXVIII), where the reaction is carried out, in the presence of a reducing agent such as triphenylphosphine/water, hydrogen/palladium-carbon, etc., in a solvent inert to the reaction, e.g., a halogenated solvent such as dichloromethane, chloroform, etc., an ether solvent such as diethyl ether, tetrahydrofuran, etc., an aromatic hydrocarbon solvent such as toluene, benzene, etc., a polar solvent such as N,N-dimethylformamide, acetonitrile, dimethylsulfoxide, etc., or an alcohol solvent such as methanol, ethanol, 2-propanol, etc., at temperatures between 0°C and the solvent-reflux temperature.

### (Process 1-7)

The compound specifically represented by formula (I-b) wherein W is C and the bond between W and U is a double bond in formula (I) above, the compound specifically represented by formula (I-c) wherein W is CH and the bond between W and U is a single bond in formula (I) above, the compound specifically represented by formula (V-d) wherein W is C and the bond between W and U is a double bond in formula (V) above, and the compound specifically represented by formula (V-e) wherein W is CH and the bond between W and U is a single bond in formula (V) above can be produced by the following process.

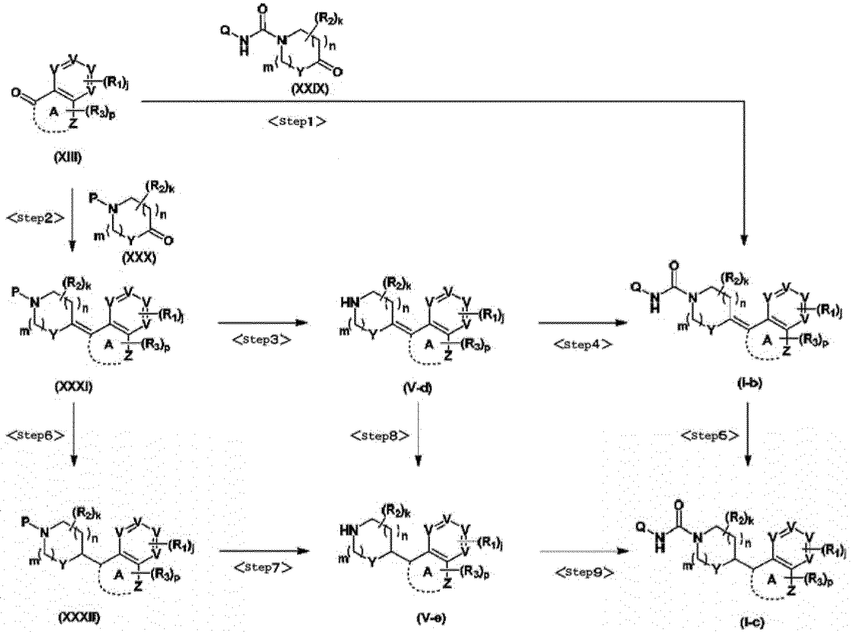

### <Step 1>

The compound represented by formula (I-b) can be produced according to known processes described in literatures, e.g., JIKKEN KAGAKU KOZA (Book Series of Experiments in Chemistry) (5th edition, 18 Synthesis of Organic Compound VI, Organic Synthesis with Metal, 426-436, 2004, Maruzen Publishing Co.), using the compound represented by formula (XIII) and the compound represented by formula (XXIX), where the reaction is carried out, in the presence of a low valency titanium species generated from titanium tetrachloride and zinc, titanium trichloride and lithium metal, etc. or samarium iodide, etc., in a solvent inert to the reaction, e.g., a halogenated solvent such as dichloromethane, chloroform, etc., an ether solvent such as dimethoxyethane, diethyl ether, tetrahydrofuran, etc., or an aromatic hydrocarbon solvent such as toluene, benzene, etc., at temperatures between -78°C and the solvent-reflux temperature.

### <Step 2>

The compound of formula ((XXXI) can be produced by the same process as used in (Process 1-7) <Step 1>, using the compound represented by formula (XIII) and the compound represented by formula (XXX).

### <Step 3>

The compound of formula (V-d) can be produced by the same process as used in (Process A) <Step 2>, using the compound represented by formula (XXXI).

### <Step 4>

The compound of formula (I-b) can be produced by the same process as used in (Process 1), using the compound represented by formula (V-d).

### <Step 5>

The compound represented by formula (I-c) can be produced according to known processes described in literatures, e.g., SHIN-JIKKEN KAGAKU KOZA (New Book Series of Experiments in Chemistry) (5th edition, 15 Oxidation and Reduction II, 333-448, 1977, Maruzen Publishing Co.), using the compound represented by formula (I-b), where the reaction is carried out, in the presence of a hydrogen source such as hydrogen, ammonium formate, etc. and in the presence and absence of an acid such as acetic acid, etc. using a catalyst such as palladium-carbon, platinum oxide, Raney nickel, etc., in a solvent inert to the reaction, e.g., an alcohol solvent such as methanol, ethanol, isopropanol, etc., an ether solvent such as dimethoxyethane, diethyl ether, tetrahydrofuran, etc., or an aromatic hydrocarbon solvent such as toluene, benzene, etc., at temperatures between 0°C and the solvent-reflux temperature.

### <Step 6>

The compound represented by formula (XXXII) can be produced by the same process as used in (Process 1-7) <Step 5>, using the compound represented by formula (XXXI).

Where the compounds of formula (XXXII) described above are racemic, isolation and purification of such stereoisomers can be performed by those skilled in the art in a conventional manner, including optical resolution using preferential crystallization or column chromatography, or asymmetric synthesis.

### <Step 7>

The compound of formula (V-e) can be produced by the same process as used in (Process A) <Step 2>, using the compound represented by formula (XXXII).

### <Step 8>

The compound of formula (V-e) can be produced by the same process as used in (Process 1-7) <Step 5>, using the compound represented by formula (V-d).

### <Step 9>

The compound of formula (I-c) can be produced by the same process as used in (Process 1), using the compound represented by formula (V-e).

### (Process F)

The compound specifically represented by formula (V-f) wherein W is COH and the bond between W and U is a single bond in formula (V) above can be produced by the following process.

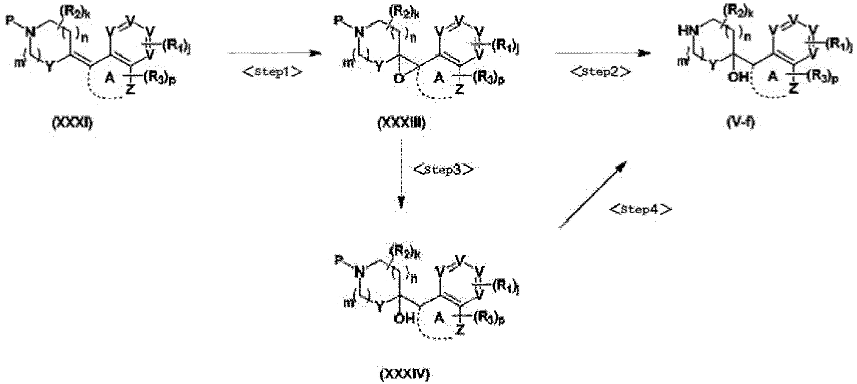

### <Step 1>

The compound represented by formula (XXXIII) can be produced according to known processes described in literatures, e.g., JIKKEN KAGAKU KOZA (Book Series of Experiments in Chemistry) (4th edition, 23 Synthesis of Organic Compound VI, Oxidation Reaction, 225-298, 1991, Maruzen Publishing Co.), using the compound represented by formula (XXXI), where the reaction is carried out using an oxidizing agent such as metachloroperbenzoic acid, trifluoroperacetic acid, dioxirane, hydrogen peroxide, etc., in the presence or absence of a base such as sodium hydrogencarbonate, potassium carbonate, etc., in a solvent inert to the reaction, e.g., a halogenated solvent such as dichloromethane, chloroform, etc., an ether solvent such as dimethoxyethane, diethyl ether, tetrahydrofuran, etc., an aromatic hydrocarbon solvent such as toluene, benzene, etc., or a hydrocarbon solvent such as hexane, heptane, cyclohexane, etc., at temperatures between 0°C and the solvent-reflux temperature.

### <Step 2>

The compound of formula (V-f) can be produced by the same process as used in (Process 1-7) <Step 5>, using the compound represented by formula (XXXIII).

### <Step 3>

The compound of formula (XXXIV) can be produced by the same process as used in (Process 1-7) <Step 5>, using the compound represented by formula (XXXIII).

### <Step 4>

The compound of formula (V-f) can be produced by the same process as used in (Process A) <Step 2>, using the compound represented by formula (XXXIV).

### (Process G)

The compound specifically represented by formula (V-g) wherein U is N, the bond between W and U is a single bond, W is CH, Y is CH₂ and P is O in formula (V) described above can be produced by the following process.

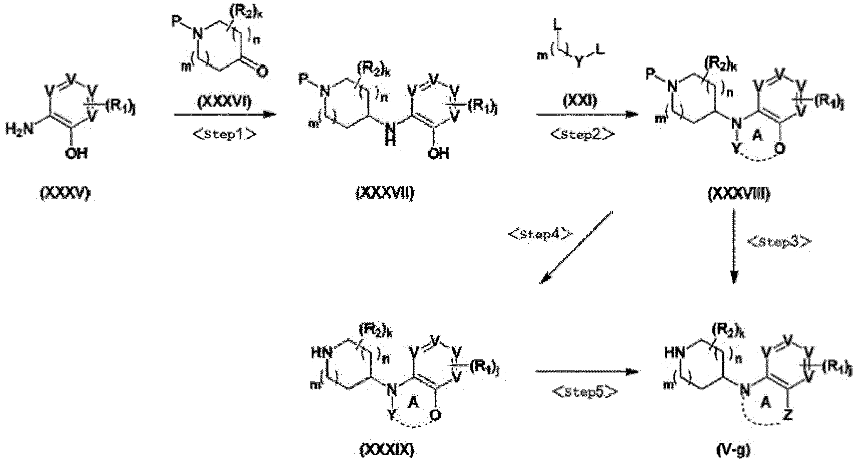

### <Step 1>

The compound of formula (XXXVII) can be produced by the same process as used in (Process 1-5), using the compound represented by formula (XXXV) and the compound represented by formula (XXXVI).

### <Step 2>

The compound represented by formula (XXXVIII) can be produced by modifications of known processes described in literatures, e.g., JIKKEN KAGAKU KOZA (Book Series of Experiments in Chemistry) (4th edition, 23 Synthesis of Organic Compound II, Alcohol/Amine, 187-243, 1992, Maruzen Publishing Co.), using the compound represented by formula (XXXVII) and the compound represented by formula (XXI), where the reaction is carried out, in the presence or absence of a base such as sodium hydride, triethylamine, pyridine, sodium hydrogencarbonate, potassium carbonate, etc., in a solvent inert to the reaction, e.g., a polar solvent such as N,N-dimethylformamide, acetonitrile, dimethylsulfoxide, etc., a halogenated solvent such as dichloromethane, chloroform, etc., an ether solvent such as dimethoxyethane, diethyl ether, tetrahydrofuran, etc., an aromatic hydrocarbon solvent such as toluene, benzene, etc., or a hydrocarbon solvent such as hexane, heptane, cyclohexane, etc., at temperatures from 0°C to the solvent-reflux temperature.

### <Step 3>

The compound of formula (V-g) can be produced by the same process as used in (Process A) <Step 2>, using the compound represented by formula (XXXVIII).

### <Step 4>

The compound of formula (XXXIX) can be produced by the same process as used in (Process A) <Step 2>, using the compound represented by formula (XXXVIII).

### <Step 5>

The compound of formula (V-g) can be produced by the same process as used in (Process C) <Step 3>, using the compound represented by formula (XXXIX).

The compounds or pharmaceutical compositions of the present invention may be used concomitantly with other drugs or agents in a manner conventionally applied to the clinical setting. These other drugs or agents available for concomitant use include, for example, analgesics such as acetaminophen, aspirin, opioid agonists (specifically, morphine, fentanyl, oxycodone, methadone, codeine, cocaine, pethidine, opium, ipecac, etc.), non-narcotic analgesics (pentazine, buprenorphine, nalorphine, cyclazocine, butorphanol, etc.), antidepressants (duloxetine, amitriptyline, imipramine, clomipramine, trimipramine, lofepramine, dosulepin, desipramine, amoxapine, nortriptyline, fluoxetine, fluvoxamine, maprotiline, mianserin, setiptiline, trazodone, etc.), antiepileptic agents or anticonvulsants (carbamazepine, phenytoin, gabapentin, pregabalin, phenobarbital, primidone, mephenytoin, nirvanol, ethotoin, trimethadione, ethosuximide, acetylpheneturide, zonisamide, acetazolamide, diazepam, clonazepam, nitrazepam, diphenylhydantoin, valproic acid, baclofen, etc.), antiarrhythmic drugs (quinidine, disopyramide, procainamide, ajmaline, prajmalium, cibenzoline, lidocaine, aprindine, tonicaid, phenytoin, flecainide, pilcicainide, propafenone, propranolol, amiodarone, verapamil, bepridil, etc., in addition to mexiletine diverted/prescribed for neuropathic pain), NSAIDs (etodolac, meloxicam, nimesulid, sodium diclofenac, mefenamic acid, zaltoprofen, sodium loxoprofen, sulindac, nabumetone, diflunisal, piroxicam, ibuprofen, naproxen, fenoprofen, acetylsalicylic acid, tolmetin, indomethacin, flurbiprofen, oxaprozin, ketoprofen, mofezolac, acetaminophen, ketorolac, zomepirac, nitroaspirin, tiaprofen, ampiroxicam, tiaramide, epirizole, etc.), antiinflammatory drugs such as COX-2 inhibitors (celecoxib, rofecoxib, etc.), etc., NR2B antagonists, bradykinin antagonists, and antimigraine drugs.

In addition, the concomitant drugs include local anesthetics (quinidine, disopyramide, procainamide, ajmaline, prajmalium, cibenzoline, lidocaine, mexiletine, aprindine, tonicaid, phenytoin, flecainide, pilcicainide, propafenone, propranolol, amiodarone, verapamil, bepridil, etc.), anesthetic agents (specifically, benzodiazepine, diazepam, midazolam, thiopental, thiamylal, propofol, baclofen, droperidol, sufentanil, etc.), and NMDA antagonists (specifically, ketamine, dextromethorphan, memantine, amantadine, etc.).

Further included are concomitant use with α2 adrenaline receptor agonists (clonidine, dexmedetomidine, tizanidine, guanfacine, guanabenz, etc.), calcium channel antagonists, potassium channel openers, etc., concomitant use with drugs for external application (capsaicin cream), or concomitant use with antiviral agents (vidarabine, aciclovir, ganciclovir, zidovudine, didanosine, amantadine, idoxuridine, α- or β-interferon, etc.). Preferably, other drugs or agents for concomitant use are morphine, gabapentin, pregabalin, diclofenac, celecoxib, etc.

The treatment may be performed not only by concomitant use with other drugs but also in combination with other therapy. The stimulation analgesic method specifically includes, for example, acupuncture, percutaneous electricity needle stimulation therapy, percutaneous electricity nerve stimulation therapy, silver spike point (SSP) treatment, peripheral nerve stimulus, spine electricity stimulus, electric spasm treatment, laser treatment, low-frequency therapy, nerve block (specifically, stellate ganglion block, epidural block, brachial plexus block, nerve root block, thoracic/lumbar sympathetic ganglion block, trigger point block, subarachnoid block, trigeminal nerve block, sympathetic nerve block, local infiltration block, peripheral nerve block, etc.) and the like.

By concomitant use with the existing drugs for diseases mentioned above, dosage of the existing drugs can be reduced, which results in alleviating side effects of the existing drugs. Of course, the concomitant use method using these drugs is not limited to the diseases mentioned above, and the drugs illustratively given for concomitant use are not limited to those shown by way of example.

Where the compound of the invention is used in combination with the concomitant drugs, they may be used in separate pharmaceutical preparations or may be formulated in one pharmaceutical preparation. In the separate pharmaceutical preparations, both may be administered simultaneously or at different times.

### [Pharmaceutical Dosage Formulation of Agent for Prevention/Treatment of the Invention]

The drug of the invention may be administered in the form of a pharmaceutical composition.

The pharmaceutical composition of the present invention may contain at least one of the compounds represented by formula (I) of the present invention, which is prepared in combination with pharmaceutically acceptable additives. More specifically, the pharmaceutical composition may be prepared into various dosage forms using the compounds of the invention in appropriate combination with recipients (e.g., lactose, sucrose, mannitol, crystalline cellulose, hydrated silica, corn starch and potato starch), binders (e.g., celluloses (hydroxypropylcellulose (HPC) and hydroxypropylmethylcellulose (HPMC)), crystalline cellulose, sugars (lactose, mannitol, sucrose, sorbitol, erythritol and xylitol), starch (corn starch and potato starch, α starch, dextrin, polyvinylpyrrolidone (PVP), macrogol, polyvinyl alcohol (PVA)), lubricants (e.g., magnesium stearate, calcium stearate, talc and carboxymethylcellulose), disintegrants (e.g., starch (corn starch and potato starch), sodium carboxymethylstarch, calmerose, calmerose calcium, croscarmellose sodium and crosspopidone), coating agents (e.g., celluloses (hydroxypropyl cellulose (HPC), hydroxypropylmethyl cellulose (HPMC), aminoalkyl methacrylate copolymer E and methacrylic acid copolymer LD), plasticizers (e.g., triethyl citrate and macrogol), shielding agents (e.g., titanium oxide), coloring agents, corrigents, antiseptic agents (e.g., benzalkonium chloride and paraoxybenzoic acid esters), isotonic agents (e.g., glycerin, sodium chloride, calcium chloride, mannitol and glucose), pH adjuster (e.g., sodium hydroxide, potassium hydroxide, sodium carbonate, hydrochloric acid, sulfuric acid and buffer such as phosphate-buffer), stabilizers (e.g., sugar, sugar alcohol and xanthan gum), dispersants, anti-oxidants (e.g., ascorbic acid, butylhydroxyanisol (BHA), propyl gallate and d1-α-tocopherol), buffering agents, preservers (e.g., paraben, benzyl alcohol and benzalkonium chloride), flavoring agents (e.g., vanillin, 1-menthol and rose oil), solubilizing agents (e.g., polyoxyethylene hydrogenated castor oil, polysorbate 80, polyethylene glycol, phospholipid cholesterol and triethanolamine), absorption enhancers (e.g., sodium glycol ate, sodium edetate, sodium caproate, acylcarnitines and limonene), gelatinizers, suspension enhancers, emulsifiers, and appropriate additives or solvents generally used.

Various dosage forms include tablets, capsules, granules, powders, pills, aerosols, inhalers, ointments, patches, suppositories, injections, lozenges, liquids, spirits, suspensions, extracts, elixirs, etc. These dosage forms may be administered to the patient through oral, subcutaneous, intramuscular, intranasal, percutaneous, intravenous, intraarterial, perineural, peridural, intrathecal, intraventricular or intrarectal route, or through inhalation, etc.

A daily dose of the compound of the present invention is generally in a range from 0.005 mg to 3.0 g, preferably 0.05 mg to 2.5 g and more preferably 0.1 mg to 1.5 g, and may be appropriately varied depending upon conditions or administration routes.

The total dose may be orally or parenterally administered as a single dose or in divided doses 2 to 6 times, or may be administered through drip infusion or consecutively.

Furthermore, all publications cited in the specification, such as related art documents, laid-open patent applications, patent publications and other patent documents, are incorporated in their entirety into the present specification as references.

### [Pharmacological Experiments]

Hereinafter, the present invention is described more specifically with reference to the Experiments but not deemed to be limited thereto. Pharmacological Experiment 1: In Vitro Assessment of Compounds

### (1) Preparation of Microsomal Fraction of Human-Derived Monocytic Leukocyte Cell Line (U-937)

The cultured U-937 cells were harvested, and 0.33-fold volume of buffer solution (50 mmol/L HEPES-NaOH (pH 7.4), 1 mmol/L EDTA, Complete EDTA-free (Roche Diagnostics)) was added to the cells, which was suspended and homogenized. After ultrasonication, the cells were further homogenized and centrifuged (600 g, 10 mins., 4°C) to collect the supernatant. The supernatant collected was again centrifuged (12000 g, 20 mins., 4°C), and the supernatant was ultracentrifuged (100000 g, 60 mins., 4°C) to collect the precipitates. The precipitates were suspended in a buffer solution (50 mmol/L HEPES-NaOH (pH 7.4), 1mmol/L EDTA) of 5-fold volume with respect to the wet weight of the precipitates to quantify the protein level with the BCA Protein Assay (PIERCE).

### (2) Preparation of Rat-Brain Microsomal Fraction

Male Sprague-Dawley (SD) rats of 10 weeks old were sacrificed by decapitation and the brains were removed. To the brains was added a buffer solution (50 mmol/L HEPES (pH 8.0), 1 mmol/L EDTA, 0.32 mol/L sucrose, Complete EDTA-free (Roche Diagnostics)) of 5-fold volume with respect to the brain wet weight, which was homogenized in a Potter homogenizer. The homogenate was centrifuged (9000 g, 10 mins., 4°C) to collect the supernatant. The supernatant was further ultracentrifuged (105000 g, 60 mins., 4°C) to collect the precipitates. The precipitates were suspended in a buffer solution (50 mmol/L HEPES (pH 8.0), 1 mmol/L EDTA) of 1/4 volume with respect to the wet weight of the precipitates to quantify the protein level with the BCA Protein Assay (PIERCE).

### (3) In Vitro Assessment of Compounds Using Microsomal Fraction

In a 384 well plate (Coming), were added the dilutions of compounds to be tested in various concentrations with 30 µL of a buffer solution (50 mmol/L HEPES (pH 7.4), 1 mmol/L EDTA, 0.1% bovine serum albumin) and the U-937 microsomal fraction (final concentration of 6 µg/well) prepared in (1) or the rat brain microsomal fraction (final concentration of 4 µg/well) prepared in (2), followed by preincubation for 5 minutes at room temperature. AAMCA (SIGMA, (final concentration of 4 µmol/L) as a substrate was added thereto, and the U-937 microsomal fraction was incubated for 120 minutes and the rat brain microsomal fraction for 30 minutes, at room temperature. Before and after the incubation, fluorescence counts were measured using EnVision 2000 (Perkin Elmer) at an excitation wavelength of 355 nm with an emission wavelength of 460 nm to calculate the difference. The count of the well added with the solvent in place of the compound tested and the count of the well added with no microsome were made 0% and 100%, respectively, and the inhibitory activity of each compound tested was determined.

The FAAH inhibitory activity of the test compound was expressed by IC₅₀ value. The compound of the present invention showing the IC₅₀ value smaller than 0.1 µmol/L, the compound showing the IC₅₀ value not smaller than 0.1 µmol/L and smaller than 1 µmol/L and the compound showing the IC₅₀ value not smaller than 1 µmol/L were designated as +++, ++ and +, respectively, which are shown in TABLE 1.

**[TABLE 1] FAAH Inhibitory Activity**

| EXAMPLE | FAAH Inhibitory Activity IC₅₀ | | EXAMPLE | FAAH Inhibitory Activity IC₅₀ | |
|---|---|---|---|---|---|
| | U-937 microsome | Rat brain microsome | | U-937 microsome | Rat brain microsome |
| 1 | ++ | + | 18 | +++ | ++ |
| 5 | ++ | ++ | 21 | ++ | ++ |
| 9 | + | ++ | 22 | + | + |
| 10 | ++ | ++ | 24 | +++ | ++ |
| 12 | ++ | +++ | 35 | +++ | +++ |
| 15 | ++ | ++ | 52 | +++ | +++ |
| 17 | +++ | +++ | 67 | +++ | +++ |

### Pharmacological Experiment 2: In Vivo Assessment of Compounds

The activity of the compounds in accordance with the present invention against various pains could be assessed by the following procedures.

### (1) Rat Formalin Test

Male SD rats of 6 to 7 weeks old are used for the test. Vehicle or a test compound is orally given to rats in a volume of 10 mL/kg. For a positive control drug, 50 mg/kg of gabapentin is used. The rats are injected subcutaneously in their footpad of hind limb with 50 µL of 0.5% formaldehyde solution and their behaviors are observed for 45 minutes. In the behavior observation, the duration time of nociceptive behaviors (licking or biting) of the formalin-injected paw is measured every 5 minutes. The nociceptive response induced by formalin stimulation is displayed as a biphasic behavior (J. Pharmacol. Exp. Ther. 263, 136-146, 1992); the nociceptive response observed within 10 minutes after the injection is termed a phase 1 response, while the response observed between 10 minutes and 45 minutes is termed a phase 2 response. The sum of duration of nociceptive behaviors measured in the phase 2 response of each rat serves as an index of nociceptive behaviors.

### (2) Neuropathic Pain Model (CCI Model)

The chronic constriction injury (CCI) model is created by some modifications of the report by Bennett et al. (Pain, 33, 87-107, 1988). Under pentobarbital anesthesia, male SD rats of 7 weeks old undergo an incision of the skin overlying the left distal femur and the left biceps femoris muscle is then exposed by blunt incision. The sciatic nerve is detached from the surrounding tissue and loosely entrapped at 4 sites with surgical braided silk thread 4-0 (Matsuda Ika-Kogyo Co.) in intervals of about 1 mm.

The nociceptive effect is assessed according to modifications of Seltzer, et al. (Pain, 43, 205-218, 1990). More specifically, 2 weeks after production of the chronic constriction injury model, the rat is mechanically stimulated by repetitively pressing von Frey filaments (Stoelting) for 3 seconds against the footpad of the hind limb at 2 frequencies per second from increasing order. The pressure values at which the rats withdraw quickly their hind limb from the stimulus are designated as the reaction thresholds.

Reduction in the reaction threshold induced by CCI is restored by an oral administration of a test compound to the rat, i.e., the efficacy of the compound as an agent for the treatment of neuropathic pain is established.

### (3) Inflammatory Pain Model (CFA-Induced Rat)

The CFA-induced rat inflammatory pain model is produced by some modifications of general methods, e.g., the method of Pomonis J.D., et al. (The Journal of Pharmacology and Experimental Therapeutics, 306, 387-393, 2003). Specifically, Complete Freund's Adjuvant (CFA, SIGMA) is mixed with equal volume of saline to prepare an emulsion, and a 100 µl aliquot of the emulsion is injected into the footpad of right hind limb.

When a test compound is orally given to the rat 1, 2 or 5 days after the CFA administration, the reduction in the nociceptive threshold is prevented, i.e., establishing the efficacy as an agent for the treatment of inflammatory pain.

### (4) Mouse PQ Writhing

Mouse PQ writhing is produced by the method of Mustafa A.A., et al. (General Pharmacology, 23, 1177-1182, 1992). Specifically, phenyl p-quinone diluted in saline is intraperitoneally administered to mice. The frequency of behaviors including body stretching, writhing, huddling, etc. is then recorded in a given period of time.

By administration of a test compound to mice prior to administration of phenyl p-quinone, the frequency of the behaviors such as stretching, writhing, huddling, etc. decreases, indicating the efficacy of the compound.

### (5) Mouse Model of Cancer-Induced Pain

Efficacy in cancer pain is assessed in a murine model with transplanted femoral bone cancer. The model is produced by some modifications of the method by Namiki A., et al. (British Journal of Anaesthesia, 102, 251-258, 2009). Specifically, the male mouse undergoes incision of the left patella under pentobarbital anesthesia followed by transplantation of mouse osteosarcoma cells NCTC clone 2472 (ATCC) into the left femoral bone from distal site. Pain is assessed by measuring the frequency of flinching/lifting, use state of the left hind limb during natural walking and degree of weight bearing on the left hind limb, 16 days after the transplantation of osteosarcoma cells.

When an improvement of the evaluation items is observed by the administration of the test compound, its efficacy in cancer pain is established. Pharmacological Experiment 3: Solubility Test

### (1) DMSO Precipitation Solubility (Kinetic Solubility)

A 10 mM solution of the compound of the invention in DMSO is added to 50 mM phosphate-buffered saline (pH 7.4) in a final concentration of 100 µM. The solution is incubated at room temperature for 1.5 hours while stirring at 600 rpm, and then filtered through a filter plate (4 µm, MultiScreen Solubility Filter Plate (Millipore)). Absorbance of the filtrate is measured on a plate reader (Powerscan HT (Dainippon Pharmaceutical Co., Ltd.)) at the maximum absorption wavelength. At the same time, solutions containing the test compound of known concentrations (1, 3, 10, 30 and 100 µM) are prepared as standard solutions for calibration; absorbance in each of the standard solutions in different concentrations is measured to prepare a calibration curve. The solubility (µM) of the compound is determined from the absorbance values of the filtrate and standard solutions.

### (2) Thermodynamic Solubility

The compound of the invention is added to water in a concentration of 1 mg/mL. The solution is allowed to stand at 37°C for 24 hours and then centrifuged. Using HPLC the peak is detected in the resulting supernatant at the maximum absorption wavelength to measure the peak area. In the same manner, each peak area is measured using solutions containing known concentrations (0.03, 0.1, 0.3, 1, 3 and 10 µg/ mL) of a test compound as standard solutions for calibration, and the solubility (µg/ mL) of the compound is determined from the peak area of the calibration curve.

### Pharmacological Experiment 4: Metabolic Stability Test

A 10 mM solution of the compound of the invention in DMSO is added to a liver microsome solution (human, rat; XenoTech) and NADPH-regenerating solution (water containing β-NADP, glucose-6-phosphate, G-6-PDH(Y) and MgCl₂) in a concentration of 1 µM. After incubation of the solution at 37°C for 10 minutes or 20 minutes, the reaction is stopped by addition of acetonitrile. The reaction solution is centrifuged and filtered through a filter plate (MultiScreen HTS-HV Plate (Millipore)). A test compound in the filtrate is measured using high performance liquid chromatography/mass spectrometry. Likewise, a sample with 0 reaction time is measured as a control, and the metabolic rate and degradation rate (%) are determined by comparing the microsome reaction sample with the control. Pharmacological Experiment 5: hERG Inhibitory Test by Patch Clamp Method

An effect on hERG (a human ether-α-go-go related gene) channel is measured with a fully automated patch-clamp system (Patchliner (Nanion)). To confirm the hERG I_{Kr} current in cells, a depolarization pulse is applied on a regular basis, while membrane potential is clamped at -80 mV. After the generated current is stabilized, a test compound is added to a perfusate. The effect of the test compound on the hERG channel is confirmed by changes in tail current induced by applying depolarization pulses having a voltage of 40 mV for 0.5 seconds and subsequent repolarization pulse having a voltage of -40 mV for 0.5 seconds. The stimulus is given once every 10 seconds. The measurement is performed at room temperature. The hERG channel inhibitory activity is calculated as a reduction ratio (suppression rate) of the tail current 2 minutes before addition of a test compound, when compared to the maximum tail current.

Calculation of this inhibitory activity enables to estimate the drug-induced QT prolongation and subsequent fatal adverse effects (ventricular tachycardia, sudden death, etc.).

### Pharmacological Experiment 6: Protein Binding Rate Test

A 10 mM solution of the compound of the invention in DMSO is added to normal plasma (human, rat) in a final concentration of 10 µM. After dialysis at 37°C for 4 hours with a rapid equilibrium dialysis device (RED Device (Linden Bioscience)), the solution inside the dialysis membrane (plasma side) and the solution outside the dialysis membrane (PBS side) are subjected to high performance liquid chromatography/mass spectrometry to measure the test compound in the samples. The fraction unbound (%) is calculated from a ratio of the PBS side to the plasma side, and the protein binding rate (%) is calculated from 100 - the fraction unbound (%).

### Pharmacological Experiment 7: Pharmacokinetics Test (Rat Cassette PK)

The compound of the invention is administering to male Slc:SD of 7 or 8 weeks old in a bolus dose of 1mg/kg (solvent for administration, DMSO : Tween 80 : ultrapure water = 1:1:8, 10 mL/kg). Subsequently, blood is collected from the cervical vein 0.5, 1, 2 and 4 hours after the administration. Blood is centrifuged (3000 rpm, 15 mins., 4°C). Using the plasma obtained, the test compound in the plasma is assayed by high performance liquid chromatography/mass spectrometry. Likewise, concentrations (µg/mL) in the plasma is determined from a calibration curve prepared using standard solutions of the test compound having known concentrations (0.01, 0.02, 0.05, 0.1, 0.2, 0.5 and 1 µg/mL). The maximum plasma concentration is expressed as Cmax (µg/mL).

The foregoing results reveal that the compounds of the invention possess an excellent FAAH inhibitory activity. The results also establish the analgesic effects in the *in vivo* pain models.

Furthermore, the preferred compounds of the invention possess FAAH-selective inhibitory activity and show the hERG (human ether-a-go-go related gene) inhibitory activity not less than 10 µmol/L in terms of the IC₅₀ value.

Accordingly, the compounds of the invention are expected to be used as an FAAH-selective inhibitor, e.g. agents for the prevention or treatment of pain, in particular, as agents for the prevention or treatment of neuropathic pain, fibromyalgia syndrome, inflammatory pain, cancer-induced pain or diabetic neuralgia.

### [EXAMPLES]

Next, the present invention will be described in more detail by referring to EXAMPLES, but the present invention is not deemed to be limited to these EXAMPLES.

The measurement of nuclear magnetic resonance (NMR) spectrum was performed using JEOL JNM-ECX300 (JEOL JNM-ECX300), FT-NMR (manufactured by JEOL), JEOL JNM-ECX400 (JEOL JNM-ECX400) or FT-NMR (manufactured by JEOL). LC Mass was analyzed using Waters FractionLynx MS System (manufactured by Waters Corporation), in which SunFire column (4.6 mm x 5cm, 5µ**m**) manufactured by Waters Corporation was used as a column and methanol and 0.05% aqueous acetic acid solution as a moving phase under gradient conditions of methanol : 0.05% aqueous acetic acid solution = 1:9 (0 min) to 9:1 (5 mins) to 9:1 (7.5 mins).

HPLC analysis was performed using Prominence (manufactured by Shimadzu Corporation).

### (EXAMPLE 1)

### N-Phenyl-4-(6-fluoro-1,2-benzisoxazo-3-yl)piperidine-1-carboxamide

Triethylamine (0.14 mL) and phenyl isocyanate (0.058 g) were added to a solution of 4-(6-fluoro-1,2-benzisoxazo-3-yl)piperidine (0.11 g) in methylene chloride (3 mL), followed by stirring overnight. After water was added to the reaction solution, the mixture was extracted with methylene chloride, washed with brine and then dried over anhydrous sodium sulfate. The solvent was removed by distillation under reduced pressure and the resulting residue was purified by silica gel column chromatography to give the title compound (0.090 g) as a white solid.

### (EXAMPLE 2)

### N-Pyridazin-3-yl-4-(5-bromo-3,4-dihydro-2H-1-benzopyran-4-yl)piperazine-1-carbo xamide

### <Step 1> Synthesis of 3-(3-bromophenoxy)propionic acid

Under ice cooling, 3-bromophenol (20.0 g) was added to a suspension of sodium hydride (5.09 g) in N,N-dimethylformamide (133 mL). The mixture was stirred at room temperature for an hour. Subsequently under ice cooling, β-propiolactone (9.45ml) was added to the mixture, followed by stirring at room temperature for 2 hours. After pH was adjusted to 3 by addition of hydrochloric acid, the mixture was extracted with ethyl acetate. The organic layer was washed with water and brine and then dried over anhydrous sodium sulfate. The solvent was removed by distillation under reduced pressure to give the title compound (30.9 g) as a white solid.

### <Step 2> Synthesis of 5-bromochroman-4-one (2-2-a) and 7-bromochroman-4-one (2-2-b)

Diphosphorus pentoxide (17.9 g) was added to methanesulfonic acid (82.0 mL), and the compound (15.4 g) obtained in (EXAMPLE 2) <Step 1> was then added to the mixture at 80°C. After stirring for 3 minutes, the reaction solution was poured onto ice and extracted with ethyl acetate. The organic layer was washed with water, saturated sodium hydrogencarbonate aqueous solution and brine, and then dried over anhydrous sodium sulfate. The solvent was removed by distillation under reduced pressure and the resulting residue was purified by silica gel column chromatography (eluate; n-hexane : ethyl acetate = 100:0 to 75:25) to give a mixture (9.8 g) of the title compounds (2-2-a and 2-2-b) as a purple solid.

### <Step 3> Synthesis of 4-[2-(t-butoxycarbonylamino)ethylamino]-5-bromo-3,4-dihydro-2H-1-benzopyrane and 4-[2-(t-butoxycarbonylamino)ethylamino]-7-bromo-3,4-dihydro-2H-1-benzopyrane

Triethylamine (17.8 mL), acetic acid (6.03 mL), magnesium sulfate (16.0 g) and molecular sieve (16.0 g) were added to a suspension of the mixture (16.0 g) obtained in (EXAMPLE 2) <Step 2> and N-t-butoxycarbonyl-1,2-diaminoethane hydrochloride (20.3 g) in ethanol (1117 mL). The mixture was stirred at room temperature for an hour. Sodium cyanotrihydroborate (5.74 g) was further added to the mixture, which was heated to reflux. A reaction was carried out in a similar manner using the compound (1.00 g) obtained in (EXAMPLE 2) <Step 2>. The insoluble matters were combined and filtered through Celite, followed by concentration under reduced pressure. After an aqueous sodium hydroxide solution and water were added, the mixture was extracted with ethyl acetate. The organic layer was washed with brine and dried over anhydrous sodium sulfate. The residue obtained by distillation of the solvent under reduced pressure was purified by silica gel column chromatography (eluate; n-hexane : ethyl acetate = 95:5 to 50:50) to give a mixture (18.0 g) of the title compounds as an orange oily substance.

### <Step 4> Synthesis of 4-[1-(t-butoxycarbonyl)-3-oxopiperazin-4-yl]-5-bromo-3,4-dihydro-2H-1-benzopyra ne and 4-[1-(t-butoxycarbonyl)-3-oxopiperazin-4-yl]-7-bromo-3,4-dihydro-2H-1-benzopyra ne

Under ice cooling, triethylamine (7.45 mL) and bromoacetyl bromide (4.66 mL) were added to a solution of the mixture (18.0 g) obtained in (EXAMPLE 2) <Step 3> in dichloromethane (400 mL). The mixture was stirred for 0.5 hours. After 0.2 N hydrochloric acid ice-cooled was added thereto, the mixture was extracted with dichloromethane. The organic layer was washed with brine and dried over anhydrous sodium sulfate. The solvent was removed by distillation under reduced pressure. A solution of the resulting residue in N,N-dimethylformamide (20 mL) was added to a suspension of 60% sodium hydride (3.21 g) in N,N-dimethylformamide (100 mL), followed by stirring at room temperature for an hour. Sodium hydride (0.38 g) was further added thereto, and the mixture was stirred overnight. After water was added thereto, the mixture was extracted with ethyl acetate. The organic layer was washed with brine and dried over anhydrous sodium sulfate. The solvent was removed by distillation under reduced pressure and the resulting residue was purified by silica gel column chromatography (eluate; n-hexane : ethyl acetate = 87:13 to 0:100) to give a mixture (11.4 g) of the title compounds as a pale yellow amorphous substance.

### <Step 5> Synthesis of 4-(2-oxopiperazin-1-yl)-5-bromo-3,4-dihydro-2H-1-benzopyrane and 4-(2-oxopiperazin-1-yl)-7-bromo-3,4-dihydro-2H-1-benzopyrane

To the mixture (10.3 g) obtained in (EXAMPLE 2) <Step 4> was added 4N hydrogen chloride-ethyl acetate solution (30.0 mL). The mixture was stirred for 3 hours. The solvent was removed by distillation under reduced pressure, and triethylamine was added to the resulting residue. The mixture was purified by silica gel column chromatography (eluate; dichloromethane : methanol = 95:5) to give a mixture (7.4 g) of the title compounds as a pale yellow solid.

### <Step 6> Synthesis of 4-piperazin-1-yl-5-bromo-3,4-dihydro-2H-1-benzopyrane and 4-piperazin-1-yl-7-bromo-3,4-dihydro-2H-1-benzopyrane

Under ice cooling, a 1M boran-tetrahydrofuran/tetrahydrofuran solution (7.42 mL) was added to a solution of the mixture (0.77 g) obtained in (EXAMPLE 2) <Step 5> in tetrahydrofuran (13 mL). The mixture was stirred at room temperature for 4 hours. Methanol and methanolic hydrochloric acid were added thereto, which was heated to reflux for an hour. After 1N sodium hydroxide aqueous solution was added thereto, the mixture was extracted with ethyl acetate. The organic layer was washed with brine and then dried over anhydrous sodium sulfate. The solvent was removed by distillation under reduced pressure to give a mixture (0.64 g) of the title compounds as a pale yellow oily compound.

### <Step 7> Synthesis of N-pyridazin-3-yl-4-(5-bromo-3,4-dihydro-2H-1-benzopyran-4-yl)piperazine-1-carbo xamide

To a solution of the mixture (3.59 g) obtained in (EXAMPLE 2) <Step 6> in acetonitrile (41 mL) were added diisopropylethylamine (4.11 mL) and phenyl pyridazin-3-ylcarbamate (3.90 g). The mixture was stirred for 3 hours. The solvent was removed by distillation under reduced pressure, and the residue was extracted with ethyl acetate. The organic layer was washed with brine and dried over anhydrous sodium sulfate. The solvent was removed by distillation under reduced pressure and the residue obtained was purified by silica gel column chromatography (eluate; n-hexane : ethyl acetate : methanol = 60:40:0 to 0:100:0 to 0:90:10) to give the title compound (0.27 g) as a yellow amorphous substance.

### (EXAMPLE 3)

### N-Pyridazin-3-yl-4-(7-bromo-3,4-dihydro-2H-1-benzopyran-4-yl)piperazine-1-carbo xamide

In (EXAMPLE 2) <Step 7>, the title compound (1.68 g) was obtained as a yellow amorphous substance.

### (EXAMPLE 4)

### N-Pyridazin-3-yl-4-(6-(trifluoromethyl)-3,4-dihydro-2H-1-benzopyran-4-yl]piperazi ne-1-carboxamide

### <Step 1> Synthesis of 4-[2-(benzyloxybenzyloxycarbonylamino)ethylamino]-6-(trifluoromethyl)-3,4-dihyd ro-2H-1-benzopyrane

The title compound (8.13 g) was obtained as a yellow oily substance in a manner similar to a modification of the process of (EXAMPLE 2) <Step 3>, in which 6-(trifluoromethyl)-chroman-4-one (5.10 g) was used as the starting material.

### <Step 2> Synthesis of 4-[1-(benzyloxycarbonyl)-3-oxopiperazin-4-yl]-6-(trifluoromethyl)-3,4-dihydro-2H-1-benzopyrane

The title compound (5.74 g) was obtained as a yellow amorphous substance in a manner similar to a modification of the process of (EXAMPLE 2) <Step 4>, in which the compound (8.04 g) obtained in (EXAMPLE 4) <Step 1> was used as the starting material.

### <Step 3> Synthesis of 4-(2-oxopiperazin-1-yl)-6-(trifluoromethyl)-3,4-dihydro-2H-1-benzopyrane

After palladium carbon was added to a methanol solution (150 mL) of the compound (5.74 g) obtained in (EXAMPLE 4) <Step 2>, the mixture was stirred for 5 hours in a hydrogen atmosphere. The mixture was filtered through a celite and the filtrate was concentrated under reduced pressure to give the title compound (4.35 g) as a yellow oily substance.

### <Step 4> Synthesis of 4-piperazine-1-yl-6-(trifluoromethyl)-3,4-dihydro-2H-1-benzopyrane

The title compound (3.66 g) was obtained as a yellow oily substance in a manner similar to (EXAMPLE 2) <Step 6>, in which the compound (3.95 g) obtained in (EXAMPLE 4) <Step 3> was used as the starting material.

### <Step 5> Synthesis of N-pyridazin-3-yl-4-[6-(trifluoromethyl)-3,4-dihydro-2H-1-benzopyran-4-yl]piperazi ne-1-carboxamide

The title compound (1.44 g) was obtained as a yellow amorphous substance in a manner similar to (EXAMPLE 2) <Step 7>, in which the compound (1.92 g) obtained in (EXAMPLE 4) <Step 4> was used as the starting material.

### (EXAMPLE 5)

### N-Pyridazin-3-yl-4-[6-[[5-(trifluoromethyl)-2-pyridinyl]oxy]-3,4-dihydro-2H-1-benz opyran-4-yl]piperazine-1-carboxamide

### <Step 1> Synthesis of 6-[[5-(trifluoromethyl)-2-pyridinyl]oxy]-chroman-4-one

To a solution of 6-hydroxychroman-4-one (0.55 g) in acetonitrile (50.0 mL) were added 2-chloro-5-(trifluoromethyl)pyridine (1.21 g) and potassium carbonate (1.11 g). The mixture was heated to reflux for 23 hours. After water was added to the reaction solution, the mixture was extracted with ethyl acetate. The organic layer was washed with water and brine, and then dried over anhydrous sodium sulfate. The solvent was removed by distillation under reduced pressure. The resulting residue was purified by silica gel column chromatography (eluate; n-hexane : ethyl acetate = 90:10 to 50:50) to give the title compound (0.45 g) as a beige solid.

### <Step 2> Synthesis of 4-[2-(benzyloxycarbonylamino)ethylamino]-6-[[5-(trifluoromethyl)-2-pyridinyl]oxy] -3,4-dihydro-2H-1-benzopyrane

The title compound (0.64 g) was obtained as a brown oily substance in a manner similar to (EXAMPLE 2) <Step 3>, in which the compound (0.45 g) obtained in (EXAMPLE 5) <Step 1> was used as the starting material.

### <Step 3> Synthesis of 4-[1-(benzyloxycarbonyl)-3-oxopiperazin-4-yl]-6-[[5-(trifluoromethyl)-2-pyridinyl]o xy]-3,4-dihydro-2H-1-benzopyrane

The title compound (0.30 g) was obtained as a grayish white solid in a manner similar to (EXAMPLE 2) <Step 4>, in which the compound (0.64 g) obtained in (EXAMPLE 5) <Step 2> was used as the starting material.

### <Step 4> Synthesis of 4-(2-oxopiperazin-1-yl)-6-((5-(trifluoromethyl)-2-pyridinyl]oxy]-3,4-dihydro-2H-1-b enzopyrane

The title compound (0.26 g) was obtained as a colorless oily substance in a manner similar to (EXAMPLE 4) <Step 3>, in which the compound (0.30 g) obtained in (EXAMPLE 5) <Step 3> was used as the starting material.

### <Step 5> Synthesis of 4-piperazin-1-yl-6-[[5-(trifluoromethyl)-2-pyridinyl]oxy]-3,4-dihydro-2H-1-benzopy rane

The title compound (0.23 g) was obtained as a grayish white solid in a manner similar to (EXAMPLE 2) <Step 6>, in which the compound (0.26 g) obtained in (EXAMPLE 5) <Step 4> was used as the starting material.

### <Step 6> Synthesis of N-pyridazin-3-yl-4-[6-[[5-(trifluoromethyl)-2-pyridinyl]oxy]-3,4-dihydro-2H-1-benz opyran-4-yl]piperazine-1-carboxamide

The title compound (0.10 g) was obtained as a yellow amorphous substance in a manner similar to (EXAMPLE 2) <Step 7>, in which the compound (0.22 g) obtained in (EXAMPLE 5) <Step 5> was used as the starting material.

### (EXAMPLE 6)

### N-Pyridazin-3-yl-4-(7-(2,2,2-trifluoroethoxy)-3,4-dihydro-2H-1-benzopyran-4-yl]pip erazine-1-carboxamide

### <Step 1> Synthesis of 7-(2,2,2-trifluoroethoxy)chroman-4-one

To a solution of 7-hydroxychroman-4-one (1.90 g) synthesized by the process described in Tetrahedron, 63(52), 12986-12993, 2007 in acetone (30.0 mL) were added potassium carbonate (3.79 g) and trifluoroethyl trifluoromethanesulfonate (1.45 mL). The mixture was stirred for 6 hours. Water and 1N sodium hydroxide aqueous solution were added to the reaction solution, followed by extraction with ethyl acetate. The organic layer was washed with water and brine, and then dried over anhydrous sodium sulfate. The solvent was removed by distillation under reduced pressure to give the title compound (1.80 g) as a brown solid.

### <Step 2> Synthesis of 4-[2-(benzyloxycarbonylamino)ethylamino]-7-(2,2,2-trifluoroethoxy)-3,4-dihydro-2 H-1-benzopyrane

The title compound (2.50 g) was obtained as a pale yellow oily substance in a manner similar to (EXAMPLE 2) <Step 3>, in which the compound (1.90 g) obtained in (EXAMPLE 6) <Step 1> was used as the starting material.

### <Step 3> Synthesis of 4-(1-(benzyloxycarbonyl)-3-oxopiperazin-4-yl]-7-(2,2,2-trifluoroethoxy)-3,4-dihydro -2H-1-benzopyrane

The title compound (1.37 g) was obtained as a white amorphous substance in a manner similar to (EXAMPLE 2) <Step 4>, in which the compound (2.50 g) obtained in (EXAMPLE 6) <Step 2> was used as the starting material.

### <Step 4> Synthesis of 4-(2-oxopiperazin-1-yl)-7-(2,2,2-trifluoroethoxy)-3,4-dihydro-2H-1-benzopyrane

The title compound (0.92 g) was obtained as a colorless amorphous substance in a manner similar to (EXAMPLE 4) <Step 3>, in which the compound (1.30 g) obtained in (EXAMPLE 6) <Step 3> was used as the starting material.

### <Step 5> Synthesis of 4-piperazin-1-yl-7-(2,2,2-trifluoroethoxy)-3,4-dihydro-2H-1-benzopyrane

The title compound (0.78 g) was obtained as a colorless amorphous substance in a manner similar to (EXAMPLE 2) <Step 6>, in which the compound (0.91 g) obtained in (EXAMPLE 6) <Step 4> was used as the starting material.

### <Step 6> Synthesis of N-pyridazin-3-y1-4-(7-(2,2,2-trifluoroethoxy)-3,4-dihydro-2H-1-benzopyran-4-yl]pip erazine-1-carboxamide

The title compound (0.53 g) was obtained as a yellow amorphous substance in a manner similar to (EXAMPLE 2) <Step 7>, in which the compound (0.75 g) obtained in (EXAMPLE 6) <Step 5> was used as the starting material.

### (EXAMPLE 7)

### N-Pyridazin-3-yl-4-[7-[[5-(trifluoromethyl)-2-pyridinyl]oxy]-3,4-dihydro-2H-1-benz opyran-4-yl]piperazine-1-carboxamide

### <Step 1> Synthesis of 7-[[5-(trifluoromethyl)-2-pyridinyl]oxy]-chroman-4-one

The title compound (1.42 g) was obtained as a pale yellow oily substance in a manner similar to (EXAMPLE 5) <Step 1>, from 7-hydroxy-chroman-4-one (3.00 g) synthesized by the process described in Tetrahedron), 63(52), 12986-12993, 2007.

### <Step 2> Synthesis of 4-[2-(benzyloxycarbonylamino)ethylamino]-7-[[5-(trifluoromethyl)-2-pyridinyl]oxy] -3,4-dihydro-2H-1-benzopyrane

The title compound (2.20 g) was obtained as a colorless oily substance in a manner similar to (EXAMPLE 2) <Step 3>, in which the compound (1.40 g) obtained in (EXAMPLE 7) <Step 1> was used as the starting material.

### <Step 3> Synthesis of 4-[1-(benzyloxycarbonyl)-3-oxopiperazin-4-yl]-7-[[5-(trifluoromethyl)-2-pyridinyl]o xy]-3,4-dihydro-2H-1-benzopyrane

The title compound (1.30 g) was obtained as a pale yellow oily substance in a manner similar to (EXAMPLE 2) <Step 4>, in which the compound (2.20 g) obtained in (EXAMPLE 7) <Step 2> was used as the starting material.

### <Step 4> Synthesis of 4-(2-oxopiperazin-1-yl)-7-[[5-(trifluoromethyl)-2-pyridinyl]oxy]-3,4-dihydro-2H-1-b enzopyrane

The title compound (0.70 g) was obtained as a colorless oily substance in a manner similar to (EXAMPLE 4) <Step 3>, in which the compound (1.30 g) obtained in (EXAMPLE 7) <Step 3> was used as the starting material.

### <Step 5> Synthesis of 4-piperazin-1-yl-7-[[5-(trifluoromethyl)-2-pyridinyl]oxy]-3,4-dihydro-2H-1-benzopy rane

The title compound (0.64 g) was obtained as a colorless amorphous substance in a manner similar to (EXAMPLE 2) <Step 6>, in which the compound (0.70 g) obtained in (EXAMPLE 7) <Step 4> was used as the starting material.

### <Step 6> Synthesis of N-pyridazin-3-yl-4-[7-[[5-(trifluoromethyl)-2-pyridinyl]oxy]-3,4-dihydro-2H-1-benz opyran-4-yl]piperazine-1-carboxamide

The title compound (0.06 g) was obtained as a yellow amorphous substance in a manner similar to (EXAMPLE 2) <Step 7>, in which the compound (0.10 g) obtained in (EXAMPLE 7) <Step 5> was used as the starting material.

The resulting title compound was optically resolved into a first fraction (retention time: 10.9 mins.) and a second fraction (retention time: 13.6 mins.), using HPLC (column: CHIRALPAK AY-H (4.6 mm x 150 mm), eluate; hexane : ethanol = 50:50 (v/v), flow rate: 1.6 mL/min., UV: 254 nm detection, column temperature: 40°C).

### (EXAMPLE 8)

### N-Pyridazin-3-yl-4-(7-(trifluoromethoxy)-3,4-dihydro-2H-1-benzopyran-4-yl]pipera zine-1-carboxamide

### <Step 1> Synthesis of 4-[2-(benzyloxycarbonylamino)ethylamino]-7-(trifluoromethoxy)-3,4-dihydro-2H-1-benzopyrane

The title compound (4.77 g) was obtained as a yellow oily substance in a manner similar to (EXAMPLE 2) <Step 3>, in which 7-(trifluoromethoxy)-chroman-4-one (5.00 g) was used as the starting material. <Step 2> Synthesis of 4-[1-(benzyloxycarbonyl)-3-oxopiperazin-4-yl]-7-(trifluoromethoxy)-3,4-dihydro-2H -1-benzopyrane

The title compound (2.42 g) was obtained as a pale yellow oily substance in a manner similar to (EXAMPLE 2) <Step 4>, in which the compound (4.45 g) obtained in (EXAMPLE 8) <Step 1> was used as the starting material.

### <Step 3> Synthesis of 4-(2-oxopiperazin-1-yl)-7-(trifluoromethoxy)-3,4-dihydro-2H-1-benzopyrane

The title compound (1.70 g) was obtained as a colorless oily substance in a manner similar to (EXAMPLE 4) <Step 3>, in which the compound (2.40 g) obtained in (EXAMPLE 8) <Step 2> was used as the starting material.

### <Step 4> Synthesis of 4-piperazin-1-yl-7-(trifluoromethoxy)-3,4-dihydro-2H-1-benzopyrane

The title compound (2.00 g) was obtained as a yellow oily substance in a manner similar to (EXAMPLE 2) <Step 6>, in which the compound (1.70 g) obtained in (EXAMPLE 8) <Step 3> was used as the starting material.

### <Step 5> Synthesis of N-pyridazin-3-yl-4-[7-(trifluoromethoxy)-3,4-dihydro-2H-1-benzopyran-4-yl]piperaz ine-1-carboxamide

The title compound (0.90 g) was obtained as a yellow amorphous substance in a manner similar to (EXAMPLE 2) <Step 7>, in which the compound (2.00 g) obtained in (EXAMPLE 8) <Step 4> was used as the starting material.

### (EXAMPLE 9)

### N-Pyridazin-3-yl-4-(2,2-dimethyl-7-(trifluoromethyl)-3,4-dihydro-2H-1-benzopyran-4-yl]piperazine-1-carboxamide

### <Step 1> Synthesis of 4-[2-(benzyloxycarbonylamino)ethylamino]-2,2-dimethyl-7-(trifluoromethyl)-3,4-di hydro-2H-1-benzopyrane

The title compound (0.80 g) was obtained as a yellow oily substance in a manner similar to (EXAMPLE 2) <Step 3>, in which 2,2-dimethyl-7-(trifluoromethyl)-chroman-4-one (2.20 g) was used as the starting material.

### <Step 2> Synthesis of 4-[1-(benzyloxycarbonyl)-3-oxopiperazin-4-yl]-2,2-dimethyl-7-(trifluoromethyl)-3,4 -dihydro-2H-1-benzopyrane

The title compound (0.50 g) was obtained as a pale yellow oily substance in a manner similar to (EXAMPLE 2) <Step 4>, in which the compound (0.80 g) obtained in (EXAMPLE 9) <Step 1> was used as the starting material.

### <Step 3> Synthesis of 4-(2-oxopiperazin-l-yl)-2,2-dimethyl-7-(trifluoromethyl)-3,4-dihydro-2H-1-benzopy rane

The title compound (0.26 g) was obtained as a colorless amorphous substance in a manner similar to (EXAMPLE 4) <Step 3>, in which the compound (0.40 g) obtained in (EXAMPLE 9) <Step 2> was used as the starting material.

### <Step 4> Synthesis of 4-piperazin-1-yl-2,2-dimethyl-7-(trifluoromethyl)-3,4-dihydro-2H-1-benzopyrane

The title compound (0.24 g) was obtained as a yellow oily substance in a manner similar to (EXAMPLE 2) <Step 6>, in which the compound (0.25 g) obtained in (EXAMPLE 9) <Step 3> was used as the starting material.

### <Step 5> Synthesis of N-pyridazin-3-yl-4-[2,2-dimethyl-7-(trifluoromethyl)-3,4-dihydro-2H-1-benzopyran-4-yl]piperazine-1-carboxamide

The title compound (0.97 g) was obtained as a yellow amorphous substance in a manner similar to (EXAMPLE 2) <Step 7>, in which the compound (0.15 g) obtained in (EXAMPLE 9) <Step 4> was used as the starting material.

### (EXAMPLE 10)

### N-Pyridazin-3-yl-4-[8-(trifluoromethyl)-2,3,4,5-tetrahydrobenz-1-oxepin-5-yl]pipera zine-1-carboxamide

### <Step 1> Synthesis of 5-[2-(benzyloxycarbonylamino)ethylamino]-8-(trifluoromethyl)-2,3,4,5-tetrahydrobe nz-1-oxepine

The title compound (2.44 g) was obtained as a white solid in a manner similar to (EXAMPLE 2) <Step 3>, in which 8-(trifluoromethyl)-2,3,4,5-tetrahydrobenz-1-oxepin-5-one (1.80 g) was used as the starting material.

### <Step 2> Synthesis of 5-[1-(benzyloxycarbonyl)-3-oxopiperazin-4-yl]-8-(trifluoromethyl)-2,3,4,5-tetrahydr obenz-1-oxepine

The title compound (1.37 g) was obtained as a colorless amorphous substance in a manner similar to (EXAMPLE 2) <Step 4>, in which the compound (2.40 g) obtained in (EXAMPLE 10) <Step 1> was used as the starting material.

### <Step 3> Synthesis of 5-(2-oxopiperazin-1-yl)-8-(trifluoromethyl)-2,3,4,5-tetrahydrobenz-1-oxepine

The title compound (0.95 g) was obtained as a grayish white solid in a manner similar to (EXAMPLE 4) <Step 3>, in which the compound (1.37 g) obtained in (EXAMPLE 10) <Step 2> was used as the starting material.

### <Step 4> Synthesis of 5-piperazin-1-yl-8-(trifluoromethyl)-2,3,4,5-tetrahydrobenz-1-oxepine

The title compound (1.00 g) was obtained as a light brown oily substance in a manner similar to (EXAMPLE 2) <Step 6>, in which the compound (0.95 g) obtained in (EXAMPLE 10) <Step 3> was used as the starting material.

### <Step 5> Synthesis of N-pyridazin-3-yl-4-[8-(trifluoromethyl)-2,3,4,5-tetrahydrobenz-l-oxepin-5-yl]pipera zine-1-carboxamide

The title compound (0.46 g) was obtained as a white solid in a manner similar to (EXAMPLE 2) <Step 7>, in which the compound (0.90 g) obtained in (EXAMPLE 10) <Step 4> was used as the starting material.

### (EXAMPLE 11)

### N-Pyridazin-3-yl-4-(7-(trifluoromethyl)-3,4-dihydro-2H-1-benzopyran-4-yl]piperazi ne-1-carboxamide

### <Step 1> Synthesis of 4-(2-(benzyloxycarbonylamino)ethylamino]-7-(trifluoromethyl)-3,4-dihydro-2H-1-b enzopyrane

The title compound (1.45 g) was obtained as a yellow solid in a manner similar to (EXAMPLE 2) <Step 3>, in which 7-(trifluoromethyl)-chroman-4-one (1.00 g) was used as the starting material.

### <Step 2> Synthesis of 4-[1-(benzyloxycarbonyl)-3-oxopiperazin-4-yl]-7-(trifluoromethyl)-3,4-dihydro-2H-1-benzopyrane

The title compound (0.53 g) was obtained as a colorless amorphous substance in a manner similar to (EXAMPLE 2) <Step 4>, in which the compound (1.00 g) obtained in (EXAMPLE 11) <Step 1> was used.

### <Step 3> Synthesis of 4-(2-oxopiperazin-1-yl)-7-(trifluoromethyl)-3,4-dihydro-2H-1-benzopyrane

The title compound (0.31 g) was obtained as a colorless amorphous substance in a manner similar to (EXAMPLE 4) <Step 3>, in which the compound (0.50 g) obtained in (EXAMPLE 11) <Step 2> was used as the starting material.

### <Step 4> Synthesis of 4-piperazine-1-yl-7-(trifluoromethyl)-3,4-dihydro-2H-1-benzopyrane

The title compound (0.034 g) was obtained as a colorless oily substance in a manner similar to (EXAMPLE 2) <Step 6>, in which the compound (0.10 g) obtained in (EXAMPLE 11) <Step 3> was used as the starting material.

### <Step 5> Synthesis of N-pyridazin-3-yl-4-(7-(trifluoromethyl)-3,4-dihydro-2H-1-benzopyran-4-yl]piperazi ne-1-carboxamide

The title compound (0.05 g) was obtained as a colorless amorphous substance in a manner similar to (EXAMPLE 2) <Step 7>, in which the compound (0.08 g) obtained in (EXAMPLE 11) <Step 4> was used as the starting material.

A part of the resulting title compound was optically resolved into a first fraction (retention time: 11.7 mins.) and a second fraction (retention time: 13.8 mins.), using HPLC (column: CHIRALPAK AD-H (4.6 mm x 150 mm), eluate; ethanol, flow rate: 1 mL/min., UV: 254 nm detection, column temperature: 40°C).

### (EXAMPLE 12)

### N-Pyridazin-3-yl-4-(6-fluoro-7-(trifluoromethyl)-3,4-dihydro-2H-1-benzopyran-4-yl] piperazine-1-carboxamide

### <Step 1> Synthesis of 4-[2-(benzyloxycarbonylamino)ethylamino]-6-fluoro-7-(trifluoromethyl)-3,4-dihydro -2H-1-benzopyrane

The title compound (3.66 g) was obtained as a pale yellow oily substance in a manner similar to (EXAMPLE 2) <Step 3>, in which 6-fluoro-7-(trifluoromethyl)-chroman-4-one (2.40 g) was used as the starting material.

### <Step 2> Synthesis of 4-[1-(benzyloxycarbonyl)-3-oxopiperazin-4-yl]-6-fluoro-7-(trifluoromethyl)-3,4-dihy dro-2H-1-benzopyrane

The title compound (4.65 g) was obtained as a yellow amorphous substance in a manner similar to (EXAMPLE 2) <Step 4>, in which the compound (3.40 g) obtained in (EXAMPLE 12) <Step 1> was used as the starting material.

### <Step 3> Synthesis of 4-(2-oxopiperazin-1-yl)-6-fluoro-7-(trifluoromethyl)-3,4-dihydro-2H-1-benzopyrane

The title compound (1.48 g) was obtained as a black solid in a manner similar to (EXAMPLE 4) <Step 3>, in which the compound (2.10 g) obtained in (EXAMPLE 12) <Step 2> was used as the starting material.

### <Step 4> Synthesis of 6-fluoro-4-piperazine-1-yl-7-(trifluoromethyl)-3,4-dihydro-2H-1-benzopyrane

The title compound (1.41 g) was obtained as a black oily substance in a manner similar to (EXAMPLE 2) <Step 6>, in which the compound (1.48 g) obtained in (EXAMPLE 12) <Step 3> was used as the starting material.

### <Step 5> Synthesis of N-pyridazin-3-yl-4-[6-fluoro-7-(trifluoromethyl)-3,4-dihydro-2H-]-benzopyran-4-yl] piperazine-1-carboxamide

The title compound (1.11 g) was obtained as a yellow solid in a manner similar to (EXAMPLE 2) <Step 7>, in which the compound (1.41 g) obtained in (EXAMPLE 12) <Step 4> was used as the starting material.

### (EXAMPLE 13)

### N-Pyridazin-3-yl-4-(8-fluoro-7-(trifluoromethyl)-3,4-dihydro-2H-1-benzopyran-4-yl] piperazine-1-carboxamide

### <Step 1> Synthesis of 4-[2-(benzyloxycarbonylamino)ethylamino]-8-fluoro-7-(trifluoromethyl)-3,4-dihydro -2H-1-benzopyrane

The title compound (2.34 g) was obtained as a pale yellow oily substance in a manner similar to (EXAMPLE 2) <Step 3>, in which 8-fluoro-7-(trifluoromethyl)-chroman-4-one (3.33 g) was used as the starting material.

### <Step 2> Synthesis of 4-[1-(benzyloxycarbonyl)-3-oxopiperazin-4-yl]-8-fluoro-7-(trifluoromethyl)-3,4-dihy dro-2H-1-benzopyrane

The title compound (1.38 g) was obtained as a colorless amorphous substance in a manner similar to (EXAMPLE 2) <Step 4>, in which the compound (2.34 g) obtained in (EXAMPLE 13) <Step 1> was used as the starting material.

### <Step 3> Synthesis of 4-(2-oxopiperazin-1-yl)-8-fluoro-7-(trifluoromethyl)-3,4-dihydro-2H-1-benzopyrane

The title compound (0.98 g) was obtained as a colorless oily substance in a manner similar to (EXAMPLE 4) <Step 3>, in which the compound (1.38 g) obtained in (EXAMPLE 13) <Step 2> was used as the starting material.

### <Step 4> Synthesis of 8-fluoro-4-piperazine-1-yl-7-(trifluoromethyl)-3,4-dihydro-2H-1-benzopyrane

The title compound (1.06 g) was obtained as a pale green oily substance in a manner similar to (EXAMPLE 2) <Step 6>, in which the compound (0.98 g) obtained in (EXAMPLE 13) <Step 3> was used as the starting material.

### <Step 5> Synthesis of N-pyridazin-3-yl-4-(8-fluoro-7-(trifluoromethyl)-3,4-dihydro-2H-1-benzopyran-4-yl] piperazine-1-carboxamide

The title compound (0.59 g) was obtained as a yellow amorphous substance in a manner similar to (EXAMPLE 2) <Step 7>, in which the compound (1.06 g) obtained in (EXAMPLE 13) <Step 4> was used as the starting material.

A part of the resulting title compound was optically resolved into a first fraction (retention time: 10.9 mins.) and a second fraction (retention time: 12.4 mins.), using HPLC (column: CHIRALPAK AD-H (4.6 mm x 150 mm), eluate; ethanol, flow rate: 1 mL/min., UV: 254 nm detection, column temperature: 40°C).

### (EXAMPLE 14)

### N-Pyridazin-3-yl-4-(3,4-dihydro-2H-1-benzopyran-4-yl)piperazine-1-carboxamide

To an ethanol solution of the compound (0.10 g) obtained in (EXAMPLE 3) were added palladium carbon (20 mg) and triethylamine (0.067 mL). The mixture was stirred for 8 hours in a hydrogen atmosphere, filtered through a celite and then concentrated. A half of the residue was purified by LC-MS to give the title compound (0.011 g) as a colorless oily substance.

### (EXAMPLE 15)

### N-Phenyl-3-yl-4-[7-(trifluoromethyl)-3,4-dihydro-2H-1-benzopyran-4-yl]-3-oxo-pip erazine-1-carboxamide

To a dichloromethane solution of the compound (0.047 g) obtained in (EXAMPLE 11) <Step 3> was added phenyl isocyanate (20 µL), followed by stirring overnight. The solvent was removed by distillation under reduced pressure and the residue was purified by silica gel column chromatography (eluate; n-hexane : ethyl acetate = 100:0 to 50:50) to give the title compound (0.041 g) as a colorless amorphous substance.

### (EXAMPLE 16)

### N-Phenyl-4-[7-(trifluoromethyl)-3,4-dihydro-2H-1-benzopyran-4-yl]piperazine-1-car boxamide

### <Step 1> Synthesis of 4-hydroxy-7-(trifluoromethyl)-3,4-dihydro-2H-1-benzopyrane

To a solution of 7-(trifluoromethyl)chroman-4-one (1.00 g) in methanol (25.0 mL) was added sodium borohydride (1.53 g), followed by stirring for 2 hours. Acetone (25 mL) was added and concentrated. After water was added to the residue, the mixture was extracted with ethyl acetate. The organic layer was washed with brine and then dried over anhydrous sodium sulfate. The solvent was removed by distillation under reduced pressure to give the title compound (1.01 g) as a white solid.

### <Step 2> Synthesis of N-phenyl-4-[7-(trifluoromethyl)-3,4-dihydro-2H-1-benzopyran-4-yl]piperazine-1-car boxamide

To a solution of the compound (0.20 g) obtained in EXAMPLE 16 <Step 1> in dichloromethane (5.0 mL) were added triethylamine (0.19 mL) and chloromethanesulfonic acid (85.9]L). The mixture was stirred for 2 hours. After dichloromethane was added thereto, the organic layer was washed with water and brine and dried over anhydrous sodium sulfate. The solvent was removed by distillation under reduced pressure. To a solution of the resulting residue in N,N-dimethylformamide (2.00 mL) were added N-phenyl-1-piperazinecarboxamide

(0.19 g) and sodium iodide (0.015 g). The mixture was stirred at 70°C for 4 hours. Thereafter, the mixture was stirred at room temperature for a day and further at 70°C for 4 hours. After water was added thereto, the mixture was extracted with ethyl acetate. The organic layer was washed with brine, and dried over anhydrous sodium sulfate. The solvent was removed by distillation under reduced pressure and the resulting residue was purified by silica gel column chromatography (eluate; n-hexane : ethyl acetate = 100:0 to 66:34) to give the title compound (0.009 g) as a pale yellow amorphous substance.

### (EXAMPLE 17)

### N-Pyridine-3-yl4-[7-(trifluoromethyl)-3,4-dihydro-2H-1-benzopyran-4-yl]piperazin e-1-carboxamide

The title compound (0.034 g) was obtained as a colorless amorphous substance in a manner similar to (EXAMPLE 2) <Step 7>, in which the compound (0.033 g) obtained in (EXAMPLE 11) <Step 4> was used as the starting material.

The following compounds were obtained in a manner similar to (EXAMPLE 2) <Step 7>, in which the compound obtained in (EXAMPLE 11) <Step 4> was used as the starting material.

### (EXAMPLE 18)

### N-(6-Methyl-pyridine-3-yl)-4-[7-(trifluoromethyl)-3,4-dihydro-2H-1-benzopyran-4-y l]piperazine-1-carboxamide

### (EXAMPLE 19)

### N-(6-Chloropyridine-3-yl)-4-[7-(trifluoromethyl)-3,4-dihydro-2H-1-benzopyran-4-yl ]piperazine-1-carboxamide

### (EXAMPLE 20)

### N-[5-(Trifluoromethyl)-pyridine-2-yl]-4-[7-(trifluoromethyl)-3,4-dihydro-2H-1-benz opyran-4-yl]piperazine-1-carboxamide

### (EXAMPLE 21)

### N-(6-Methoxypyridazin-2-yl)-4-[7-(trifluoromethyl)-3,4-dihydro-2H-1-benzopyran-4 -yl]piperazine-1-carboxamide

### (EXAMPLE 22)

### N-(1,3-Thiazol-2-yl)-4-[7-(trifluoromethyl)-3,4-dihydro-2H-1-benzopyran-4-yl]piper azine-1-carboxamide

### (EXAMPLE 23)

### N-Pyridazin-3-yl-4-[7-(trifluoromethyl)-2,3-dihydrochromen-4-ylidene]piperizine-1-carboxamide

### <Step 1> Synthesis of benzyl 4-[7-(trifluoromethyl)-2,3-dihydrochromen-4-ylidene]piperizine-1-carboxylate

Under ice cooling, zinc (0.61 g) and then a dichloromethane solution (5.10 mL) of 1.0 M titanium tetrachloride were added to a solution of 7-(trifluoromethyl)chroman-4-one (0.50 g) and N-benzyloxycarbonyl-4-piperidone (1.09 g) in tetrahydrofuran (10.0 mL). The mixture was stirred with heating to reflux for 20 minutes. After reverting to room temperature, 2N hydrochloric acid was added to the mixture, followed by extraction with ethyl acetate. The organic layer was washed with saturated sodium hydrogencarbonate aqueous solution, and then dried over anhydrous sodium sulfate. The solvent was removed by distillation under reduced pressure. The resulting residue was purified by silica gel column chromatography (eluate; n-hexane : ethyl acetate = 100:0 to 67:33) to give the title compound (0.25 g) as a colorless oily substance.

### <Step 2> Synthesis of N-pyridazin-3-yl-4-[7-(trifluoromethyl)-2,3-dihydrochromen-4-ylidene]piperizine-1-carboxamide

To a solution of the compound (40.0 mg) obtained in EXAMPLE 23 <Step 1> and triethylamine (2.0 µL) in dichloromethane (2.0 mL) were added palladium (II) acetate (1.08 mg) and trietylsilane (22.96 µL). The mixture was stirred at room temperature for 16 hours. Water (5 mL) was added to the mixture, followed by extraction with ethyl acetate. The organic layer was washed with brine and then dried over anhydrous sodium sulfate. The solvent was removed by distillation under reduced pressure. The resulting residue was dissolved in acetonitrile (2.00 mL), and diisopropylethylamine (50.87 µL) and phenyl (pyridazin-3-yl) carbamate (38.63 mg) were added to the solution. The mixture was stirred at room temperature for 2 hours. After concentration of the reaction solution, the residue was purified by silica gel column chromatography (eluate; n-hexane : ethyl acetate : methanol = 50:50:0 to 0:100:0 to 0:98:2) to give the title compound (8.1 mg) as a brown solid.

### (EXAMPLE 24)

### N-Pyridazin-3-yl-4-[7-(trifluoromethyl)-2,3-dihydro-1,4-benzoxazin-4-yl]piperidine-1-carboxamide

### <Step 1> Synthesis of tert-butyl 4-[2-hydroxy-4-(trifluoromethyl)anilino]piperidine-1-carboxylate

To a solution of 2-amino-5-(trifluoromethyl)phenol (0.30 g) and N-benzyloxycarbonyl-4-piperidone (0.37 g) in methanol (10.0 mL) was added decaborane (62.10 mg). The mixture was stirred at room temperature for 1.5 hours. After concentration of the reaction solution, water (10 mL) was added to the mixture followed by extraction with ethyl acetate. The organic layer was washed with water and brine, and then dried over anhydrous sodium sulfate. The solvent was removed by distillation under reduced pressure, and the resulting residue was purified by silica gel column chromatography (eluate; n-hexane : ethyl acetate = 85:15-67:33) to give the title compound (0.55 g) as a yellow solid substance.

### <Step 2> Synthesis of tert-butyl 4-[2,3-dihydro-3-oxo-7-(trifluoromethyl)-1,4-benzoxazin-4-yl]piperidine-1-carboxyl ate

To a solution of the compound (0.10 g) obtained in EXAMPLE 24 <Step 1> in N,N-dimethylformamide (5.00 mL) was added sodium hydride (12.21 mg) at 0°C. After stirring for 30 minutes, methyl bromoacetate (26.61 µL) was added thereto, the mixture was stirred at room temperature for 2 hours. Water (5 mL) was added and the mixture was extracted with ethyl acetate. The organic layer was washed with water, and then dried over anhydrous sodium sulfate. The solvent was removed by distillation under reduced pressure. The resulting residue was dissolved in methanol (4.00 mL) and tetrahydrofuran (2.00 mL), and 5% sodium hydroxide (2.00 mL) aqueous solution was added to the solution. The mixture was stirred at room temperature for an hour. Water was added to the mixture, followed by extraction with ethyl acetate. The organic layer was dried over anhydrous sodium sulfate. The solvent was removed by distillation under reduced pressure. The resulting residue was purified by silica gel column chromatography (eluate; n-hexane : ethyl acetate = 85:15-67:33) to give the title compound (59.0 mg) as a white solid.

To the compound (59.00 mg) obtained in EXAMPLE 24) <Step 2> was added 4N hydrogen chloride-ethyl acetate solution. The mixture was stirred at room temperature for 16 hours. After concentration of the reaction solution, the concentrate was washed with hexane to give the title compound (52.3 mg) as a white solid.

### <Step 4> Synthesis of 4-piperidin-4-yl-7-(trifluoromethyl)-2H-2,3-dihydro-1,4-benzoxazin-3(4H)-one hydrochloride

The title compound (28.4 mg) was obtained as a yellow oily substance in a manner similar to (EXAMPLE 2) <Step 6>, in which the compound (30.00 mg) obtained in (EXAMPLE 24) <Step 3> was used as the starting material.

### <Step 5> Synthesis of N-pyridazin-3-yl-4-[7-(trifluoromethyl)-2,3-dihydro-1,4-benzoxazin-4-yl]piperidine-1-carboxamide

The title compound (6.0 mg) was obtained as a white solid in a manner similar to (EXAMPLE 2) <Step 7>, in which the compound (25.00 mg) obtained in (EXAMPLE 24) <Step 4> was used as the starting material.

### (EXAMPLE 25)

### N-Pyridazin-3-yl-4-[7-(trifluoromethyl)-3,4-dihydro-2H-chromen-4-yl]piperidine-1-carboxamide

### <Step 1> Synthesis of 4-[7-(trifluoromethyl)chroman-4-yl]piperidine

The title compound (69 mg) was obtained as an oily substance in a manner similar to (EXAMPLE 4) <Step 3>, in which the compound (0.10 g) obtained in (EXAMPLE 23) <Step 1> was used as the starting material.

### <Step 2> Synthesis of N-pyridazin-3-yl-4-[7-(trifluoromethyl)-3,4-dihydro-2H-chromen-4-yl]piperidine-1-c arboxamide

The title compound (79 mg) was obtained as a yellow amorphous substance in a manner similar to (EXAMPLE 2) <Step 7>, in which the compound (60.00 mg) obtained in (EXAMPLE 25) <Step 1> was used as the starting material.

A part of the resulting title compound was optically resolved into a first fraction (retention time: 9.3 mins.) and a second fraction (retention time: 10.5 mins.), using HPLC (column: CHIRALPAK AD-H (4.6 mm x 150 mm), eluate; hexane : ethanol = 50:50 (v/v), flow rate: 1.6 mL/min., UV: 254 nm detection, column temperature: 40°C).

### (EXAMPLE 26)

### 4-Hydroxy-N-pyridazin-3-yl-4-[7-(trifluoromethyl)-3,4-dihydro-2H-chromen-4-yl]pi peridine-1-carboxamide

### <Step 1> Synthesis of [1-(benzyloxycarbonyl)piperidine]-4-spiro-2'-oxacyclopropane-3'-spiro-4"-[7"-(triflu oromethyl)chromane]

To a solution of the compound (0.50 g) obtained in (EXAMPLE 23) <Step 1> in dichloromethane (5.00 mL) was added m-chloroperbenzoic acid (0.32 g). The mixture was stirred at room temperature for 40 minutes. Saturated sodium bicarbonate aqueous solution (35 mL) was added to the reaction solution, followed by extraction with dichloromethane (35 mL). The organic layer was dried over anhydrous sodium sulfate. The solvent was removed by distillation under reduced pressure, and the resulting residue was purified by silica gel column chromatography (eluate; n-hexane : ethyl acetate = 85:15 to 70:30) to give the title compound (0.41 g) as a white solid.

### <Step 2> Synthesis of 4-[7-(trifluoromethyl)-3,4-dihydro-2H-chromen-4-yl]piperidin-4-ol

The title compound (55 mg) was obtained as a light brown amorphous substance in a manner similar to (EXAMPLE 4) <Step 3>, in which the compound (80.00 mg) obtained in (EXAMPLE 26) <Step 1> was used as the starting material.

### <Step 3> Synthesis of 4-hydroxy-N-pyridazin-3-yl-4-[7-(trifluoromethyl)-3,4-dihydro-2H-chromen-4-yl]pi peridine-1-carboxamide

The title compound (11 mg) was obtained as a colorless amorphous substance in a manner similar to (EXAMPLE 2) <Step 7>, in which the compound (13.00 mg) obtained in (EXAMPLE 26) <Step 2> was used as the starting material.

### (EXAMPLE 27)

### 4-[1-Acetyl-7-(trifluoromethyl)-3,4-dihydro-2H-quinolin-4-yl]-N-pyridazin-3-ylpipe razine-1-carboxamide

### <Step 1> Synthesis of 3-[3-(trifluoromethyl)anilino]propanoic acid

Under heating to reflux, 3-(trifluoromethyl)aniline (10.00 g), acrylic acid (6.39 mL) and water (20.00 mL) were stirred for an hour. After cooling, 1N sodium hydroxide aqueous solution was added to the reaction solution and pH was adjusted to approximately 10. The mixture was then washed with ethyl acetate. After pH was adjusted to approximately 3 by adding 10% hydrochloric acid to the aqueous layer, the mixture was extracted with ethyl acetate. The organic layer was washed with brine, and then dried over anhydrous sodium sulfate. The solvent was removed by distillation under reduced pressure. The resulting residue was purified by silica gel column chromatography (eluate; n-hexane : ethyl acetate = 1:2) to give the title compound (11.78 g) as a colorless oily substance.

### <Step 2> Synthesis of 7-(trifluoromethyl)-2,3-dihydro-1H-quinolin-4-one

The compound (11.50 g) obtained in (EXAMPLE 27) <Step 1> and polyphosphoric acid (166.69 mL) were stirred at 120°C for 3 hours. After the reaction solution was poured onto ice water, the mixture was extracted with ethyl acetate. The organic layer was washed with brine, and then dried over anhydrous sodium sulfate. The solvent was removed by distillation under reduced pressure, and the resulting residue was purified by silica gel column chromatography (eluate; n-hexane : ethyl acetate = 75:25-65:35) to give the title compound (1.0 g) as a yellow solid.

### <Step 3> Synthesis of 1-acetyl-7-(trifluoromethyl)-2,3-dihydroquinolin-4-one

To a dichloromethane solution (20.00 mL) of the compound (1.00 g) obtained in (EXAMPLE 27) <Step 2> were added pyridine (0.57 mL) and acetyl chloride (0.43 mL). The mixture was stirred for an hour. Water was added to the mixture, followed by extraction with methylene chloride. The organic layer was washed with 1N hydrochloric acid, 1N sodium hydroxide aqueous solution and brine, and then dried over anhydrous sodium sulfate. The solvent was removed by distillation under reduced pressure to give the title compound (1.2 g) as a yellow powder.

### <Step 4> Synthesis of 1-[4-hydroxy-7-(trifluoromethyl)-3,4-dihydro-2H-quinolin-1-yl]ethanone

The title compound (0.50 g) was obtained as a yellow oily substance in a manner similar to (EXAMPLE 16) <Step 1>, in which the compound (0.50 g) obtained in (EXAMPLE 27) <Step 3> was used as the starting material.

### <Step 5> Synthesis of 1-[4-chloro-7-(trifluoromethyl)-3,4-dihydro-2H-quinolin-1-yl]ethanone

Under ice cooling, methanesulfonyl chloride (0.22 mL) was added to a dichloromethane solution (10.00 mL) of the compound (0.50 g) obtained in (EXAMPLE 27) <Step 4> and triethylamine (0.54 mL), and stirred for 20 minutes. After stirring at room temperature for further 40 minutes, the mixture was heated to reflux for 3 hours while stirring. Water was added to separate the organic phase. The solvent was removed by distillation under reduced pressure, and the resulting residue was purified by silica gel column chromatography to give the title compound (407.9 mg) as a colorless oily substance.

### <Step 6> Synthesis of tert-butyl 4-[1-acetyl-7-(trifluoromethyl)-3,4-dihydro-2H-quinolin-4-yl]piperazine-1-carboxyla te

Under heating to reflux, an acetonitrile solution (10.00 mL) of the compound (0.40 g) obtained in (EXAMPLE 27) <Step 5>, sodium iodide (43.19 mg), sodium carbonate (0.31 g) and 1,1-dimethylethylpiperazine-1-carboxylate (0.27 g) was stirred for 6 hours. The mixture was filtered through a celite and the filtrate was concentrated. The resulting residue was purified by silica gel column chromatography to give the title compound (345 mg) as a white solid.

### <Step 7> Synthesis of 1-[4-piperazin-1-yl-7-(trifluoromethyl)-3,4-dihydro-2H-quinolin-1-yl]ethanone hydrochloride

The title compound (150 mg) was obtained as a white solid in a manner similar to (EXAMPLE 24) <Step 3>, in which the compound (0.19 g) obtained in (EXAMPLE 27) <Step 6> was used as the starting material.

### <Step 8> Synthesis of 4-[1-acetyl-7-(trifluoromethyl)-3,4-dihydro-2H-quinolin-4-yl]-N-pyridazin-3-ylpiper azine-1-carboxamide

The title compound (151.5 mg) was obtained as a white solid in a manner similar to (EXAMPLE 2) <Step 7>, in which the compound (0.14 g) obtained in (EXAMPLE 27) <Step 7> was used as the starting material.

A part of the resulting title compound was optically resolved into a first fraction (retention time: 9.5 mins.) and a second fraction (retention time: 12.8 mins.), using HPLC (column: CHIRALPAK AD-H (4.6 mm x 150 mm), eluate; hexane : ethanol = 50:50 (v/v), flow rate: 1.6 mL/min., UV: 254 nm detection, column temperature: 40°C).

### (EXAMPLE 28)

### N-(Pyridazin-3-yl)-4-[7-(trifluoromethyl)-1,2,3,4-tetrahydroquinolin-4-yl]piperazine-1 (4H)-carboxamide

### <Step 1> Synthesis of benzyl 4-oxo-7-(trifluoromethyl)-2,3-dihydroquinoline-1-carboxylate

Under ice cooling, potassium carbonate (2.25 g) and water (7.50 mL) were added to a tetrahydrofuran solution (10.00 mL) of the compound (3.50 g) obtained in (EXAMPLE 27) <Step 2> and benzyl chloroformate (2.75 mL). The mixture was stirred at room temperature overnight. After benzyl chloroformate (1.38 mL) was additionally added thereto, the mixture was further stirred overnight. Water was added to the reaction solution, followed by extraction with ethyl acetate. The solvent was removed by distillation under reduced pressure, and the resulting residue was purified by silica gel column chromatography (eluate; n-hexane : ethyl acetate = 80:20) to give the title compound (5.68 g) as a yellow oily substance.

### <Step 2> Synthesis of benzyl 4-hydroxy-7-(trifluoromethyl)-3,4-dihydro-2H-quinoline-1-carboxylate

The title compound (5.5 g) was obtained as a yellow solid in a manner similar to (EXAMPLE 16) <Step 1>, in which the compound (5.68 g) obtained in (EXAMPLE 28) <Step 1> was used as the starting material.

### <Step 3> Synthesis of benzyl 4-chloro-7-(trifluoromethyl)-3,4-dihydro-2H-quinoline-1-carboxylate

The title compound (5.39 g) was obtained as a yellow oily substance in a manner similar to (EXAMPLE 27) <Step 5>, in which the compound (5.50 g) obtained in (EXAMPLE 28) <Step 2> was used as the starting material.

### <Step 4> Synthesis of benzyl 4-[4-[(2-methylpropan-2-yl)oxycarbonyl]piperazin-1-yl]-7-(trifluoromethyl)-3,4-dih ydro-2H-quinoline-1-carboxylate

The title compound (6.85 g) was obtained as a pale yellow amorphous substance in a manner similar to (EXAMPLE 27) <Step 6>, in which the compound (5.36 g) obtained in (EXAMPLE 28) <Step 3> was used as the starting material.

### <Step 5> Synthesis of benzyl 4-piperazin-1-yl-7-(trifluoromethyl)-3,4-dihydro-2H-quinoline-1-carboxylate dihydrochloride

The title compound (0.70 g) was obtained as a brown solid in a manner similar to (EXAMPLE 24) <Step 3>, in which the compound (0.85 g) obtained in (EXAMPLE 28) <Step 4> was used as the starting material.

### <Step 6> Synthesis of benzyl 4-[4-(pyridazin-3-ylcarbamoyl)piperazine-1-yl]-7-(trifluoromethyl)-3,4-dihydro-2H-quinoline-1-carboxylate

The title compound (337 mg) was obtained as a yellow amorphous substance in a manner similar to (EXAMPLE 2) <Step 7>, in which the compound (0.40 g) obtained in (EXAMPLE 28) <Step 5> was used as the starting material.

### <Step 7> Synthesis of N-(pyridazin-3-yl)-4-[7-(trifluoromethyl)-1,2,3,4-tetrahydroquinolin-4-yl]piperazine-1-carboxamide

The title compound (166 mg) was obtained as a white solid in a manner similar to (EXAMPLE 4) <Step 3>, in which the compound (0.30 g) obtained in (EXAMPLE 28) <Step 6> was used as the starting material.

### (EXAMPLE 29)

### 4-[1-Methylsulfonyl-7-(trifluoromethyl)-3,4-dihydro-2H-quinolin-4-yl]-N-pyridazin-3-ylpiperazine-1-carboxamide

Under ice cooling, methanesulfonyl chloride (17.14 µL) was added to a dichloromethane solution (10.00 mL) of the compound (60.00 mg) obtained in (EXAMPLE 28) <Step 7> and triethylamine (41.15µL). The mixture was stirred at room temperature overnight. Water was added thereto, followed by extraction with ethyl acetate. The solvent was removed by distillation under reduced pressure, and the resulting residue was purified by silica gel column chromatography (eluate; n-hexane : ethyl acetate = 50:50 to 20:80) to give the title compound (21 mg) as a white solid.

### (EXAMPLE 30)

### (3S)-3-Methyl-N-pyridazin-3-yl-4-[7-(trifluoromethyl)-3,4-dihydro-2H-chromen-4-y l]piperazine-1-carboxamide

### <Step 1> Synthesis of 4-chloro-7-(trifluoromethyl)-chromane

Under ice cooling, methanesulfonyl chloride (0.80 mL) was added to a dichloromethane solution (15.00 mL) of the compound (1.50 g) obtained in (EXAMPLE 16) <Step 1> and triethylamine (1.92 mL). The mixture was stirred at room temperature for an hour. The mixture was stirred at 60°C for further 3 hours. Water (20 mL) was added thereto, followed by extraction with ethyl acetate. The organic layer was washed with brine, and then dried over anhydrous sodium sulfate. The solvent was removed by distillation under reduced pressure, and the resulting residue was purified by silica gel column chromatography (eluate; n-hexane : ethyl acetate = 90:10 to 85:15) to give the title compound (1.42 g) as a colorless oily substance.

### <Step 2> Synthesis of tert-butyl (3S)-3-methyl-4-[7-(trifluoromethyl)-3,4-dihydro-2H-chromen-4-yl]piperazine-1-car boxylate

The title compound (0.67 g) was obtained as a yellow oily substance in a manner similar to (EXAMPLE 27) <Step 6>, in which the compound (2.50 g) obtained in (EXAMPLE 30) <Step 1> was used as the starting material.

### <Step 3> Synthesis of (2S)-2-methyl-1-[7-(trifluoromethyl)-3,4-dihydro-2H-chromen-4-yl]piperazine

The title compound (0.22 g) was obtained as a yellow oily substance in a manner similar to (EXAMPLE 2) <Step 5>, in which the compound (0.30 g) obtained in (EXAMPLE 30) <Step 2> was used as the starting material.

### <Step 4> Synthesis of (3S)-3-methyl-N-pyridazin-3-yl-4-[7-(trifluoromethyl)-3,4-dihydro-2H-chromen-4-yl ]piperazine-1-carboxamide

The title compound (0.24 g) was obtained as a yellow amorphous substance in a manner similar to (EXAMPLE 2) <Step 7>, in which the compound (0.22 g) obtained in (EXAMPLE 30) <Step 3> was used as the starting material.

### (EXAMPLE 31)

### (3R)-3-Methyl-N-pyridazin-3-yl-4-[7-(trifluoromethyl)-3,4-dihydro-2H-chromen-4-y l]piperazine-1-carboxamide

### <Step 1> Synthesis of tert-butyl (3R)-3-methyl-4-[7-(trifluoromethyl)-3,4-dihydro-2H-chromen-4-yl]piperazine-1-car boxylate

The title compound (0.92 g) was obtained as a yellow oily substance in a manner similar to (EXAMPLE 27) <Step 6>, in which the compound (2.50 g) obtained in (EXAMPLE 30) <Step 1> was used as the starting material.

### <Step 2> Synthesis of (2R)-2-methyl-1-[7-(trifluoromethyl)-3,4-dihydro-2H-chromen-4-yl]piperazine

The title compound (0.22 g) was obtained as a yellow oily substance in a manner similar to (EXAMPLE 2) <Step 5>, in which the compound (0.30 g) obtained in (EXAMPLE 31) <Step 1> was used as the starting material.

### <Step 3> Synthesis of (3R)-3-methyl-N-pyridazin-3-yl-4-[7-(trifluoromethyl)-3,4-dihydro-2H-chromen-4-y l]piperazine-1-carboxamide

The title compound (0.23 g) was obtained as a yellow amorphous substance in a manner similar to (EXAMPLE 2) <Step 7>, in which the compound (0.20 g) obtained in (EXAMPLE 30) <Step 2> was used as the starting material.

### (EXAMPLE 32)

### 4-(7-Cyano-3,4-dihydro-2H-chromen-4-yl)-N-pyridazin-3-ylpiperazine-1-carboxami de

### <Step 1> Synthesis of tert-butyl 4-(7-bromo-3,4-dihydro-2H-chromen-4-yl)piperazine-1-carboxylate

Under ice cooling, 1M borane tetrahydrofuran/tetrahydrofuran solution (71.3 mL) was added to a tetrahydrofuran solution (120 mL) of the compound (7.40 g) obtained in (EXAMPLE 2) <Step 5>. The mixture was stirred at room temperature overnight. After methanol (25 mL) and 10% hydrogen chloride-methanol solution (40 mL) were added, the mixture was stirred for an hour while heating to reflux. The white solid precipitated was taken out by filtration and dried under reduced pressure to give a white solid (4.88 g). Di-t-butyl dicarbonate (1.83 g) was added to a dichloromethane solution (65 mL) of this white solid (2.40 g) and triethylamine (2.70 mL), which was stirred at room temperature for 3 hours. After 8N ammonia-methanol solution (0.24 mL) was added, the mixture was diluted in ethyl acetate. The reaction solution was washed with saturated sodium hydrogencarbonate aqueous solution, and then dried over anhydrous sodium sulfate. The solvent was removed by distillation under reduced pressure, and the resulting residue was purified by amine silica gel column chromatography (eluate; n-hexane : ethyl acetate = 95:5 to 92:8) to give the title compound (0.87 g) as a white solid.

### <Step 2> Synthesis of t-butyl 4-(7-cyano-3,4-dihydro-2H-chromen-4-yl)piperazine-1-carboxylate

To an N,N-dimethylformamide solution (5.00 mL) of the compound (100 mg) obtained in (EXAMPLE 32) <Step 1> were added zinc cyanide (29.40 mg) and tetrakis(triphenylphosphine)palladium (28.90 mg). The mixture was stirred at 100°C for 4 hours. Water was added thereto, followed by extraction with ethyl acetate. The organic layer was washed with brine, and then dried over anhydrous sodium sulfate. The solvent was removed by distillation under reduced pressure, and the resulting residue was purified by amine silica gel thin layer column chromatography (eluate; n-hexane : ethyl acetate = 80:20) to give the title compound (61.7 mg) as a pale yellow solid.

### <Step 3> Synthesis of 4-piperazin-1-yl-3,4-dihydro-2H-chromene-7-carbonitrile dihydrochloride

The title compound (23.2 mg) was obtained as a white solid in a manner similar to (EXAMPLE 24) <Step 3>, in which the compound (34.00 mg) obtained in (EXAMPLE 32) <Step 2> was used as the starting material.

### <Step 4> Synthesis of 4-(7-cyano-3,4-dihydro-2H-chromen-4-yl)-N-pyridazin-3-ylpiperazine-1-carboxamid e

The title compound (9.4 mg) was obtained as a colorless amorphous substance in a manner similar to (EXAMPLE 2) <Step 7>, in which the compound (16.60 mg) obtained in (EXAMPLE 32) <Step 3> was used as the starting material.

### (EXAMPLE 33)

### 4-(7,8-Difluoro-3,4-dihydro-2H-chromen-4-yl)-N-pyridazin-3-ylpiperazine-1-carbox amide

### <Step 1> Synthesis of 3-(2,3-difluorophenoxy)propionic acid

Under ice cooling, 60% sodium hydride (3.40 g) was added to an N,N-dimethylformamide solution (77.00 mL) of 2,3-difluorophenol (10.00 g). The mixture was stirred at room temperature for 1.5 hours. Under ice cooling, β-propiolactone (8.60 g) was added and stirred at room temperature for 2 hours. Under ice cooling, water was added and then conc. hydrochloric acid was added to adjust pH to 4 or less. The mixture was extracted with ethyl acetate. The organic layer was washed with brine, and then dried over anhydrous sodium sulfate. The solvent was removed by distillation under reduced pressure, and the resulting residue was triturated with hexane to give the title compound (9.9 g) as a white solid.

### <Step 2> Synthesis of 7,8-difluorochroman-4-one

Diphosphorus pentoxide (13.90 g) was added portion by portion to methanesulfonic acid (63.60 mL) heated to 80°C. After the compound (9.90 g) obtained in (EXAMPLE 33) <Step 1> was added thereto, the mixture was stirred for 5 minutes. The reaction solution was poured onto ice and extracted with ethyl acetate. The organic layer was washed with water and then brine, and dried over anhydrous sodium sulfate. The solvent was removed by distillation under reduced pressure, and the resulting residue was purified by silica gel column chromatography (eluate; n-hexane : ethyl acetate = 100:0 to 95:5 to 80:20) to give the title compound (7 g) as a beige solid.

### <Step 3> Synthesis of 7,8-difluoro-3,4-dihydro-2H-chromen-4-ol

The title compound (6.29 g) was obtained as a pale yellow solid in a manner similar to (EXAMPLE 16) <Step 1>, in which the compound (7.00 g) obtained in (EXAMPLE 33) <Step 2> was used as the starting material.

### <Step 4> Synthesis of t-butyl 4-(7,8-difluoro-3,4-dihydro-2H-chromen-4-yl)piperazine-1-carboxylate

A colorless oily substance (1.6 g) was obtained in a manner similar to (EXAMPLE 30) <Step 1>, in which the compound (6.28 g) obtained in (EXAMPLE 33) <Step 3> was used as the starting material.

The title compound (1.69 g) was obtained as a colorless oily substance in a manner similar to (EXAMPLE 27) <Step 6>, in which this colorless oily substance (1.60 g) was used as the starting material.

### <Step 5> Synthesis of 1-(7,8-difluoro-3,4-dihydro-2H-chromen-4-yl)piperazine dihydrochloride

The title compound (0.56 g) was obtained as a white solid in a manner similar to (EXAMPLE 24) <Step 3>, in which the compound (0.65 g) obtained in (EXAMPLE 33) <Step 4> was used as the starting material.

### <Step 6> Synthesis of 4-(7,8-difluoro-3,4-dihydro-2H-chromen-4-yl)-N-pyridazin-3-ylpiperazine-1-carbox amide

The title compound (224 mg) was obtained as a beige amorphous substance in a manner similar to (EXAMPLE 2) <Step 7>, in which the compound (0.26 g) obtained in (EXAMPLE 33) <Step 5> was used as the starting material.

### (EXAMPLE 34)

### N-(Pyridazin-3-yl)-4-[8-(trifluoromethyl)-3,4-dihydro-2H-chromen-4-yl]piperazine-1-carboxamide

### <Step 1> Synthesis of 3-[(2-trifluoromethyl)phenoxy]propionic acid

The title compound (13.9 g) was obtained as a white solid in a manner similar to (EXAMPLE 33) <Step 1>, in which 2-(trifluoromethyl)phenol (10.00 g) was used as the starting material.

### <Step 2> Synthesis of 8-(trifluoromethyl)chroman-4-one

The title compound (604 mg) was obtained as a colorless oily substance in a manner similar to (EXAMPLE 33) <Step 2>, in which the compound (1.00 g) obtained in (EXAMPLE 34) <Step 1> was used as the starting material.

### <Step 3> Synthesis of 8-(trifluoromethyl)-3,4-dihydro-2H-chromen-4-ol

The title compound (512 mg) was obtained as a pale yellow oily substance in a manner similar to (EXAMPLE 16) <Step 1>, in which the compound (600 mg) obtained in (EXAMPLE 34) <Step 2> was used as the starting material.

### <Step 4> Synthesis of tert-butyl 4-[8-(trifluoromethyl)-3,4-dihydro-2H-chromen-4-yl]piperazine-1-carboxylate

A white solid (412 mg) was obtained in a manner similar to (EXAMPLE 30) <Step 1>, in which the compound (500 mg) obtained in (EXAMPLE 34) <Step 3> was used as the starting material. The title compound (509 mg) was obtained as a colorless oily substance in a manner similar to (EXAMPLE 27) <Step 6>, in which this white solid (400 mg) was used as the starting material.

### <Step 5> Synthesis of 1-[8-(trifluoromethyl)-3,4-dihydro-2H-chromen-4-yl]piperazine

The title compound (352 mg) was obtained as a white solid in a manner similar to (EXAMPLE 2) <Step 5>, in which the compound (500 mg) obtained in (EXAMPLE 34) <Step 4> was used as the starting material.

### <Step 6> Synthesis of N-pyridazin-3-yl-4-[8-(trifluoromethyl)-3,4-dihydro-2H-chromen-4-yl]piperazine-1-carboxamide

The title compound (250 mg) was obtained as a pale yellow solid in a manner similar to (EXAMPLE 2) <Step 7>, in which the compound (200 mg) obtained in (EXAMPLE 34) <Step 5> was used as the starting material.

### (EXAMPLE 35)

### 4-(7,8-Difluoro-3,4-dihydro-2H-chromen-4-yl)-N-(pyridine-3-yl)piperazine-1-carbo xamide

The title compound (198.9 mg) was obtained as a colorless amorphous substance in a manner similar to (EXAMPLE 2) <Step 7>, in which the compound (0.25 g) obtained in (EXAMPLE 33) <Step 6> was used as the starting material.

A part of the resulting title compound was optically resolved into a first fraction (retention time: 8.5 mins.) and a second fraction (retention time: 10.8 mins.), using HPLC (column: CHIRALPAK AS-H (4.6 mm x 150 mm), eluate; hexane : ethanol = 90:10 (v/v), flow rate: 3 mL/min., UV: 254 nm detection, column temperature: 40°C).

### (EXAMPLE 36)

### 4-(8-Chloro-3,4-dihydro-2H-chromen-4-yl)-N-(pyridazin-3-yl)-piperazine-1-carboxa mide

### <Step 1> Synthesis of 3-(3-chlorophenoxy)propionic acid

The title compound (14.8 g) was obtained as a white solid in a manner similar to (EXAMPLE 33) <Step 1>, in which 3-chlorophenol (10.00 g) was used as the starting material.

### <Step 2> Synthesis of 7-chlorochroman-4-one (a) and 5-chlorochroman-4-one (b)

A mixture (6.64 g) of the title compound was obtained as a purple solid in a manner similar to (EXAMPLE 33) <Step 2>, in which the compound (10.50 g) obtained in (EXAMPLE 36) <Step 1> was used as the starting material.

### <Step 3> Synthesis of 7-chloro-3,4-dihydro-2H-chromen-4-ol

The title compound (4.0 g) was obtained as a white solid in a manner similar to (EXAMPLE 16) <Step 1>, in which the compound (6.63 g) obtained in (EXAMPLE 36) <Step 2> was used as the starting material.

### <Step 4> Synthesis of tert-butyl 4-(7-chloro-3,4-dihydro-2H-chromen-4-yl)piperazine-1-carboxylate

A purple oily substance (0.478 g) was obtained in a manner similar to (EXAMPLE 30) <Step 1>, in which the compound (4.01 g) obtained in (EXAMPLE 36) <Step 3> was used as the starting material.

The title compound (0.38 g) was obtained as a yellow solid in a manner similar to (EXAMPLE 27) <Step 6>, in which this purple oily substance (0.47 g) was used as the starting material.

### <Step 5> Synthesis of 1-(7-chloro-3,4-dihydro-2H-chromen-4-yl)piperazine dihydrochloride

The title compound (0.31 g) was obtained as a beige solid in a manner similar to (EXAMPLE 24) <Step 3>, in which the compound (0.36 g) obtained in (EXAMPLE 36) <Step 4> was used as the starting material.

### <Step 6> Synthesis of 4-(7-chloro-3,4-dihydro-2H-chromen-4-yl)-N-(pyridazin-3-yl)-piperazine-1-carboxa mide

The title compound (210 mg) as a beige solid in a manner similar to (EXAMPLE 2) <Step 7>, in which the compound (0.29 g) obtained in (EXAMPLE 36) <Step 5> was used as the starting material.

### (EXAMPLE 37)

### 4-[8-Chloro-7-(trifluoromethyl)-3,4-dihydro-2H-chromen-4-yl]-N-(pyridazin-3-yl)pi perazine-1-carboxamide

### <Step 1> Synthesis of 3-[[2-chloro-3-(trifluoromethyl)phenoxy]]propionic acid

The title compound (10.2 g) was obtained as a white solid in a manner similar to (EXAMPLE 33) <Step 1>, in which 2-chloro-3-(trifluoromethyl)phenol (6.76 mL) was used as the starting material.

### <Step 2> Synthesis of 8-chloro-7-(trifluoromethyl)chroman-4-one

The title compound (4.71 g) was obtained as a white solid in a manner similar to (EXAMPLE 33) <Step 2>, in which the compound (10.20 g) obtained in (EXAMPLE 37) <Step 1> was used as the starting material.

### <Step 3> Synthesis of 8-chloro-7-(trifluoromethyl)-3,4-dihydro-2H-chromen-4-ol

The title compound (4.41 g) was obtained as a white solid in a manner similar to (EXAMPLE 16) <Step 1>, in which the compound (4.70 g) obtained in (EXAMPLE 37) <Step 2> was used as the starting material.

### <Step 4> Synthesis of tert-butyl 4-[8-chloro-7-(trifluoromethyl)-3,4-dihydro-2H-chromen-4-yl]piperazine-1-carboxyl ate

A colorless oily substance (3.81 g) was obtained in a manner similar to (EXAMPLE 30) <Step 1>, in which the compound (4.39 g) obtained in (EXAMPLE 37) <Step 3> was used as the starting material. The title compound (5.0 g) was obtained as a white solid in a manner similar to (EXAMPLE 27) <Step 6>, in which this colorless oily substance (3.79 g) was used as the starting material.

### <Step 5> Synthesis of 1-[8-chloro-7-(trifluoromethyl)-3,4-dihydro-2H-chromen-4-yl]piperazine dihydrochloride

The title compound (0.79 g) was obtained as a white solid in a manner similar to (EXAMPLE 24) <Step 3>, in which the compound (0.80 g) obtained in (EXAMPLE 37) <Step 4> was used as the starting material.

### <Step 6> Synthesis of 4-[8-chloro-7-(trifluoromethyl)-3,4-dihydro-2H-chromen-4-yl]-N-(pyridazin-3-yl)pip erazine-1-carboxamide

The title compound (816 mg) was obtained as a yellow solid in a manner similar to (EXAMPLE 2) <Step 7>, in which the compound (0.77 g) obtained in (EXAMPLE 37) <Step 5> was used as the starting material.

A part of the resulting title compound was optically resolved into a first fraction (retention time: 9.3 mins.) and a second fraction (retention time: 12.8 mins.), using HPLC (column: CHIRALPAK AY-H (4.6 mm x 150 mm), eluate; ethanol, flow rate: 1.0 mL/min., UV: 254 nm detection, column temperature: 40°C).

### (EXAMPLE 38)

### N-(Pyridazin-3-yl)-4-[8-[5-(trifluoromethyl)pyridine-2-yl]oxy-3,4-dihydro-2H-chro men-4-yl]piperazine-1-carboxamide

### <Step 1> Synthesis of 2-(2-phenylmethoxyphenoxy)-5-(trifluoromethyl)pyridine

The title compound (7 g) was obtained as a light brown solid in a manner similar to (EXAMPLE 5) <Step 1>, in which 2-benzyloxyphenol (5.00 g) was used as the starting material.

### <Step 2> Synthesis of 2-[5-(trifluoromethyl)pyridine-2-yl]oxyphenol

The title compound (5.2 g) was obtained as a colorless oily substance in a manner similar to (EXAMPLE 4) <Step 3>, in which the compound (7.00 g) obtained in (EXAMPLE 38) <Step 1> was used as the starting material.

### <Step 3> Synthesis of 3-[2-[5-(trifluoromethyl)pyridine-2-yl]oxyphenoxy]propanoic acid

The title compound (5.4 g) was obtained as a light brown solid in a manner similar to (EXAMPLE 33) <Step 1>, in which the compound (4.60 g) obtained in (EXAMPLE 38) <Step 2> was used as the starting material.

### <Step 4> Synthesis of 8-[5-(trifluoromethyl)pyridine-2-yl]oxy-2,3-dihydrochromen-4-one

The title compound (2.6 g) was obtained as a yellow oily substance in a manner similar to (EXAMPLE 33) <Step 2>, in which the compound (5.20 g) obtained in (EXAMPLE 38) <Step 3> was used as the starting material.

### <Step 5> Synthesis of benzyl N-[2-[[8-[5-(trifluoromethyl)pyridine-2-yl]oxy-3,4-dihydro-2H-chromen-4-yl]amino ]ethyl] carbamate

The title compound (2.3 g) was obtained as a colorless oily substance in a manner similar to (EXAMPLE 2) <Step 3>, in which the compound (2.20 g) obtained in (EXAMPLE 38) <Step 4> was used as the starting material.

### <Step 6> Synthesis of benzyl 3-oxo-4-[8-(5-trifluoromethyl)pyridine-2-yl)oxy-3,4-dihydro-2H-chromen-4-yl]piper azine-1-carboxylate

The title compound (1.3 g) was obtained as a pale yellow oily substance in a manner similar to (EXAMPLE 2) <Step 4>, in which the compound (2.20 g) obtained in (EXAMPLE 38) <Step 5> was used as the starting material.

### <Step 7> Synthesis of 1-[8-[5-(trifluoromethyl)pyridine-2-yl]oxy-3,4-dihydro-2H-chromen-4-yl]piperazin-2-one

The title compound (0.8 g) was obtained as a colorless amorphous substance in a manner similar to (EXAMPLE 4) <Step 3>, in which the compound (1.30 g) obtained in (EXAMPLE 38) <Step 6> was used as the starting material.

### <Step 8> Synthesis of 1-[8-[5-(trifluoromethyl)pyridine-2-yl]oxy-3,4-dihydro-2H-chromen-4-yl]piperazine

The title compound (0.67 g) was obtained as a brown oily substance in a manner similar to (EXAMPLE 2) <Step 6>, in which the compound (0.70 g) obtained in (EXAMPLE 38) <Step 7> was used as the starting material.

### <Step 9> Synthesis of N-(pyridazin-3-yl)-4-[8-[5-(trifluoromethyl)pyridine-2-yl]oxy-3,4-dihydro-2H-chro men-4-yl]piperazine-1-carboxamide

The title compound (0.49 g) was obtained as a yellow amorphous substance in a manner similar to (EXAMPLE 2) <Step 7>, in which the compound (0.56 g) obtained in (EXAMPLE 38) <Step 8> was used as the starting material.

The resulting title compound was optically resolved into a first fraction (retention time: 8.6 mins.) and a second fraction (retention time: 12.7 mins.), using HPLC (column: CHIRALPAK OD-H (4.6 mm x 150 mm), eluate; hexane : ethanol = 50:50 (v/v), flow rate: 1.6 mL/min., UV: 254 nm detection, column temperature: 40°C).

### (EXAMPLE 39)

### 4-(7,8-Dichloro-3,4-dihydro-2H-chromen-4-yl)-N-(pyridazin-3-yl)piperazine-1-carb oxamide

### <Step 1> Synthesis of 3-(2,3-dichlorophenoxy)propionic acid

The title compound (17.2 g) was obtained as a white solid in a manner similar to (EXAMPLE 33) <Step 1>, in which 2,3-dichlorophenol (10.00 g) was used as the starting material.

### <Step 2> Synthesis of 7,8-dichlorochroman-4-one

The title compound (7.17 g) was obtained as an orange solid in a manner similar to (EXAMPLE 33) <Step 2>, in which the compound (16.90 g) obtained in (EXAMPLE 39) <Step 1> was used as the starting material.

### <Step 3> Synthesis of 7,8-dichlorochroman-4-ol

The title compound (6.55 g) was obtained as a yellow solid in a manner similar to (EXAMPLE 16) <Step 1>, in which the compound (7.10 g) obtained in (EXAMPLE 39) <Step 2> was used as the starting material.

### <Step 4> Synthesis of t-butyl 4-(7,8-dichlorochroman-4-yl)piperazine-1-carboxylate

A white solid (5.3 g) was obtained in a manner similar to (EXAMPLE 30) <Step 1>, in which the compound (6.52 g) obtained in (EXAMPLE 39) <Step 3> was used as the starting material. The title compound (6.1 g) was obtained as a white solid in a manner similar to (EXAMPLE 27) <Step 6>, in which this white solid (5.19 g) was used as the starting material.

### <Step 5> Synthesis of 1-(7,8-dichlorochroman-4-yl)piperazine dihydrochloride

The title compound (527 mg) was obtained as a pale yellow solid in a manner similar to (EXAMPLE 24) <Step 3>, in which the compound (0.60 g) obtained in (EXAMPLE 39) <Step 4> was used as the starting material.

### <Step 6> Synthesis of 4-(7,8-dichloro-3,4-dihydro-2H-chromen-4-yl)-N-(pyridazin-3-yl)piperazine-1-carbo xamide

The title compound (0.47 g) was obtained as a colorless amorphous substance in a manner similar to (EXAMPLE 2) <Step 7>, in which the compound (0.51 g) obtained in (EXAMPLE 39) <Step 5> was used as the starting material.

A part of the resulting title compound was optically resolved into a first fraction (retention time: 10.9 mins.) and a second fraction (retention time: 12.6 mins.), using HPLC (column: CHIRALPAK AD-H (4.6 mm x 150 mm), eluate; hexane : ethanol = 50:50 (v/v, flow rate: 1.6 mL/min., UV: 254 nm detection, column temperature: 40°C).

Compounds 40 - 50 in the following EXAMPLES were obtained in a manner similar to (EXAMPLE 2) <Step 7>, in which the compound obtained in (EXAMPLE 11) <Step 4> was used as the starting material.

### (EXAMPLE 40)

### N-(5-Chloropyridine-2-yl)-4-[7-(trifluoromethyl)-3,4-dihydro-2H-chromen-4-yl]pipe razine-1-carboxamide

### (EXAMPLE 41)

### N-(Pyridine-2-yl)-4[7-(trifluoromethyl)-3,4-dihydro-2H-chromen-4-yl]piperazine-1-carboxamide

### (EXAMPLE 42)

### N-(5-Bromopyridine-2-yl)-4-[7-(trifluoromethyl)-3,4-dihydro-2H-chromen-4-yl]pipe razine-1-carboxamide

### (EXAMPLE 43)

### N-(6-Methoxypyridine-3-yl)-4-[7-(trifluoromethyl)-3,4-dihydro-2H-chromen-4-yl]pi perazine-1-carboxamide 2,2,2-trifluoroacetic acid salt

### (EXAMPLE 44)

### N-[6-(dimethylamino)pyridine-3-yl]-4-[7-(trifluoromethyl)-3,4-dihydro-2H-chromen -4-yl]piperazine-1-carboxamide

### (EXAMPLE 45)

### N-(1H-Tetrazol-5-yl)-4-[7-(trifluoromethyl)-3,4-dihydro-2H-chromen-4-yl]piperazin e-1-carboxamide

### (EXAMPLE 46)

### N-(Pyrimidin-2-yl)-4-[7-(trifluoromethyl)-3,4-dihydro-2H-chromen-4-yl]piperazine-1-carboxamide

### (EXAMPLE 47)

### N-(6-Chloropyridazin-3-yl)-4-[7-(trifluoromethyl)-3,4-dihydro-2H-chromen-4-yl]pip erazine-1-carboxamide

### (EXAMPLE 48)

### N-(5-Methylpyridine-3-yl)-4-[7-(trifluoromethyl)-3,4-dihydro-2H-chromen-4-yl]pipe razine-1-carboxamide

### (EXAMPLE 49)

### N-(4-Methylpyridine-3-yl)-4-[7-(trifluoromethyl)-3,4-dihydro-2H-chromen-4-yl]pipe razine-1-carboxamide

### (EXAMPLE 50)

### N-(6-Methoxypyrimidin-4-yl)-4-[7-(trifluoromethyl)-3,4-dihydro-2H-chromen-4-yl] piperazine-1-carboxamide

### (EXAMPLE 51)

### N-(1,2-Benzoxazol-3-yl)-4-[7-(trifluoromethyl)-3,4-dihydro-2H-chromen-4-yl]piper azine-1-carboxamide

The title compound (88 mg) was obtained as a white solid in a manner similar to (EXAMPLE 2) <Step 7>, in which the compound (150 mg) obtained in (EXAMPLE 11) <Step 4> was used as the starting material.

A part of the resulting title compound was optically resolved into a first fraction (retention time: 7.9 mins.) and a second fraction (retention time: 12.0 mins.), using HPLC (column: CHIRALPAK AY-H (4.6 mm x 150 mm), eluate; hexane : ethanol = 90:10 (v/v), flow rate: 3 mL/min., UV: 254 nm detection, column temperature: 40°C).

### (EXAMPLE 52)

### N-(Pyrazin-2-yl)-4-[7-(trifluoromethyl)-3,4-dihydro-2H-chromen-4-yl]piperazine-1-carboxamide

### <Step 1> Synthesis of 2,2,2-trichloro-N-(pyrazin-2-yl)acetamide

The title compound (1.9 g) was obtained as a brown solid in a manner similar to (EXAMPLE 27) <Step 3>in which aminopyrazine (1.00 g) and trichloroacetyl chloride (1.29 mL) were used as the starting materials.

### <Step 2> Synthesis of N-(pyrazin-2-yl)-4-[7-(trifluoromethyl)-3,4-dihydro-2H-chromen-4-yl]piperazine-l-c arboxamide

To an acetonitrile solution (3 mL) of the compound (0.30 g) obtained in (EXAMPLE 11) <Step 4> and 1,8-diazabicyclo[5.4.0]undec-7-ene (0.31 mL) was added the compound (0.28 g) obtained in (EXAMPLE 52) <Step 1>. The mixture was stirred for 14 hours while heating to reflux. Water was added thereto, followed by extraction with ethyl acetate. The solvent was removed by distillation under reduced pressure, and the resulting residue was purified by silica gel column chromatography (eluate; n-hexane : ethyl acetate = 20:80 to 10:90) to give the title compound (0.11 g) as a yellow amorphous substance.

A part of the resulting title compound was optically resolved into a first fraction (retention time: 9.4 mins.) and a second fraction (retention time: 13.2 mins.), using HPLC (column: CHIRALPAK AY-H (4.6 mm x 150 mm), eluate; hexane : ethanol = 90:10 (v/v), flow rate: 3 mL/min., UV: 254 nm detection, column temperature: 40°C).

Compounds 53 - 65 in the following EXAMPLES were obtained in a manner similar to (EXAMPLE 2) <Step 7>, in which the compound obtained in (EXAMPLE 11) <Step 4> was used as the starting material.

### (EXAMPLE 53)

### N-(5-Methylpyridine-2-yl)-4-[7-(trifluoromethyl)-3,4-dihydro-2H-chromen-4-yl]pipe razine- 1-carboxamide

### (EXAMPLE 54)

### N-(5-Bromopyrimidin-2-yl)-4-[7-(trifluoromethyl)-3,4-dihydro-2H-chromen-4-yl]pi perazine-1-carboxamide

### (EXAMPLE 55)

### N-(1,2-Oxazol-3-yl)-4-[7-(trifluoromethyl)-3,4-dihydro-2H-chromen-4-yl]piperazine -1-arboxamide

### (EXAMPLE 56)

### N-(5-Methyl-1,2-oxazol-3-yl)-4-[7-(trifluoromethyl)-3,4-dihydro-2H-chromen-4-yl] piperazine-1-carboxamide

### (EXAMPLE 57)

### N-(3,4-Dimethyl-1,2-oxazol-5-yl)-4-[7-(trifluoromethyl)-3,4-dihydro-2H-chromen-4 -yl]piperazine-1-carboxamide

### (EXAMPLE 58)

### N-(1,3,4-Thiadiazol-2-yl)-4-[7-(trifluoromethyl)-3,4-dihydro-2H-chromen-4-yl]piper azine-1-carboxamide

### (EXAMPLE 59)

### N-(Pyrimidin-4-yl)-4-[7-(trifluoromethyl)-3,4-dihydro-2H-chromen-4-yl]piperazine-1-carboxamide

### (EXAMPLE 60)

### N-(1H-Pyrazol-5-yl)-4-[7-(trifluoromethyl)-3,4-dihydro-2H-chromen-4-yl]piperazine -1-arboxamide

### (EXAMPLE 61)

### N-(5-Methyl-1H-pyrazol-3-yl)-4-(7-(trifluoromethyl)-3,4-dihydro-2H-chromen-4-yl] piperazine-1-carboxamide

### (EXAMPLE 62)

### N-[6-(Methylamino)pyridine-3-yl]-4-[7-(trifluoromethyl)-3,4-dihydro-2H-chromen-4 -yl]piperazine-1-carboxamide

### (EXAMPLE 63)

### N-(2-Ethylpyrazol-3-yl)-4-[7-(trifluoromethyl)-3,4-dihydro-2H-chromen-4-yl]pipera zine-1-carboxamide

### (EXAMPLE 64)

### N-(2-Methylpyrazol-3-yl)-4-[7-(trifluoromethyl)-3,4-dihydro-2H-chromen-4-yl]piper azine-1-carboxamide

### (EXAMPLE 65)

### N-(6-Aminopyridine-3-yl)-4-[7-(trifluoromethyl)-3,4-dihydro-2H-chromen-4-yl]pipe raaine-1-carboxamide

### (EXAMPLE 66)

### N-(6-Acetamidopyridin-3-yl)-4-[7-(trifluoromethyl)-3,4-dihydro-2H-chromen-4-yl]p iperaaine-1-carboxamide

### <Step 1> Synthesis of tert-butyl 4-[7-(trifluoromethyl)chroman-4-yl]piperazine-1-carboxylate

The title compound (5.6 g) was obtained as a yellow amorphous substance in a manner similar to (EXAMPLE 27) <Step 6>, in which the compound (4.50 g) obtained in (EXAMPLE 30) <Step 1> was used as the starting material.

### <Step 2> Synthesis of 1-[7-(trifluoromethyl)chroman-4-yl]piperazine dihydrochloride

The title compound (1.91 g) was obtained as a white solid in a manner similar to (EXAMPLE 24) <Step 3>, in which the compound (2.00 g) obtained in (EXAMPLE 66) <Step 1> was used as the starting material.

### <Step 3> Synthesis of N-(6-acetamidopyridine-3-yl)-4-[7-(trifluoromethyl)-3,4-dihydro-2H-chromen-4-yl]p iperazine-1-carboxamide

The title compound (0.72 g) was obtained as a white solid in a manner similar to (EXAMPLE 2) <Step 7>, in which the compound (0.60 g) obtained in (EXAMPLE 66) <Step 2> was used as the starting material.

A part of the resulting title compound was optically resolved into a first fraction (retention time: 6.4 mins.) and a second fraction (retention time: 8.8 mins.), using HPLC (column: CHIRALPAK IC (4.6 mm x 150 mm), eluate; hexane : ethanol = 90:10 (v/v), flow rate: 3 mL/min., UV: 254 nm detection, column temperature: 40°C).

### (EXAMPLE 67)

### N-(6-Carbamoylpyridine-3-yl)-4-[7-(trifluoromethyl)-3,4-dihydro-2H-chromen-4-yl] piperazine-1-carboxamide

### <Step 1> Synthesis of phenyl N-(6-carbamoylpyridine-3-yl) carbamate

Under ice cooling, a tetrahydrofuran solution (3 mL) of phenyl chloroformate (0.62 mL) was added to a tetrahydrofuran-acetonitrile solution (1:2, 9 mL) of 6-aminonicotinamide (0.61 g) and pyridine(0.40 mL), and the mixture was stirred for 0.5 hours. The mixture was stirred at room temperature for further 2 hours. The solid precipitated was taken out by filtration and washed with ethyl acetate to give the title compound (0.84 g) as a pale yellow solid.

### <Step 2> Synthesis of N-(6-carbamoylpyridine-3-yl)-4-[7-(trifluoromethyl)-3,4-dihydro-2H-chromen-4-yl] piperazine-1-carboxamide

The title compound (0.29 g) was obtained as a beige solid in a manner similar to (EXAMPLE 2) <Step 7>, in which the compound (0.35 g) obtained in (EXAMPLE 67) <Step 1> and the compound (0.32 g) obtained in (EXAMPLE 11)

### <Step 4> were used as the starting materials.

The resulting title compound was optically resolved into a first fraction (retention time: 9.1 mins.) and a second fraction (retention time: 11.5 mins.), using HPLC (column: CHIRALPAK AY-H (4.6 mm x 150 mm), eluate; hexane : ethanol = 90:10 (v/v), flow rate: 3 mL/min., UV: 254 nm detection, column temperature: 40°C).

### (EXAMPLE 68)

### N-(3-Chloropyrazin-2-yl)-4-[7-(trifluoromethyl)-3,4-dihydro-2H-chromen-4-yl]piper azine-1-carboxamide

### <Step 1> Synthesis of 2,2,2-trichloro-N-(3-chloropyrazin-2-yl)acetamide

The title compound (1.7 g) was obtained as a brown oily substance in a manner similar to (EXAMPLE 27) <Step 3>, in which 2-amino-3-chloropyrazine (2.00 g) and trichloroacetyl chloride (1.90 mL) were used as the starting materials.

### <Step 2> Synthesis of N-(3-chloropyrazin-2-yl)-4-[7-(trifluoromethyl)-3,4-dihydro-2H-chromen-4-yl]piper azine-1-carboxamide

The title compound (79.3 mg) was obtained as a yellow solid in a manner similar to (EXAMPLE 52) <Step 2>, in which the compound (1.69 g) obtained in (EXAMPLE 68) <Step 1> and the compound (1.60 g) obtained in (EXAMPLE 11)

### <Step 4> were used as the starting materials.

The resulting title compound was optically resolved into a first fraction (retention time: 8.6 mins.) and a second fraction (retention time: 11.5 mins.), using HPLC (column: CHIRALPAK AY-H (4.6 mm x 150 mm), eluate; ethanol, flow rate: 1 mL/min., UV: 254 nm detection, column temperature: 40°C).

### (EXAMPLE 69)

### N-(6-Cyanopyridine-3-yl)-4-[7-(trifluoromethyl)-3,4-dihydro-2H-chromen-4-yl]pipe razine-1-carboxamide

The title compound (407 mg) was obtained as a white solid in a manner similar to (EXAMPLE 2) <Step 7>, in which the compound (0.60 g) obtained in (EXAMPLE 66) <Step 3> and phenyl (6-cyanopyridine-3-yl)carbamate (0.48 g) were used as the starting materials.

The resulting title compound was optically resolved into a first fraction (retention time: 6.4 mins.) and a second fraction (retention time: 7.4 mins.), using HPLC (column: CHIRALPAK AY-H (4.6 mm x 150 mm), eluate; hexane : ethanol = 90:10 (v/v), flow rate: 3 mL/min., UV: 254 nm detection, column temperature: 40°C).

Similarly, the optical isomers can be fractionated from the other compounds of EXAMPLES which contain a chiral center, using HPLC (Daicel Chemical. Industries, Ltd., 5 µH series column for normal phase mode: CHIRALPAK AD-H (20 mm x 250 mm), etc. by appropriately choosing the eluate from hexane: isopropanol = 90:10 (v/v), etc.

The structures of the compounds synthesized in (EXAMPLE 1) through (EXAMPLE 69) described above are shown in [Compound List 1]. The intermediates, etc. in these EXAMPLES are also shown in [Compound List 2] and [Compound List 3]. The NMR data in the EXAMPLES are shown in the table below, wherein no asterisk mark denotes data at 300 MHz and the mark * denotes data at 400 MHz.

### [Compound List 1]

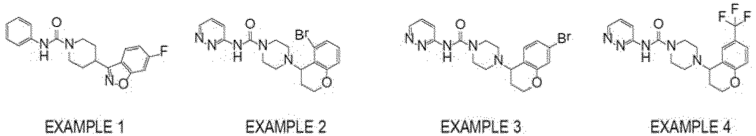

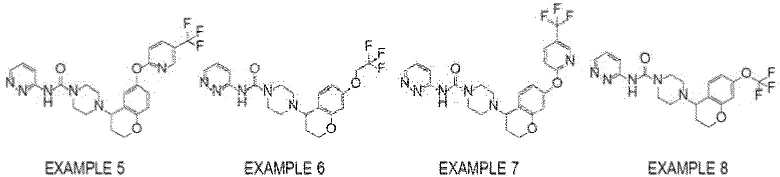

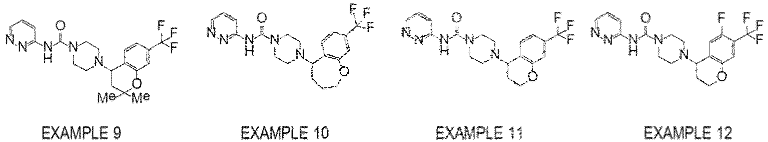

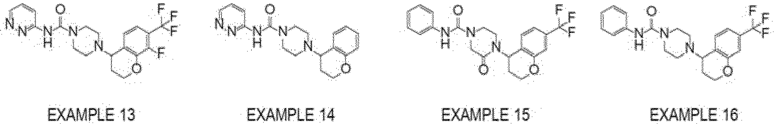

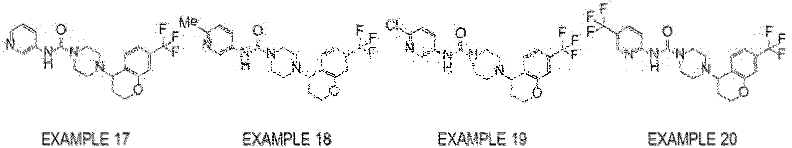

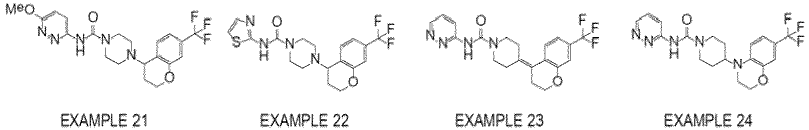

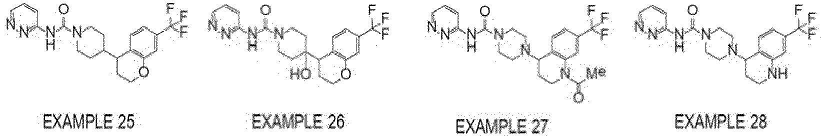

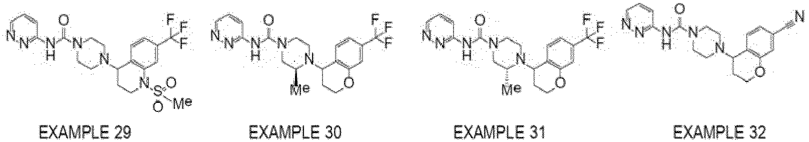

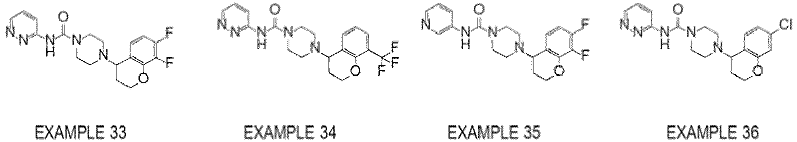

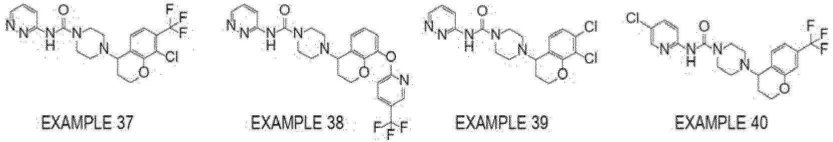

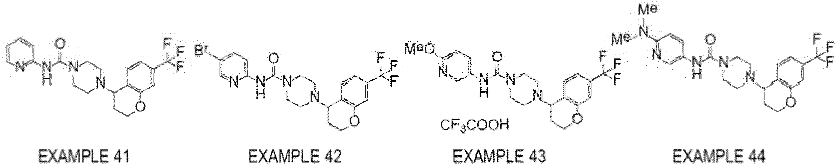

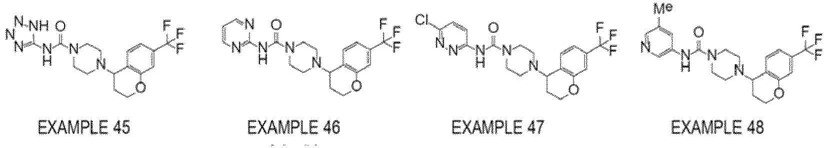

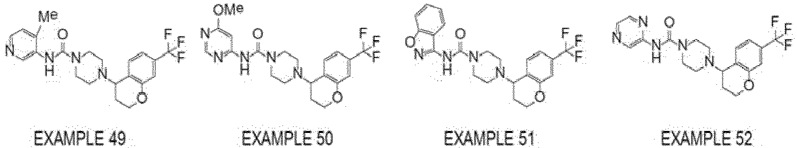

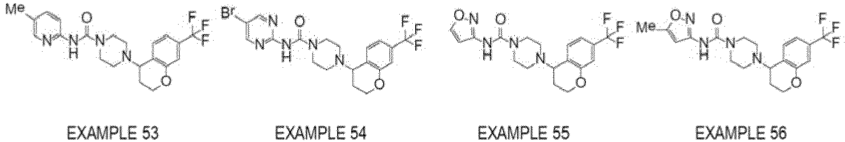

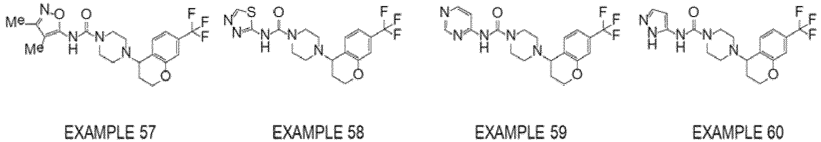

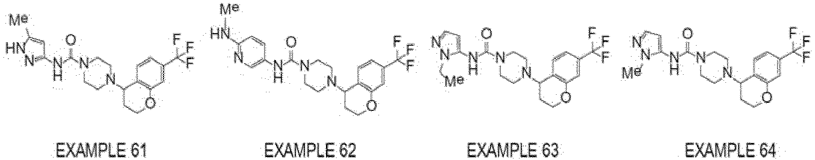

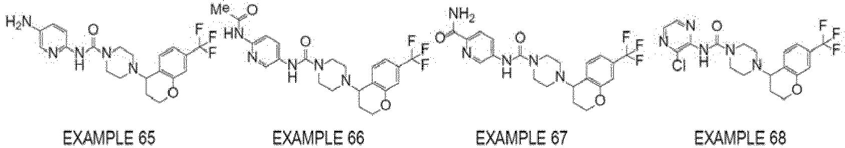

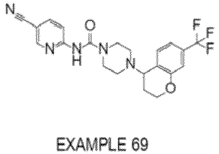

**[TABLE 2-1]**

| EXAMPLE No. | MS-ESI (m/z) (M+H)⁺ | Retention Time (min) | EXAMPLE No. | MS-ESI (m/z) (M+H)⁺ | Retention Time (min) |
|---|---|---|---|---|---|
| 1 | 340 | 5.47 | 12 | 426 | 5.78 |
| 2 | 416* | 5.48 | 13 | 426 | 5.62 |
| 3 | 416* | 5.55 | 14 | 338* | 3.57 |
| 4 | 408 | 5.58 | 15 | 420 | 6.20 |
| 5 | 501 | 5.67 | 16 | 406 | 6.10 |
| 6 | 438 | 4.90 | 17 | 407 | 5.60 |
| 7 | 501 | 5.48 | 18 | 421 | 5.38 |
| 8 | 424 | 5.72 | 19 | 441 | 6.05 |
| 9 | 436 | 6.15 | 20 | 475 | 6.40 |
| 10 | 422 | 4.62 | 21 | 438 | 5.92 |
| 11 | 408 | 5.68 | 22 | 413 | 5.92 |

| | | | | | |
|---|---|---|---|---|---|
| *: (M-1)⁻ | | | | | |

**[TABLE 2-2]**

| EX. No. | MS-ESI (m/z) (M+H)⁺ | Retention Time (min) | EX. No. | MS-ES I (m/z) (M+H)⁺ | Retention Time (min) |
|---|---|---|---|---|---|
| 23 | 405 | 5.88 | 47 | 442 | 5.93 |
| 24 | 408 | 5.57 | 48 | 421 | 5.72 |
| 25 | 407 | 5.73 | 49 | 421 | 5.47 |
| 26 | 423 | 5.37 | 50 | 438 | 6.10 |
| 27 | 449 | 4,98 | 51 | 447 | 6.07 |
| 28 | 407 | 3.97 | 52 | 408 | 5.70 |
| 29 | 485 | 5.23 | 53 | 423# | 5.97 |
| 30 | 422 | 5.93 | 54 | 486 | 5.98 |
| 31 | 422 | 5.87 | 55 | 397 | 5.75 |
| 32 | 365 | 4.70 | 56 | 441 | 5.90 |
| 33 | 376 | 4.97 | 57 | 425 | 5.83 |
| 34 | 408 | 5.37 | 58 | 414 | 5.77 |
| 35 | 375 | 4.65 | 59 | 408 | 5.85 |
| 36 | 374 | 5.28 | 60 | 396 | 5.57 |
| 37 | 442 | 5.77 | 61 | 410 | 5.72 |
| 38 | 501 | 5.35 | 62 | 436 | 3.82 |
| 39 | 408 | 5.73 | 63 | 424 | 5.65 |
| 40 | 441 | 6.28 | 64 | 410 | 5.53 |
| 41 | 407 | 5.78 | 65 | 422 | 3.78 |
| 42 | 507# | 6.33 | 66 | 464 | 5.65 |
| 43 | 437 | 5.92 | 67 | 450 | 5.62 |
| 44 | 450 | 4.48 | 68 | 464# | 5.75 |
| 45 | 398 | 6.52 | 69 | 432 | 5.92 |
| 46 | 408 | 5.48 | | | |

| | | | | | |
|---|---|---|---|---|---|
| #: (M+Na)⁺ | | | | | |

**[TABLE 3]**

| EX. No. | NMR data (δ: ppm) <no asterisk: 300MHz, *: 400MHz > |
|---|---|
| 2* | *(CDCl₃)* 8.81 (1H, d, J = 3Hz), 8.34-8.21 (1H, m), 8.11 (1H, brs), 7.46-7.33 (1H, m), 7.14 (1H, dd, J = 8,1 Hz), 7.07-7.01 (1H, m), 6.83-6.78 (1H, m), 4.47-4.36 (1H, m), 4.31-4.21 (1H, m), 4.00-3.91 (1H, m), 3.66-3.39 (4H, m), 2.96-2.79 (2H, m), 2.71-2.48 (2H, m), 2.13-2.03 (1H, m), 1.88-1.74 (1H, m). |
| 3 | *(CDCl₃)* 8.87-8.77 (1H, m), 8.33-8.21 (1H, m), 8.09 (1H, brs), 7.47-7.32 (2H, m), 7.02 (1H, dd, J = 8, 2Hz), 6.97 (1H, d, J = 2Hz), 4.41-4.30 (1H, m), 4.18-4.07 (1H, m), 3.86 (1H, dd, J = 9, 6Hz), 3.72-3.42 (4H, m), 2.80-2.41 (4H, m), 2.15-1.87 (2H, m). |
| 4* | *(CDCl₃)* 8.83 (1H, d, J = 4Hz), 8.28 (1H, d, J = 9Hz), 7.87 (1H, s), 7.80 (1H, s), 7.48-7.34 (2H, m), 6.87 (1H, d, J = 9 Hz), 4.46-4.39 (1H, m), 4.24-4.14 (1H, m), 3.95 (1H, dd, J = 9, 6Hz), 3.70-3.53 (4H, m), 2.80-2.64 (2H, m), 2.64-2.46 (2H, m), 2.15-1.94 (2H, m). |
| 5* | *(CDCl₃)* 8.85 (1H, d, J = 5Hz), 8.44 (1H, s), 8.29 (1H, d, J = 9Hz), 7.89 (1H, dd, J = 9, 2Hz), 7.63 (1H, s), 7.42 (1H, dd, J = 9, 5Hz), 7.35 (1H, d, J = 3Hz), 7.00 (1H, d, J = 9Hz), 6.95 (1H, dd, J = 9, 3Hz), 6.87 (1H, d, J = 9Hz), 4.44-4.35 (1H, m), 4.17 (1H, ddd, J = 11, 11, 2Hz), 4.02 (1H, dd, J = 9, 6Hz), 3.65-3.51 (4H, m), 2.79-2.70 (2H, m), 2.63-2.55 (2H, m), 2.16-1.95 (2H, m). |
| 6 | *(CDCl₃)* 8.85 (1H, d, J = 3 Hz), 8.29 (1H, d, J = 9 Hz), 7.71 (1H, s), 7.47-7.36 (2H, m), 6.55 (1H, dd, J = 9, 2Hz), 6.39 (1H, d, J = 3Hz),4.42-4.32 (1H, m), 4.32 (2H, q, J = 8Hz), 4.20-4.09 (1H, m), 3.92-3.83 (1H, m), 3.67-3.51 (4H, m), 2.76-2.64 (2H, m), 2.61-2.50 (2H, m), 2.13-1.89 (2H, m). |
| 7* | *(CDCl₃)* 8.85 (1H, d, J = 4 Hz), 8.50-8.45 (1H, m), 8.30 (1H, d, J = 8 Hz), 7.91 (1H, dd, J = 9, 3 Hz), 7.80 (1H, brs), 7.48-7.40 (1H, m), 7.43 (1H, dd, J = 9, 5 Hz), 7.02 (1H, d, J = 9 Hz), 6.76-6.67 (1H, m), 6.63 (1H, d, J = 3 Hz), 4.43-4.36 (1H, m), 4.23-4.15 (1H, m), 3.98-3.91 (1H, m), 3.68-3.55 (4H, m), 2.81-2.71 (2H, m), 2.65-2.55 (2H, m), 2.16-2.05 (1H, m), 2.03-1.94 (1H, m). |
| 8 | *(CDCl₃)* 8.83 (1H, d, J = 4 Hz), 8.28 (1H, d, J = 9 Hz), 7.78 (1H, brs), 7.50 (1H, d, J = 8 Hz), 7.46-7.36 (1H, m), 6.76 (1H, d, J = 8 Hz), 6.68 (1H, s), 4.44-4.33 (1H, m), 4.22-4.10 (1H, m), 3.96-3.86 (1H, m), 3.66-3.51 (4H, m), 2.75-2.63 (2H, m), 2.61-2.49 (2H, m), 2.14-1.89 (2H, m). |
| 9* | *(CDCl₃)* 8.85 (1H, d, J = 4 Hz), 8.29 (1H, d, J = 9 Hz), 7.81-7.72 (2H, m), 7.47-7.39 (1H, m), 7.15 (1H, d, J = 8 Hz), 7.06 (1H, s), 4.10-4.01 (1H, m), 3.69-3.55 (4H, m), 2.71-2.55 (4H, m), 1.92-1.83 (1H, m), 1.79 (1H, dd, J = 12, 12 Hz), 1.48 (3H, s), 1.27 (3H, s). |
| 10* | *(CDCl₃)* 8.83 (1H, d, J = 4 Hz), 8.29 (1H, d, J = 9 Hz), 7.99 (1H, s), 7.48-7.36 (2H, m), 7.32-7.24 (2H, m), 4.33-4.22 (1H, m), 4.01-3.91 (1H, m), 3.69-3.53 (5H, m), 2.70-2.58 (2H, m), 2.47-2.35 (2H, m), 2.31-2.18 (1H, m), 2.12-2.00 (1H, m), 1.99-1.88 (1H, m), 1.87-1.76 (1H, m). |
| 11 | *(CDCl₃)* 8.84 (1H, d, J = 4 Hz), 8.28 (1H, d, J = 9 Hz), 7.80 (1H, brs), 7.63 (1H, d, J = 8 Hz), 7.46-7.36 (1H, m), 7.14 (1H, d, J = 8 Hz), 7.06 (1H, s), 4.46-4.36 (1H, m), 4.23-4.12 (1H, m), 4.01-3.92 (1H, m), 3.69-3.51 (4H, m), 2.76-2.51 (4H, m), 2.16-1.93 (2H, m). |
| 12 | *(CDCl₃)* 8.86 (1H, d, J = 4 Hz), 8.29 (1H, d, J = 9 Hz), 7.68 (1H, brs), 7.50-7.37 (2H, m), 7.03 (1H, d, J = 6 Hz), 4.46-4.33 (1H, m), 4.21-4.08 (1H, m), 4.06-3.92 (1H, m), 3.68-3.52 (4H, m), 2.78-2.52 (4H, m), 2.15-1.92 (2H, m). |
| 13 | *(CDCl₃)* 8.84 (1H, d, J = 4 Hz), 8.27 (1H, d, J = 9 Hz), 7.84 (1H, brs), 7.47-7.34 (2H, m), 7.09 (1H, dd, J = 7, 8Hz), 4.57-4.47 (1H, m), 4.29-4.18 (1H, m), 4.03-3.93 (1H, m), 3.68-3.52 (4H, m), 2.75-2.50 (4H, m), 2.19-1.96 (2H, m). |
| 14 | *(CDCl₃)* 8.82 (1H, d, J = 4Hz), 8.33 (1H, d, J = 9 Hz), 7.50 (1H, d, J = 8 Hz), 7.44 (1H, dd, J = 9, 4Hz), 7.16 (1H, dd, J = 8, 8Hz), 6.92 (1H, dd, J = 8, 8 Hz), 6.81 (1H, d, J = 8 Hz), 4.43-4.32 (1H, m), 4.22-4.10 (1H, m), 4.00-3.87 (1H, m), 3.69-3.53 (4H, m), 2.77-2.66 (2H, m), 2.62-2.51 (2H, m), 2.17-1.91 (2H, m). |
| 15* | *(CDCl₃)* 7.42-7.28 (4H, m), 7.20-7.05 (4H, m), 6.41 (1H, s), 6.13-6.05 (1H, m), 4.44-4.39 (1H, m), 4.37-4.32 (2H, m), 4.31-4.21 (1H, m), 4.03-3.93 (1H, m), 3.51-3.42 (1H, m), 3.36-3.28 (1H, m), 3.14-3.04 (1H, m), 2.28-2.11 (2H, m). |
| 16 | *(DMSO-d₆)* 8.51 (1H, s), 7.72 (1H, d, J = 8 Hz), 7.45 (2H, dd, J = 9, 1 Hz), 7.27-7.19 (3H, m), 7.09-7.06 (1H, m), 6.97-6.90 (1H, m), 4.46-4.35 (1H, m), 4.30-4.14 (1H, m), 4.02 (1H, dd, J = 7, 7Hz), 3.58-3.41 (4H, m), 2.62-2.42 (4H, m), 2.06-1.95 (2H, m). |
| 17* | *(CDCl₃)* 8.42 (1H, d, J = 2 Hz), 8.28 (1H, dd, J = 5, 1 Hz), 8.00-7.95 (1H, m), 7.63 (1H, d, J = 8 Hz), 7.25-7.22 (1H, m), 7.14 (1H, d, J = 8 Hz), 7.06 (1H, s), 6.40 (1H, s), 4.45-4.37 (1H, m), 4.22-4.13 (1H, m), 4.00-3.94 (1H, m), 3.57-3.52 (4H, m), 2.73-2.65 (2H, m), 2.61-2.53 (2H, m), 2.14-1.95 (2H, m). |
| 23 | *(CDCl₃)* 8.84 (1H, d, J = 5 Hz), 8.32 (1H, d, J = 9 Hz), 8.17-7.85 (1H, m), 7.43 (1H, dd, J = 9, 5 Hz), 7.26 (2H, d, J = 8 Hz), 7.13-7.06 (2H, m), 4.34 (2H, t, J = 6 Hz), 3.71 (2H, t, J = 6 Hz), 3.64 (2H, t, J = 6 Hz), 2.93-2.82 (2H, m), 2.71-2.57 (4H, m). |
| 35 | *(CDCl₃)* 8.42 (1H, d, J = 2 Hz), 8.28 (1H, dd, J = 4,1 Hz), 8.01-7.94 (1H, m), 7.29-7.15 (2H, m), 6.75-6.64 (1H, m), 6.40 (1H, s), 4.54-4.43 (1H, m), 4.28-4.17 (1H, m), 3.94-3.84 (1H, m), 3.56-3.49 (4H, m), 2.74-2.62 (2H, m), 2.60-2.49 (2H, m), 2.16-1.92 (2H, m). |
| 52* | *(CDCl₃)* 9.36 (1H, d, J = 2 Hz), 8.25 (1H, d, J = 3 Hz), 8.15 (1H, dd, J = 3, 1 Hz), 7.63 (1H, d, J = 8 Hz), 7.14 (1H, d, J = 8 Hz), 7.11-7.03 (2H, m), 4.45-4.37 (1H, m), 4.22-4.13 (1H, m), 4.00-3.94 (1H, m), 3.63-3.52 (4H, m), 2.74-2.65 (2H, m), 2.62-2.53 (2H, m), 2.14-1.95 (2H, m). |
| 67 | *(CDCl₃)* 8.51 (1H, d, J = 2 Hz), 8.13 (1H, d, J = 8 Hz), 7.99 (1H, dd, J = 8, 2 Hz), 7.73-7.66 (1H, m), 7.63 (1H, d, J = 8 Hz), 7.14 (1H, d, J = 8 Hz), 7.06 (1H, d, J = 2 Hz), 6.62 (1H, s), 5.54-5.44 (1H, m), 4.46-4.36 (1H, m), 4.23-4.12 (1H, m), 4.01-3.93 (1H, m), 3.56 (4H, t, J =5 Hz), 2.75-2.64 (2H, m), 2.63-2.52 (2H, m), 2.19-1.93 (2H, m). |

### [Compound List 2]

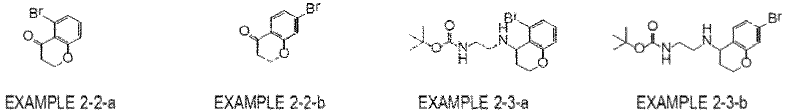

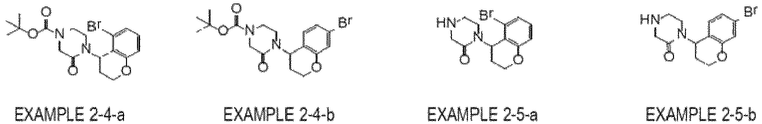

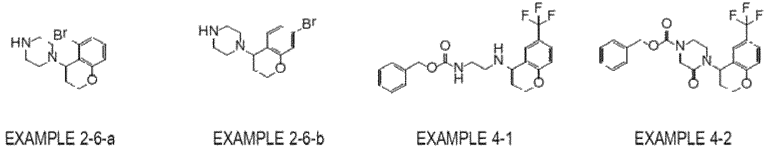

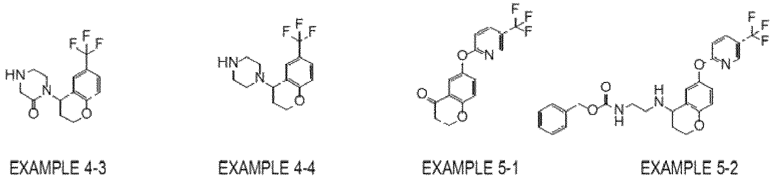

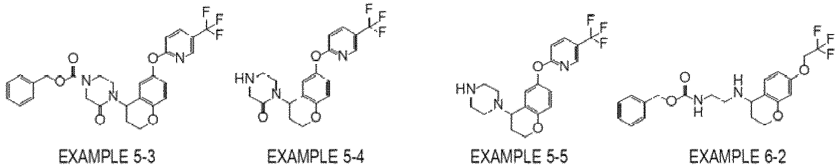

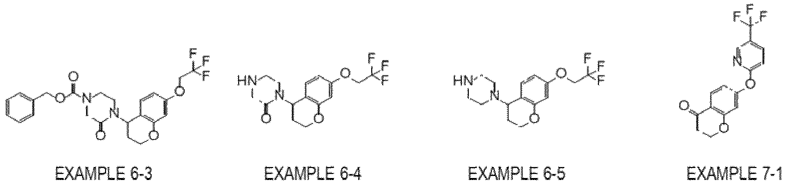

### [Compound List 3]

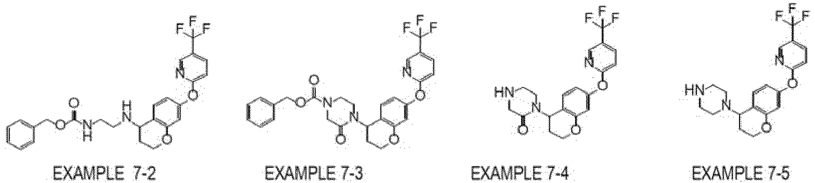

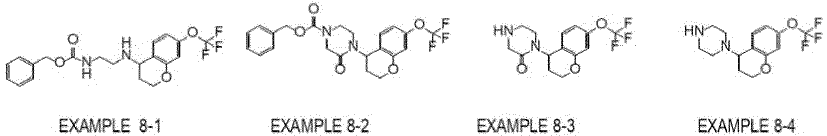

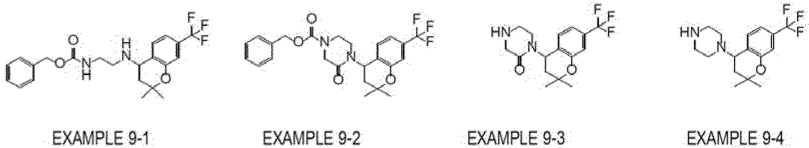

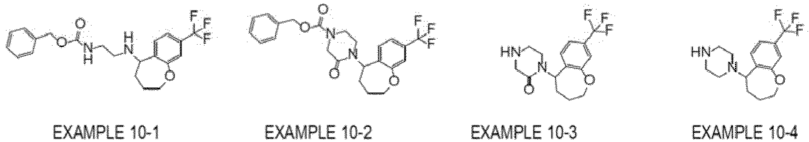

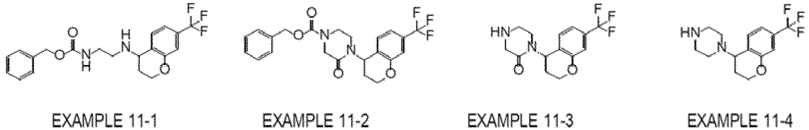

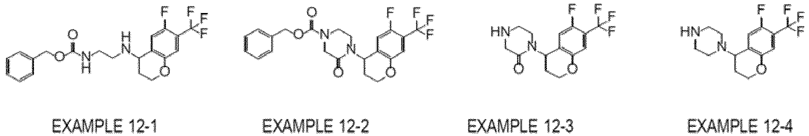

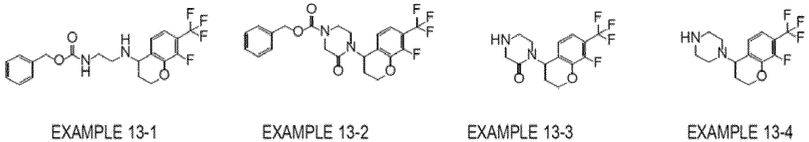

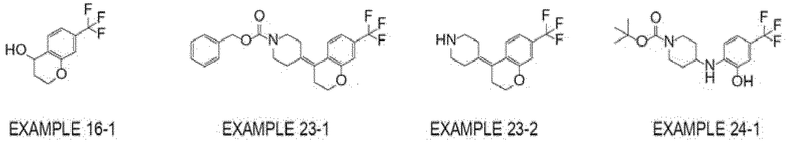

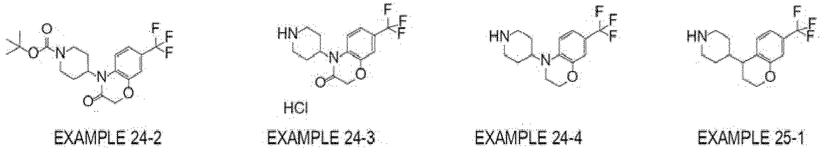

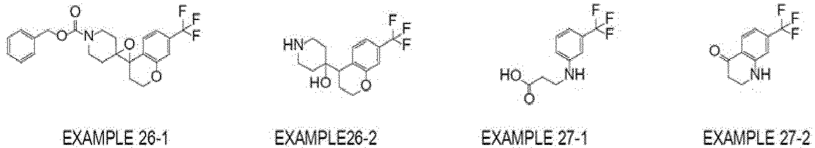

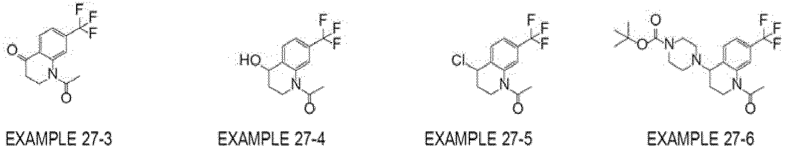

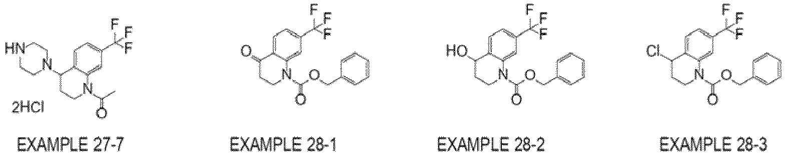

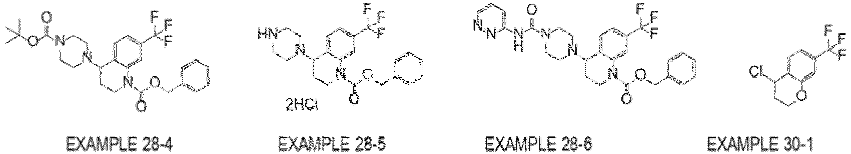

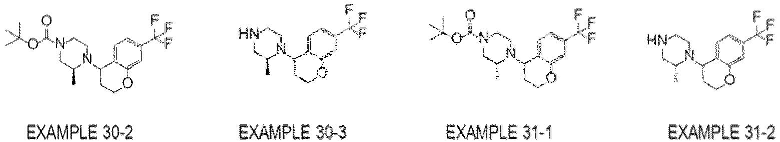

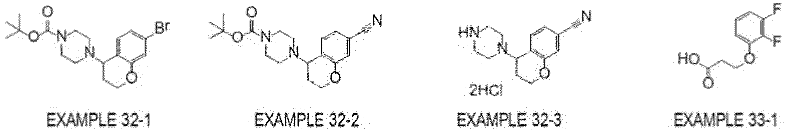

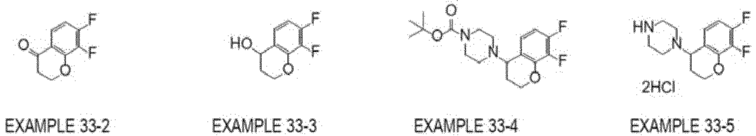

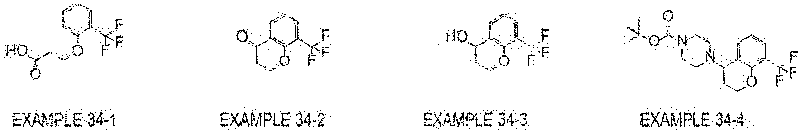

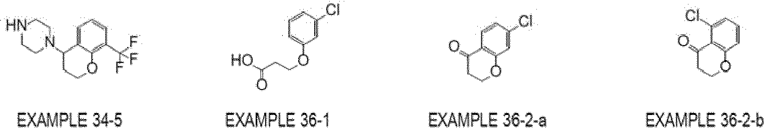

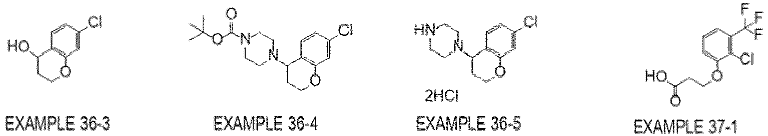

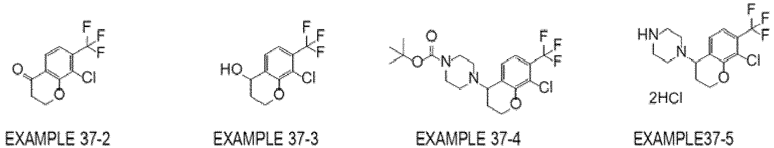

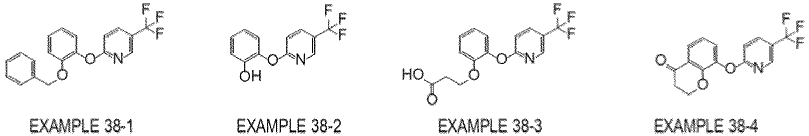

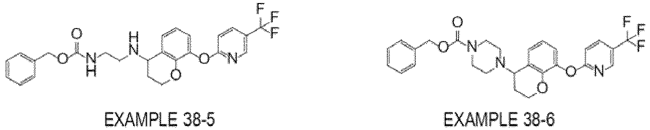

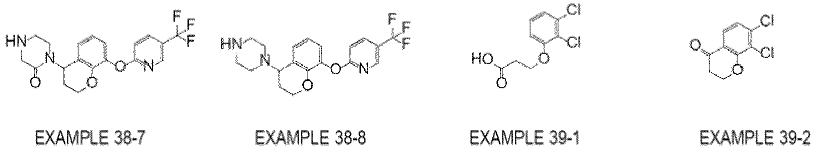

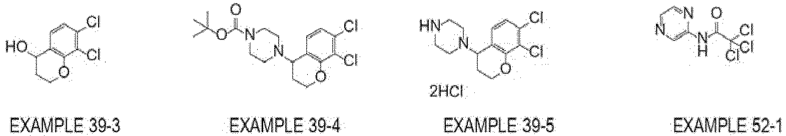

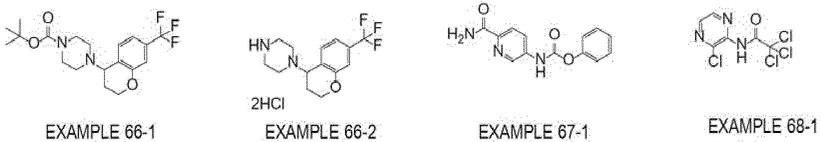

**[TABLE 4-1]**

| EX. No. | MS-ESI (m/z) (M+H)⁺ | Retention Time (min) | EX. No. | MS-ESI (m/z) (M+H)⁺ | Retention Time (min) |
|---|---|---|---|---|---|
| 2-2-a | 227 | 4.65 | 7-5 | 380 | 3.53 |
| 2-3-a | 371 | 3.90 | 8-1 | 411 | 4.20 |
| 2-3-b | 371 | 3.75 | 8-2 | 451 | 6.03 |
| 2-4-a | 411 | 5.68 | 8-3 | 317 | 2.98 |
| 2-4-b | 411 | 5.90 | 8-4 | 303 | 3.35 |
| 2-5-a | 311 | 1.38 | 9-1 | 423 | 4.52 |
| 2-5-b | 311 | 2.15 | 9-2 | 463 | 6.16 |
| 2-6-a | 297 | 2.67 | 9-3 | 329 | 3.62 |
| 2-6-b | 297 | 2.87 | 9-4 | 315 | 3.66 |
| 4-1 | 395 | 4.07 | 10-1 | 409 | 4.08 |
| 4-2 | 435 | 5.92 | 10-2 | 449 | 5.88 |
| 4-3 | 301 | 2.28 | 10-3 | 315 | 2.92 |
| 4-4 | 267 | 5.67 | 10-4 | 301 | 3.02 |
| 5-1 | 310 | 5.52 | 11-1 | 395 | 4.22 |
| 5-2 | 488 | 4.30 | 11-2 | 435 | 6.05 |
| 5-3 | 528 | 6.03 | 11-3 | 301 | 2.63 |
| 5-4 | 394 | 3.47 | 11-4 | 287 | 3.07 |
| 5-5 | 380 | 3.48 | 12-1 | 413 | 4.63 |
| 6-2 | 425 | 3.78 | 12-2 | 453 | 5.98 |
| 6-3 | 465 | 5.75 | 12-3 | 319 | 2.88 |
| 6-4 | 331 | 2.57 | 12-4 | 305 | 3.18 |
| 6-5 | 317 | 2.90 | 13-1 | 413 | 4.45 |
| 7-1 | 310 | 5.53 | 13-2 | 453 | 5.83 |
| 7-2 | 488 | 4.13 | 13-3 | 319 | 2.63 |
| 7-3 | 528 | 5.98 | 13-4 | 305 | 3.13 |
| 7-4 | 394 | 3.49 | | | |

| | | | | | |
|---|---|---|---|---|---|
| *: (M-H)⁻ | | | | | |

**[TABLE 4-2]**

| EX No. | MS-ESI (m/z) (M+H)⁺ | Retention Time (min) | EX No. | MS-ESI (m/z) (M+H)⁺ | Retention Time (min) |
|---|---|---|---|---|---|
| 23-1 | 440# | 6.62 | 34-1 | 257# | 5.48 |
| 23-2 | 284 | 3.15 | 34-2 | 217 | 5.03 |
| 24-1 | 383# | 6.00 | 34-3 | 201 (M-H2O+H)⁺ | 4.97 |
| 24-2 | 423# | 6.05 | 34-4 | 409# | 6.25 |
| 24-3 | 301 | 2.52 | 34-5 | 287 | 2.80 |
| 24-4 | 287 | 2.83 | 36-1 | 199* | 5.78 |
| 25-1 | 286 | 3.08 | 36-2 | 183 | 5.15 |
| 26-1 | 456# | 6.45 | 36-3 | 167 (M-H2O+H)⁺ | 4.82 |
| 26-2 | 302 | 2.57 | 36-4 | 375# | 6.47 |
| 27-1 | 234 | 5.57 | 36-5 | 253 | 2.58 |
| 27-3 | 258 | 4.72 | 37-1 | 267* | 6.02 |
| 27-4 | 260 | 4.60 | 37-2 | 249* | 5.65 |
| 27-5 | 278 | 5.38 | 37-4 | 443# | 6.52 |
| 27-6 | 428 | 5.98 | 37-5 | 321 | 3.22 |
| 27-7 | 328 | 2.53 | 38-1 | 346 | 6.18 |
| 28-1 | 372# | 6.02 | 38-2 | 256 | 5.25 |
| 28-2 | 374# | 5.93 | 38-3 | 328 | 5.87 |
| 28-3 | 392# | 6.38 | 38-4 | 310 | 5.33 |
| 28-4 | 520 | 6.57 | 38-5 | 488 | 4.12 |
| 28-5 | 420 | 4.27 | 38-6 | 528 | 5.97 |
| 28-6 | 541 | 6.03 | 38-7 | 394 | 3.25 |
| 30-1 | 223 (M-HCl+Na)⁺ | 6.03 | 38-8 | 380 | 3.45 |
| 30-2 | 423# | 6.52 | 39-1 | 257# | 5.65 |
| 30-3 | 301 | 3.27 | 39-2 | 217 | 5.28 |
| 31-1 | 401 | 6.65 | 39-3 | 219 | 5.12 |
| 31-2 | 301 | 3.40 | 39-4 | 409# | 6.52 |
| 32-1 | 419# | 6.53 | 39-5 | 287 | 2.97 |
| 32-2 | 366# | 5.93 | 52-1 | 240 | 4.33 |
| 32-3 | 244 | 0.92 | 66-1 | 387 | 6.48 |
| 33-1 | 225# | 4.95 | 66-2 | 287 | 3.03 |
| 33-3 | 169 (M-H₂O+1)⁺ | 4.38 | 67-1 | 256* | 4.40 |
| 33-4 | 377# | 6.12 | 68-1 | 234 | 4.43 |
| 33-5 | 255 | 1.48 | | | |

| | | | | | |
|---|---|---|---|---|---|
| *: (M-H)⁻ #: (M+Na) ⁺ | | | | | |

**[TABLE 5]**

| EX. No. | NMR data (δ:ppm) <no asterisk: 300MHz, *: 400MHz > |
|---|---|
| 4-1* | *(CDCl₃)* 7.53 (1H, s), 7.42-7.28 (6H, m), 6.87 (1H, d, J = 8 Hz), 5.20-5.06 (1H, m), 5.11 (2H, s), 4.39-4.28 (1H, m), 4.28-4.19 (1H, m), 3.88-3.73 (1H, m), 3.42-3.25(2H, m), 2.98-2.80 (2H, m), 2.10-1.98 (1H, m), 1.98-1.83 (1H, m) |
| 4-2* | *(CDCl₃)* 7.49-7.27 (5H, m), 7.41 (1H, dd, J = 9, 2Hz), 7.21(1H, brs), 6.92(1H, d, J = 9Hz), 6.11-6.02 (1H, m), 5.16 (2H, s), 4.49-4.34 (2H, m), 4.31-4.17 (2H, m), 3.98-3.80 (1H, m), 3.47-3.31 (1H, m), 3.31-3.12 (1H, m), 3.06-2.88 (1H, m), 2.24-2.07 (2H, m) |
| 4-3* | *(CDCl₃)* 7.44-7.37 (1H, m), 7.24 (1H, brs), 6.91 (1H, d, J = 9Hz), 6.12 (1H, dd, J = 11, 6 Hz), 4.41 (1H, ddd, J = 11, 4, 4 Hz), 4.26 (1H, ddd, J = 11, 11, 2 Hz), 3.75 (1H, d, J = 17Hz), 3.69 (1H, d, J =17 Hz), 3.24-3.05 (2H, m), 3.01-2.86 (2H, m), 2.29-2.15 (1H, m), 2.15-2.06 (1H, m) |
| 4-4* | *(CDCl₃)* 7.85-7.78 (1H, m), 7.35 (1H, dd, J = 9, 3 Hz), 6.83 (1H, d, J = 9Hz), 4.47-4.36 (1H, m), 4.22-4.11 (1H, m), 3.83 (1H, dd, J = 9, 5Hz), 2.97-2.83 (4H, m), 2.66-2.52 (2H, m), 2.52-2.39 (2H, m), 2.16-2.04 (1H, m), 2.03-1.94 (1H, m) |
| 7-1 | *(CDCl₃)* 8.52-8.44 (1H, m), 8.02-7.93 (2H, m), 7.10 (1H, d, J = 9 Hz), 6.86-6.77 (2H, m), 4.59 (2H, t, J = 7 Hz), 2.83 (2H, t, J = 7 Hz). |
| 7-2* | *(CDCl₃)* 8.51-8.44 (1H, m), 7.89 (1H, dd, J = 9, 3 Hz), 7.43-7.26 (6H, m), 7.00 (1H, d, J = 9 Hz), 6.67 (1H, dd, J = 8, 2 Hz), 6.62 (1H, d, J = 2 Hz), 5.24-5.16 (1H, m), 5.12 (2H, s), 4.36-4.26 (1H, m), 4.26-4.18 (1H, m), 3.83-3.75 (1H, m), 3.41-3.29 (2H, m), 2.90 (2H, t, J = 6 Hz), 2.12-2.01 (1H, m), 1.97-1.86 (1H, m). |
| 7-3* | (CDCl₃) 8.49-8.43 (1H, m), 7.92 (1H, dd, J = 9, 3 Hz), 7.46-7.30 (5H, m), 7.08-6.97 (2H, m), 6.71 (1H, dd, J = 8, 2 Hz), 6.67 (1H, d, J = 2 Hz), 6.09-6.01 (1H, m), 5.18 (2H, s), 4.42-4.31 (2H, m), 4.29-4.20 (2H, m), 3.94-3.76 (1H, m), 3.52-3.38 (1H, m), 3.32-3.18 (1H, m), 3.17-3.05 (1H, m), 2.20-2.09 (2H, m). |
| 7-4* | *(CDCl₃)* 8.46 (1H, s), 7.96-7.87 (1H, m), 7.06 (1H, d, J = 8Hz), 7.01 (1H, d, J = 9 Hz), 6.75-6.68 (1H, m), 6.66 (1H, d, J = 2 Hz), 6.16-6.07 (1H, m), 4.44-4.31 (1H, m), 4.31-4.19 (1H, m), 3.71 (2H, s), 3.27-2.95 (4H, m), 2.30-2.05 (2H, m). |
| 8-1 | *(CDCl₃)* 7.39-7.29 (5H, m), 7.23 (1H, d, J = 8 Hz), 6.71 (1H, d, J = 8 Hz), 6.67 (1H, s), 5.21-5.06 (1H, m), 5.10 (2H, s), 4.33-4.14 (2H, m), 3.75 (1H, t, J = 4 Hz), 3.40-3.2s (2H, m), 2.92-2.79 (2H, m), 2.09-1.96 (1H, m), 1.95-1.80 (1H, m). |
| 8-2 | *(CDCl₃₎)* 7.43-7.31 (5H, m), 6.98 (1H, d, J = 9 Hz), 6.77 (1H, d, J = 9 Hz), 6.73 (1H, s), 6.08-5.98 (1H, m), 5.18 (2H, s), 4.43-4.31 (2H, m), 4.28-4.18 (2H, m), 3.94-3.78 (1H, m), 3.50-3.36 (1H, m), 3.29-3.15 (1H, m), 3.09-2.95 (1H, m), 2.19-2.08 (2H, m). |
| 8-3 | *(CDCl₃)* 7.00 (1H, dd, J = 8, 1 Hz), 6.79-6.73 (1H, m), 6.73-6.67 (1H, m), 6.08 (1H, dd, J = 10, 7 Hz), 4.43-4.31 (2H, m), 4.23 (1H, ddd, J = 11, 11, 2 Hz), 3.69 (2H, s), 3.23-3.04 (2H, m), 3.03-2.88 (2H, m), 2.28-2.02 (2H, m). |
| 11-1 | *(CDCl₃)* 7.41-7.30 (6H, m), 7.10 (1H, d, J = 8 Hz), 7.07 (1H, s), 5.21-5.06 (3H, m), 4.38-4.19 (2H, m), 3.86-3.77 (1H, m), 3.34 (2H, ddd, J = 6, 6, 5 Hz), 2.95-2.81 (2H, m), 2.13-2.00 (1H, m), 1.99-1.87 (1H, m). |
| 11-2* | *(CDCl₃)* 7.45-7.31 (5H, m), 7.18-7.05 (3H, m), 6.13-6.05 (1H, m), 5.18 (2H, s), 4.45-4.35 (2H, m), 4.31-4.19 (2H, m), 3.99-3.77 (1H, m), 3.53-3.37 (1H, m), 3.30-3.14 (1H, m), 3.07-2.93 (1H, m), 2.24-2.08 (2H, m). |
| 11-3 | *(CDCl₃)* 7.18-7.06 (3H, m), 6.15 (1H, dd, J = 10, 7 Hz), 4.41 (1H, ddd, J = 8, 4, 4Hz), 4.25 (1H, ddd, J = 11, 11, 2Hz), 3.72 (2H, s), 3.24-3.06 (2H, m), 3.04-2.89 (2H, m), 2.32-2.05 (2H, m). |
| 11-4 | *(CDCl₃)* 7.64 (1H, d, J = 8 Hz), 7.11 (1H, d, J = 8 Hz), 7.02 (1H, s), 4.45-4.35 (1H, m), 4.21-4.10 (1H, m), 3.90-3.80 (1H, m), 2.97-2.82 (4H, m), 2.64-2.54 (2H, m), 2.51-2.41 (2H, m), 2.18-1.92 (2H, m). |
| 13-1 | *(CDCl₃)* 7.40-7.29 (5H, m), 7.12 (1H, d, J = 8Hz), 7.03 (1H, dd, J = 8, 6Hz), 5.19-5.05 (3H, m), 4.45-4.28 (2H, m), 3.89-3.80 (1H, m), 3.34 (2H, ddd, J = 6, 6, 5 Hz), 2.95-2.79 (2H, m), 2.21-2.03 (1H, m), 2.02-1.88 (1H, m). |
| 13-2 | *(CDCl₃)* 7.43-7.29 (5H, m), 7.08 (1H, dd, J = 8, 6Hz), 6.84 (1H, d, J = 8 Hz), 6.09 (1H, dd, J = 9, 9Hz), 5.17 (2H, s), 4.51 (1H, ddd, J = 8, 4, 4Hz), 4.39 (1H, d, J = 19Hz), 4.34-4.23 (1H, m), 4.22 (1H, d, J = 19Hz), 3.98-3.78 (1H, m), 3.50-3.35 (1H, m), 3.32-3.17 (1H, m), 3.05-2.91 (1H, m), 2.24-2.09 (2H, m). |
| 13-3 | *(CDCl₃)* 7.13-7.04 (1H, m), 6.88 (1H, d, J = 9 Hz), 6.15 (1H, dd, J = 10, 6 Hz), 4.52 (1H, ddd, J = 11, 4, 4 Hz), 4.30 (1H, ddd, J = 11, 11, 2Hz), 3.71 (2H, s), 3.26-3.06 (2H, m), 3.03-2.87 (2H, m), 2.34-2.06 (2H, m). |
| 16-1 | *(CDCl₃)* 7.45 (1H, d, J = 8 Hz), 7.17 (1H, d, J = 8 Hz), 7.11 (1H, s), 4.88-4.81 (1H, m), 4.35-4.29 (2H, m), 2.26-2.02 (2H, m), 1.89 (1H, d, J = 5 Hz). |
| 27-2 | *(CDCl₃) δ:* 7.93 (1H, d, J = 9 Hz), 6.96-6.91 (2H, m), 4.64-4.53 (1H, m), 3.67-3.60 (2H, m), 2.75 (2H, td, J = 7, 1 Hz). |
| 33-2 | *(CDCl₃)* δ: 7.71-7.65 (1H, m), 6.88-6.79 (1H, m), 4.66 (2H, t, J = 7 Hz), 2.86 (2H, t, J = 7 Hz). |
| 37-3 | *(CDCl₃)* δ*:* 7.34 (1H, d, J = 8 Hz), 7.26 (1H, d, J = 8 Hz), 4.86 (1.0H, dd, J = 5,5 Hz), 4.52-4.37 (2H, m), 2.24-2.04 (2H, m), 1.89 (1H, d, J = 5 Hz). |

## Claims

1. A compound represented by general formula (I): 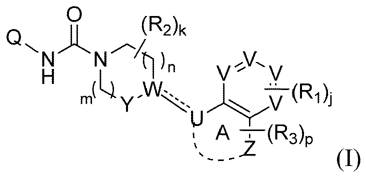 (wherein:
U represents C, CH or N, V may be the same or different and each independently represents CH or N, W represents C, CH or N, Y represents CH₂ or C=O, and Z represents O (oxygen atom) or NR₄, wherein when U is CH, the hydrogen atoms bonded to the carbon atom may be substituted with R₃, when W is CH or Y is CH₂, 1 or 2 of the hydrogen atoms bonded to the carbon atoms may be substituted with R₂; j represents an integer of 0 to 3, k represents an integer of 0 to 2, m represents an integer of 0 to 2, n represents an integer of 0 to 2, and p represents an integer of 0 to 4;
Q represents a (6- to 10-membered ring) aryl or a (5- to 12-membered ring) heteroaryl, which may be substituted with 1 to 4 substituents optionally selected from substituent group T;
the substituent group T consists of the groups below:
1) a halogen;
2) a -(C₁-C₆)-alkyl, a -(C₂-C₆)-alkenyl or a -(C₂-C₆)-alkynyl, wherein the alkyl, alkenyl or alkynyl is unsubstituted or each independently may be substituted with 1 to 5 substituents optionally selected from =O (oxo), a halogen, a -(C₁-C₆)-haloalkyl, an -OR₅ or an -N(R₅)(R₆);
3) a -(C₁-C₆)-haloalkyl;
4) -OH;
5) an -O-(C₁-C₆)-alkyl, wherein the alkyl is unsubstituted or each independently may be substituted with 1 to 3 substituents from a halogen or a -(C₁-C₆)-haloalkyl;
6) -CN;
7) a -C(O)-N(R₅)(R₆);
8) a -(C₁-C₆)-alkyl-OR₅;
9) a -(3- to 12-membered ring) heteroalicyclic;
10) an -N(R₅)(R₆); and,
11) an -N(R₅)-C(O)-R₆;
ring A in formula (I): 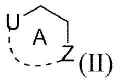 is a 5- to 7-membered ring, the dotted line in the ring A is a linkage having 1 to 3 atoms selected from carbon, nitrogen, oxygen and sulfur atoms, wherein the linkage may be saturated bonds or may partially contain unsaturated bonds;
the bond 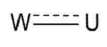 between W and U represents N-CH, N-C, CH-N, CH-CH, CH-C or C=C;
R₁ may be the same or different and each independently represents a group optionally selected from the groups below:
1) a halogen;
2) a -(C₁-C₆)-alkyl, a -(C₂-C₆)-alkenyl or a -(C₂-C₆)-alkynyl, wherein the alkyl, alkenyl or alkynyl is unsubstituted or each independently may be substituted with 1 to 5 substituents optionally selected from =O (oxo), a halogen, a -(C₁-C₆)-haloalkyl, an -OR₅ or an -N(R₅)(R₆);
3) a -(C₁-C₆)-haloalkyl;
4) -OH;
5) an -O-(C₁-C₆)-alkyl, wherein the alkyl is unsubstituted or each independently may be substituted with 1 to 3 substituents from a halogen or a -(C₁-C₆)-haloalkyl;
6) -CN;
7) an -N(R₅)(R₆);
8) a -C(O)-N(R₅)(R₆);
9) an -N(R₅)-C(O)-R₆;
10) an -N(R₅)-SO₂-R₆;
11) an -SO₂-(C₁-C₆)-alkyl;
12) an -SO₂-N(R₅)(R₆);
13) an -SO₂-(C₁-C₆)-haloalkyl;
14) an -S-(C₁-C₆)-alkyl;
15) an -S-(C₁-C₆)-haloalkyl;
16) a -(C₁-C₆)-alkyl-N(R₅)(R₆);
17) an -N(R₅)-C(O)-N(R₅)(R₆);
18) an -N(R₅)-C(O)-OR₅;
19) a -(C₁-C₆)-alkyl-OR₅;
20) a -(3- to 12-membered ring) heteroalicyclic;
21) a -(6- to 10-membered ring) aryl;
22) a -(5- to 12-membered ring) heteroaryl;
23) an -O-(6- to 10-membered ring) aryl;
24) an -O-(5- to 12-membered ring) heteroaryl;
25) a -C(O)-(6- to 10-membered ring) aryl;
26) a -C(O)-(5- to 12-membered ring) heteroaryl;
27) an -SO₂-(6- to 10-membered ring) aryl;
28) an -SO₂-(5- to 12-membered ring) heteroaryl;
29) an -N(R₅)-(CO)-(6- to 10-membered ring) aryl; and,
30) an -N(R₅)-(CO)-(5- to 12-membered ring) heteroaryl;
wherein each of the (6- to 10-membered ring) aryl and the (5- to 12-membered ring) heteroaryl in 21) the -(6- to 10-membered ring) aryl, 22) the -(5- to 12-membered ring) heteroaryl, 23) the -O-(6- to 10-membered ring) aryl, 24) the -O-(5- to 12-membered ring) heteroaryl, 25) the -C(O)-(6- to 10-membered ring) aryl, 26) the -C(O)-(5- to 12-membered ring) heteroaryl, 27) the -SO₂-(6- to 10-membered ring) aryl, 28) the -SO**₂**-(5- to 12-membered ring) heteroaryl, 29) the -N(R₅)-(CO)-(6- to 10-membered ring) aryl and 30) the -N(R₅)-(CO)-(5- to 12-membered ring) heteroaryl may be substituted with 1 to 4 substituents optionally selected from the substituent groups 1) to 20) for R₁;
R₂ may be the same or different and is selected from a -(C₁-C₆)-alkyl, a -(C₂-C₆)-alkenyl, a -(C₂-C₆)-alkynyl, -OH and an -O-(C₁-C₆)-alkyl, wherein the -(C₁-C₆)-alkyl, -(C₂-C₆)-alkenyl or -(C₂-C₆)-alkynyl is unsubstituted or each independently may be substituted with 1 to 5 substituents selected from =O (oxo), a -(C₁-C₆)-haloalkyl, an -OR₅ and an N(R₅)(R₆), and said R₂ may combine with each other to form a 3- to 6-membered carbon ring;
R₃ represents a group optionally selected from the groups below:
1) a -(C₁-C₆)-alkyl, a -(C₂-C₆)-alkenyl or a -(C₂-C₆)-alkynyl, wherein said alkyl, alkenyl or alkynyl is unsubstituted or each independently may be substituted with 1 to 5 substituents optionally selected from =O (oxo), a halogen, a -(C₁-C₆)-haloalkyl, an -OR₅ or an -N(R₅)(R₆);
2) -OH;
3) a -C(O)-N(R₅)(R₆);
4) an -N(R₅)-C(O)-R₆;
5) an -N(R₅)-SO₂-R₆;
6) an -N(R₅)-C(O)-N(R₅)(R₆);
7) an -N(R₅)-(CO)-(6- to 10-membered ring) aryl; and,
8) an -N(R₅)-(CO)- (5- to 12-membered ring) heteroaryl;
wherein the (6- to 10-membered ring) aryl and (5- to 12-membered ring) heteroaryl in 7) the -NR₅-(CO)-(6- to 10-membered ring) aryl and 8) the -NR₅-(CO)- (5- to 12-membered ring) heteroaryl each may be substituted with 1 to 4 substituents optionally selected from the substituent groups 1) to 21) for R₁;
R₄ represents a group optionally selected from the groups below:
1) hydrogen atom;
2) a -(C₁-C₆)-alkyl, a -(C₁-C₆)-alkenyl or a -(C₁-C₆)-alkynyl, wherein the alkyl, alkenyl or alkynyl is unsubstituted or each independently may be substituted with 1 to 5 substituents optionally selected from =O (oxo), a halogen, a -(C₁-C₆)-haloalkyl, an -OR₅ and an -N(R₅)(R₆);
3) a -C(O)-N(R₅)(R₆);
4) an -SO₂-(C₁-C₆)-alkyl;
5) a -(3- to 12-membered ring) heteroalicyclic;
6) a -(6- to 10-membered ring) aryl;
7) a -(5- to 12-membered ring) heteroaryl;
8) a -C(O)-(6- to 10-membered ring) aryl;
9) a -C(O)-(5- to 12-membered ring) heteroaryl
10) an -SO₂-(6- to 10-membered ring) aryl
11) an -SO₂-(5- to 12-membered ring) heteroaryl; and,
12) a -C(O) -(C₁-C₆)-alkyl;
and,
R₅ and R₆ each independently represents an atom or group selected from hydrogen atom, a -(C₃-C₈)-cycloalkyl and a -(C₁-C₆)-alkyl, wherein the -(C₁-C₆)-alkyl independently may be substituted with 1 to 5 substituents selected from -OH, an -O-(C₁-C₆)-alkyl, a -(C₁-C₆)-haloalkyl and an -O-(C₁-C₆)-haloalkyl);
or a pharmaceutically acceptable salt thereof or a solvate thereof.

2. A pharmaceutical composition comprising as an active ingredient at least one of the compound according to claim 1, a pharmaceutically acceptable salt thereof and a solvate thereof.

3. A pharmaceutical composition comprising as an active ingredient at least one of the compound according to claim 1, a pharmaceutically acceptable salt thereof and a solvate thereof, in which the active ingredient is concomitantly used with other drugs or agents.
